# EUROPEAN PATENT APPLICATION

(11) **EP 1 985 621 A1**
(43) Date of publication of application: **29.10.2008**
(21) Application number: 07713600.0
(22) Date of filing: 12.01.2007
(51) Int. Cl.: C07J 9/00, B01J 21/08, B01J 23/44, C07J 1/00, C07J 17/00, C07J 41/00

(54) **PROCESS FOR PRODUCTION OF STEROIDS**

(30) Priority: 12.01.2006 JP 2006004710; 18.01.2006 JP 2006010233
(71) Applicant: Mitsubishi Chemical Corporation, Minato-ku Tokyo 108-0014 (JP)
(72) Inventor: TAKEHARA, Jun, Yokohama-shi, Kanagawa, 227-8502 (JP); FUJIWARA, Naoya, Yokohama-shi, Kanagawa, 227-8502 (JP); ENDOU, Kyouko, Yokohama-shi, Kanagawa, 227-8502 (JP); KAWAI, Junya, Yokohama-shi, Kanagawa, 227-8502 (JP); HOSOKAWA, Akemi, Yokohama-shi, Kanagawa, 227-8502 (JP); SUMITANI, Naoko, Yokohama-shi, Kanagawa, 227-8502 (JP)
(74) Representative: Polypatent
(86) International application number: PCT/JP2007/050297
(87) International publication number: WO 2007/080951

(57) **Abstract**

It is an object of the present invention to provide a novel method for producing a steroid compound. The present invention provides a method for producing 5β-3,7-dioxocholanic acid or an ester derivative thereof, using, as a raw material, a sterol having double bonds at position 5 and at position 24, such as cholesta-5,7,24-trien-3β-ol, ergosta-5,7,24(28)-trien-3β-ol, desmosterol, fucosterol, or ergosta-5,24(28)-dien-3β-ol, via the following 4 steps:
(I) a step involving oxidation of a hydroxyl group at position 3 and isomerization of a double bond at position 5 to position 4;
(II) a step involving the oxidative cleavage of a side chain to convert position 24 to a carboxyl group or an ester derivative thereof;
(III) a step of introducing an oxygen functional group into position 7; and
(IV) a step of constructing a 5β configuration by reductive saturation of a double bond at position 4.

## Description

### TECHNICAL FIELD

The present invention relates to a method for producing a steroid compound. The present invention specifically relates to a method for producing 5β-3,7-dioxocholanic acid or an ester derivative thereof by reductive saturation of a steroid compound having a double bond at position 4 to construct a 5β configuration. The present invention more specifically relates to a method for producing 5β-3,7-dioxocholanic acid or an ester derivative thereof, using, as a raw material, a sterol having double bonds at position 5 and at position 24, such as cholesta-5,7,24-trien-3β-ol, ergosta-5,7,24(28)-trien-3β-ol, desmosterol, fucosterol, or ergosta-5,24(28)-dien-3β-ol, via the following 4 steps:
(I) a step involving oxidation of a hydroxyl group at position 3 and isomerization of a double bond at position 5 to position 4;
(II) a step involving the oxidative cleavage of a side chain to convert position 24 to a carboxyl group or an ester derivative thereof;
(III) a step of introducing an oxygen functional group into position 7; and
(IV) a step of constructing a 5β configuration by reductive saturation of a double bond at position 4.

More specifically, the present invention relates to a method for producing 5β-3,7-dioxocholanic acid or an ester derivative thereof via cholesta-4,6,24-trien-3-one by a chemical synthesis method using cholesta-5,7,24-trien-3β-ol as a raw material. This 5β-3,7-dioxocholanic acid and an ester derivative thereof are useful as synthetic intermediates of medicaments, such as ursodeoxycholic acid or chenodeoxycholic acid.

### BACKGROUND ART

As methods for producing 5β-3,7-dioxocholanic acid or an ester derivative thereof, methods of oxidizing the hydroxyl groups of raw materials having the same skeletons and the same numbers of carbon atoms, such as chenodeoxycholic acid or ursodeoxycholic acid derived from bile acid, have been reported (see Patent Documents 2, 3, 4 and 5, and Non-Patent Documents 1 and 2, for example). In addition, as methods for producing chenodeoxycholic acid or ursodeoxycholic acid, the following methods have been reported: (1) methods using bile acids contained in animals as raw materials (see Patent Documents 6 and 7, and Non-Patent Documents 1 and 2, for example); (2) methods of inducing chenodeoxycholic acid or ursodeoxycholic acid from sterols derived from plants, such as stigmasterol (see Patent Document 8 and Non-Patent Document 3, for example); and (3) a method of inducing chenodeoxycholic acid or ursodeoxycholic acid from raw materials having small side chain carbon numbers, such as progesterone (see Patent Document 9, for example).

However, in the case of the method described in (1) above, raw materials derived from natural resources are expensive, and it is difficult to obtain sufficient quantities of such raw materials. Thus, it has been desired to establish an inexpensive chemical synthesis method. In addition, in the case of the methods described in (2) and (3) above, raw materials derived from natural resources are expensive as in the case of the method of (1), and these methods require an extremely large number of steps in total, such as a step of adjusting the carbon number of a side chain to that of a desired compound or a multistage oxidation step necessary for introduction of a functional group into position 7. Moreover, these methods also require expensive reactants, and thus they are not economical.

On the other hand, Non-Patent Document 4 describes a method comprising Oppenauer oxidation of a steroid compound having a double bond at position 5 and a hydroxyl group at position 3, so as to convert the steroid compound to a 3-oxo-4-ene steroid compound, for example.

Moreover, Patent Document 10 and Non-Patent Document 5 describe that reductive saturation of a double bond at position 4 is effective as a means for constructing a 5β configuration, for example.

Furthermore, Non-Patent Document 6 describes a method involving epoxidation of a 3-oxo-4,6-dien steroid compound so as to convert the compound to a 3-oxo-4-en-6,7-epoxy steroid compound as a means for introducing an oxygen functional group into the position 7 of a steroid compound, for example. Further, Non-Patent Document 7 describes a method of converting a 3-oxo-4-ene steroid compound to a 3-oxo-4-en-7-ol steroid compound using microorganisms, for example. Still further, Non-Patent Document 8 describes a method of converting a 3-oxo steroid compound to a 3-oxo-7-ol steroid compound using microorganisms, for example. Still further, Non-Patent Document 9 describes a method of converting a 5β-3-hydroxy steroid compound to a 5β-3,7-dihydroxy steroid compound using microorganisms, for example.

Moreover, based on general findings of organic chemistry, as a method involving the oxidative cleavage of a double bond, a method of cleaving the double bond via epoxidation or glycolization, a method of cleaving the double bond via ketone, a direct cleavage method using ozone, and the like have been known.

Cholesta-4,6,24-trien-3-one, which is an intermediate in the case of using cholesta-5,7,24-trien-3β-ol as a raw material, is useful as a synthetic intermediate of various types of steroid medicaments. However, conventionally, cholesta-4,6,24-trien-3-one has been derived from natural resource materials, and it has been produced via a long reaction step. Thus, application of cholesta-4,6,24-trien-3-one has had a certain limit in terms of cost and quantity. For example, Non-Patent Document 10 describes a method for producing cholesta-4,6,24-trien-3-one, which comprises performing Oppenauer oxidation on cholesta-5,24-dien-3-ol (desmo) to obtain 3-oxo-4,24-dien, enol-etherifying the obtained compound to obtain 3-ethoxy-3,5,24-trien, and then oxidizing the obtained compound with manganese dioxide.

On the other hand, Patent Document 1 describes a method for producing cholesta-5,7,24-trien-3β-ol, which comprises modifying Eumycetes that produce ergosterol via zymosterol in a metabolic engineering manner, so as to produce a mutant strain, culturing the mutant strain, and then collecting cholesta-5,7,24-trien-3β-ol from the culture.
[Patent Document 1] JP Patent Publication (Kokai) No. 2004-141125 A
[Patent Document 2] JP Patent Publication (Kokai) No. 52-78864 A (1977)
[Patent Document 3] JP Patent Publication (Kokai) No. 52-78863 A (1977)
[Patent Document 4] Spanish Patent No. 489661
[Patent Document 5] French Patent No. 2453182
[Patent Document 6] JP Patent Publication (Kokai) No. 64-61496 A (1989)
[Patent Document 7] JP Patent Publication (Kokoku) No. 3-5399 B (1991) (JP Patent Publication (Kokai) No. 58-029799 A (1983))
[Patent Document 8] Chinese Patent No. 1217336
[Patent Document 9] Chinese Patent No. 1308085
[Patent Document 10] International Publication WO02/088166
[Non-Patent Document 1] Bulletin of the Chemical Society of Japan, 1955, Vol. 76, p. 297
[Non-Patent Document 2] J. Chem. Soc., Perkin. 1, 1990, Vol. 1, p. 1
[Non-Patent Document 3] Yunnan Daxue Xuebao, Ziran Kexueban, 1998, Vol. 20, p. 399
[Non-Patent Document 4] Org. Synth. Col. Vol. III, 1955, p. 207
[Non-Patent Document 5] Steroids, 1983, Vol. 42, No. 6, p. 707
[Non-Patent Document 6] Helv. Chim. Acta, 1971, Vol. 54, No. 8, p. 2775
[Non-Patent Document 7] Appl. Environ. Microbiol., 1986, Vol. 51, p. 946
[Non-Patent Document 8] J. Chem. Res., Synop., 1986, No. 2, p. 48
[Non-Patent Document 9] Appl. Environ. Microbiol., 1982, Vol. 44, No. 6, p. 1249
[Non-Patent Document 10] Biochem.Biophys.Res.Commun., 1965, Vol. 21, No. 2, p. 149

### DISCLOSURE OF THE INVENTION

### Problems to be Solved by the Invention

It is an object of the present invention to provide a method for producing 5β-3,7-dioxocholanic acid or an ester derivative thereof, using a sterol having double bonds at position 5 and at position 24 as a raw material, more specifically, using cholesta-5,7,24-trien-3β-ol, ergosta-5,7,24(28)-trien-3β-ol, desmosterol, fucosterol or ergosta-5,24(28)-dien-3β-ol as a raw material, via the following 4 steps:
(I) a step involving oxidation of a hydroxyl group at position 3 and isomerization of a double bond at position 5 to position 4;
(II) a step involving the oxidative cleavage of a side chain to convert position 24 to a carboxyl group or an ester derivative thereof;
(III) a step of introducing an oxygen functional group into position 7; and
(IV) a step of constructing a 5β configuration by reductive saturation of a double bond at position 4.

More specifically, it is an object of the present invention to provide a method for synthesizing 5β-3,7-dioxocholanic acid that is a compound having oxygen functional groups at positions 3 and 7 and having carboxylic acid or ester at position 24, or an ester derivative thereof, via cholesta-4,6,24-trien-3-one, using cholesta-5,7,24-trien-3β-ol as a raw material.

### Means for Solving the Problems

In order to efficiently produce 5β-3,7-dioxocholanic acid or an ester derivative thereof from materials other than those having the same skeletons and same numbers of carbon atoms, it is preferable to use a steroid material capable of constructing the same side chain carbon numbers in a fewer steps. Accordingly, in the case of steroids having a double bond at position 24, as a result of oxidative cleavage, its position 24 can be induced to a carboxyl group or an ester derivative thereof.

Moreover, in the case of 3-sterols having a double bond at position 5, as a result of oxidation of position 3 and isomerization of a double bond at position 5 to position 4, reductive saturation of the compound occurs, and thus 5β configuration can be constructed.

Furthermore, in the case of 3-oxo-4,6-diene steroids, an oxygen functional group may be introduced into position 7 by epoxidation of a double bond at position 6, and in the case of 3-oxo-4-ene steroids, 3-oxo steroids, or 3-hydroxy steoids and 3-hydoxysteroids, hydroxylation of position 7 can be carried out using microorganisms.

As a result of intensive studies directed towards achieving the aforementioned objects, the present inventors have found that, when cholesta-5,7,24-trien-3β-ol is used as a raw material, for example, oxidation of the cholesta-5,7,24-trien-3β-ol to a ketone body at position 3 and isomerization of a double bond at position 5 to position 4 are first carried out, and thereafter, a double bond at position 7 is isomerized to a double bond at position 6, so that a 3-keto-4,6,24-triene compound can be obtained.

Moreover, the inventors have reached the following findings. Double bonds at position 6 and at position 24 of cholesta-4,6,24-trien-3-one are epoxidized, and saturation of a double bond at position 4 due to hydrogenation, the reductive cleavage of a carbon-oxygen bond at position 6, and construction of a 5β configuration are then carried out. Thereafter, 24,25-epoxy is hydrolyzed to 24,25-diol, and oxidation of a hydroxyl group at position 7 to ketone and the oxidative cleavage thereof to 24-carboxylic acid are carried out. Thereafter, in some cases, the 24-carboxylic acid is further esterified, so as to synthesize 5β-3,7-dioxocholanic acid that is useful as a synthetic intermediate of various types of steroids, such as ursodeoxycholic acid or chenodeoxycholic acid, and an ester derivative thereof.

Furthermore, the inventors have also reached the following findings. After epoxidation of the double bonds at position 6 and at position 24 in the aforementioned reaction, even if the reaction order is changed, namely, even if only the epoxy at position 24 is hydrolyzed to diol, and thereafter, hydrogenation of the epoxy at position 6 and saturation of the double bond at position 4 are carried out, 5β-3,7-dioxocholanic acid and an ester derivative thereof can be synthesized.

Further, the inventors have also reached the following findings. Only the position 24 of cholesta-4,6,24-trien-3-on is epoxidized, it is hydrolyzed to diol, the double bond at position 6 is epoxidized, and hydrogenation of the epoxy at position 6, saturation of the double bond at position 4, oxidation of the hydroxyl group at position 7 to ketone, and the oxidative cleavage thereof to 24-carboxylic acid are carried out. In some cases, the 24-carboyxlic acid is further esterified. Thus, 5β-3,7-dioxocholanic acid and an ester derivative thereof can be synthesized.

Still further, the inventors have also reached the following findings. After hydrogenation of the epoxy at position 6 and saturation of the double bond at position 4 in the aforementioned reaction, the hydroxyl group at position 7 is oxidized, the epoxy at position 24 is hydrolyzed, the oxidative cleavage thereof to 24-carboxylic acid is carried out, and in some cases, the 24-carboxylic acid is further esterified, so that 5β-3,7-dioxocholanic acid and an ester derivative thereof can be synthesized.

Still further, the inventors have also reached the following findings. The double bonds at position 6 and at position 24 of cholesta-4,6,24-trien-3-on are epoxidized. Thereafter, saturation of the double bond at position 4 due to hydrogenation, the reductive cleavage of the carbon-oxygen bond at position 6, and construction of a 5β configuration are carried out. Further, reduction of the ketone at position 3 to a hydroxyl group is carried out, and protection of 3,7-hydroxyl group is then carried out. Thereafter, the epoxy at position 24 is isomerized to ketone, and it is then oxidized to a 24-isopropyl ester. Thereafter, hydrolysis to 24-carboxylic acid and deprotection are carried out, and finally, the hydroxyl groups at positions 3 and 7 are oxidized. Further, in some cases, the 24-carboxylic acid is esterified. Thus, 5β-3,7-dioxocholanic acid or an ester derivative thereof can be synthesized.

Still further, the inventors have also reached the following findings. Only the position 24 of cholesta-4,6,24-trien-3-on is epoxidized, and the epoxy at position 24 is isomerized to ketone. Thereafter, the double bond at position 6 is epoxidized, and saturation of the double bond at position 4 due to hydrogenation, the reductive cleavage of the carbon-oxygen bond at position 6, and construction of a 5β configuration are carried out. Thereafter, the ketone at position 3 is reduced to a hydroxyl group, and the hydroxyl groups at positions 3 and 7 are protected, followed by oxidation to a 24-isopropyl ester. Thereafter, hydrolysis to 24-carboxylic acid and deprotection are carried out, and finally, the hydroxyl groups at positions 3 and 7 are oxidized. Further, in some cases, the 24-carboxylic acid is esterified. Thus, 5β-3,7-dioxocholanic acid or an ester derivative thereof can be synthesized.

Still further, the inventors have also reached the following findings. Only the position 24 of cholesta-4,6,24-trien-3-on is epoxidized, and it is hydrolyzed to diol. The oxidative cleavage to 24-aldehyde, oxidation to 24-carboxylic acid, and esterification of 24-carboxylic acid are carried out. The double bond at position 6 is epoxidized, and saturation of the double bond at position 4 due to hydrogenation, the reductive cleavage of the carbon-oxygen bond at position 6, and construction of a 5β configuration are carried out. Finally, the hydroxyl group at position 7 is oxidized. Thus, the ester derivative of 5β-3,7-dioxocholanic acid can be synthesized.

Accordingly, based on the aforementioned findings, the present inventors have found that 5β-3,7-dioxocholanic acid or an ester derivative thereof can be produced using, as a raw material, a sterol having double bonds at position 5 and at position 24, such as cholesta-5,7,24-trien-3β-ol, ergosta-5,7,24(28)-trien-3β-ol, desmosterol, fucosterol, or ergosta-5,24(28)-dien-3β-ol, via the following 4 steps:
(I) a step involving oxidation of a hydroxyl group at position 3 and isomerization of a double bond at position 5 to position 4;
(II) a step involving the oxidative cleavage of a side chain to convert position 24 to a carboxyl group or an ester derivative thereof;
(III) a step of introducing an oxygen functional group into position 7; and
(IV) a step of constructing a 5β configuration by reductive saturation of a double bond at position 4, thereby completing the present invention.

The schematic view of the aforementioned steps (I) to (IV) will be shown below. 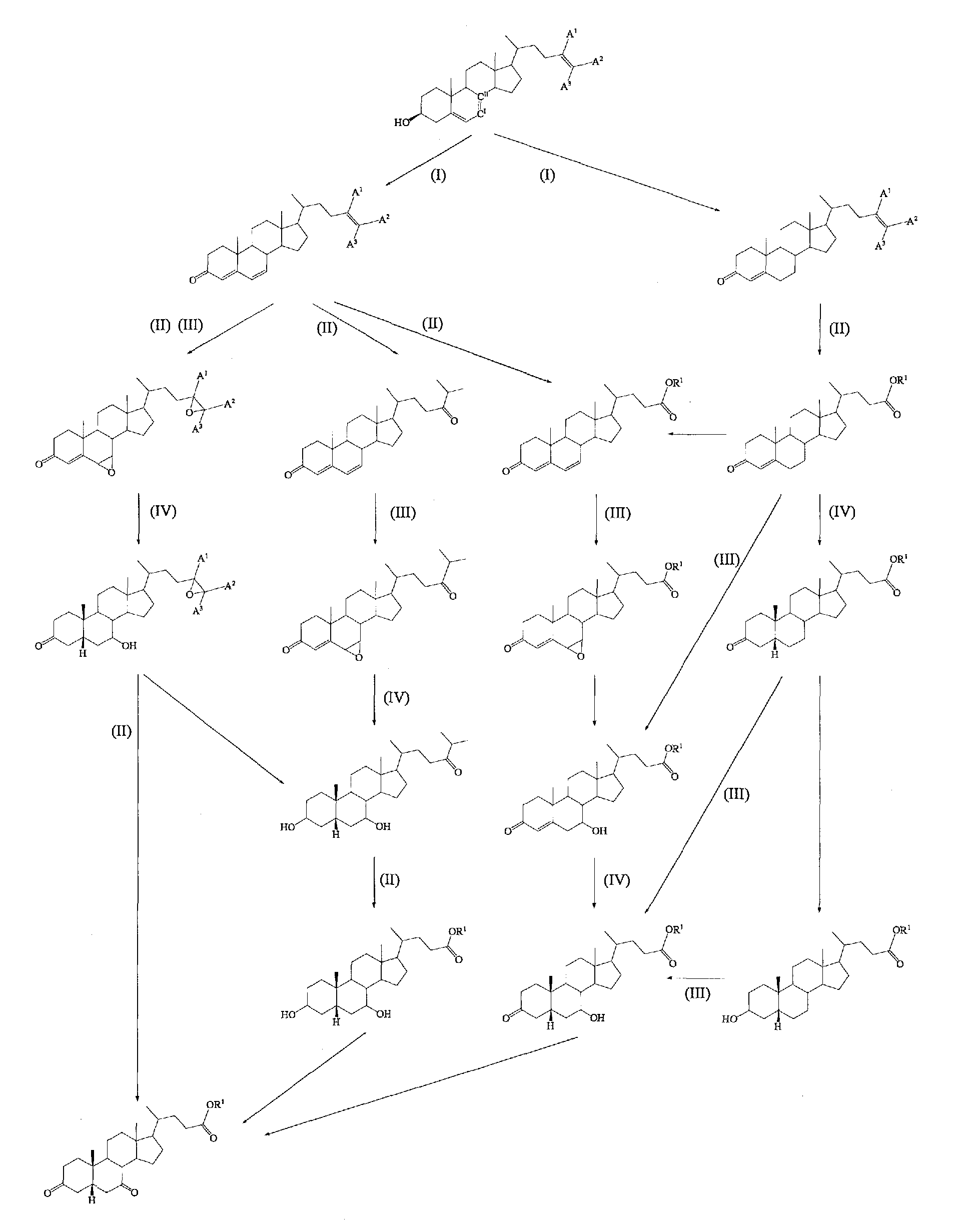 wherein A¹ represents a hydrogen atom or an isopropyl group; each of A² and A³ independently represents a methyl group when A¹ is a hydrogen atom, or a hydrogen atom or a methyl group when A¹ is an isopropyl group; the bond between C^{I} and C^{II} represents a single bond or a double bond; and R¹ represents a hydrogen atom or an alkyl group containing 1 to 6 carbon atoms.

The schematic view of the aforementioned step (II) will be shown below. 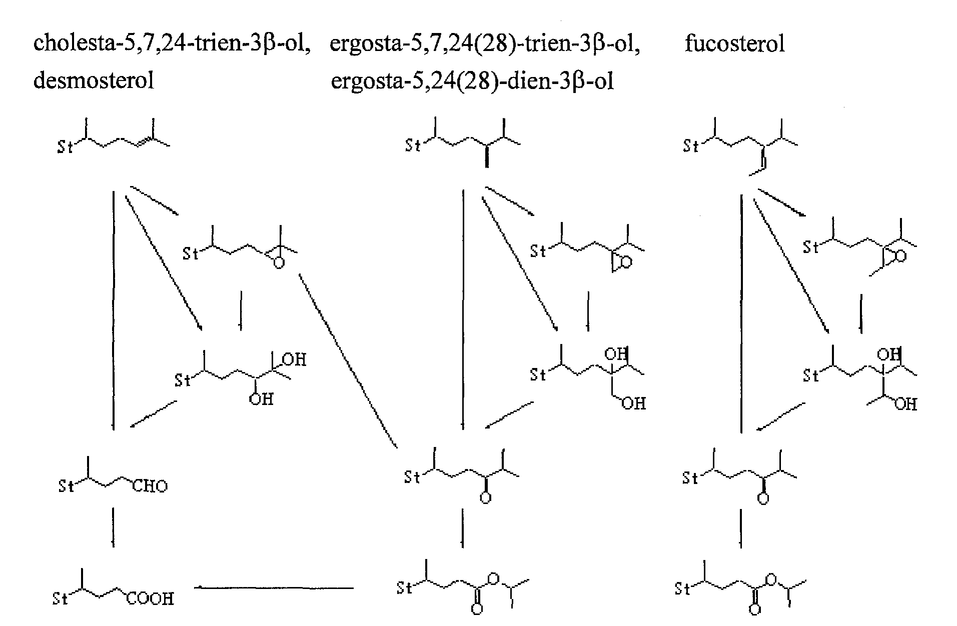 wherein St represents a steroid skeleton consisting of ring A, ring B, ring C, and ring D, wherein with regard to the aforementioned steroid skeleton, (1) it binds to a side chain shown in the formula at position C17, (2) it may have a hydroxyl group, a protected hydroxyl group, a keto group, or an epoxy group on the ring A, ring B, ring C, and ring D, (3) C-C bonds at one or more positions selected from the group consisting of positions C1 to C8 may have double bonds, and (4) one or more positions selected from the group consisting of positions C4, C10, C13 and C14 may be substituted with methyl groups.

That is to say, according to the present invention, the inventions according to the following (1) to (56) are provided:
(1) A method for producing 5β-3,7-dioxocholanic acid (8), ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (8), (32a), (32b), (32c) or (32d), or an ester derivative thereof: 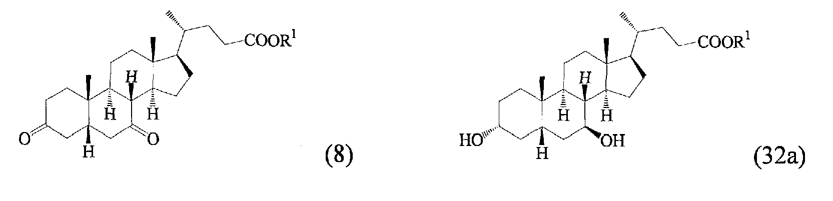 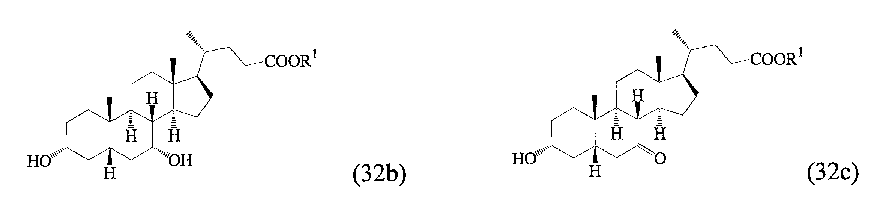 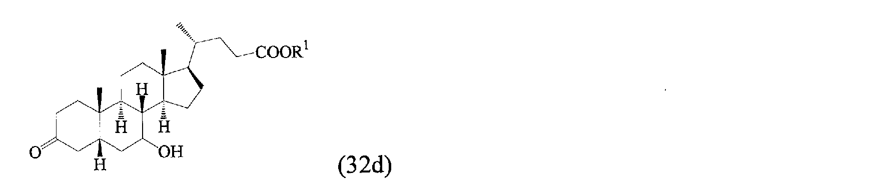 wherein R¹ represents a hydrogen atom or an alkyl group containing 1 to 6 carbon atoms,
   which comprises a step of constructing a 5β configuration by reductive saturation of a double bond at position 4, using, as a raw material, a steroid compound containing 22 or more carbon atoms generated from carbohydrate by a fermentation method.
(2) A method for producing 5β-3,7-dioxocholanic acid (8), ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (8), (32a), (32b), (32c) or (32d), or an ester derivative thereof: 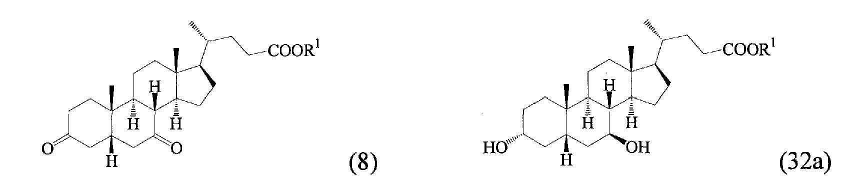 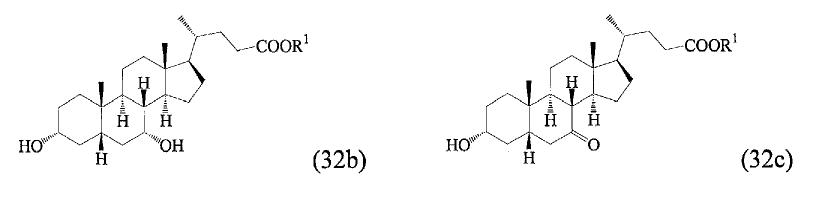 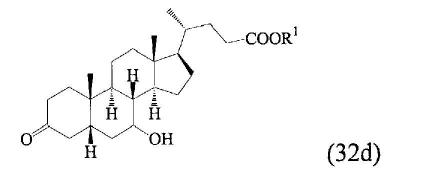 wherein R¹ represents a hydrogen atom or an alkyl group containing 1 to 6 carbon atoms,
   wherein a 5β configuration is constructed by reductive saturation of a double bond at position 4 in a steroid compound represented by the following formula (A1), (A2), (A3), (A4), (A5), (A6), (A7) or (A8): 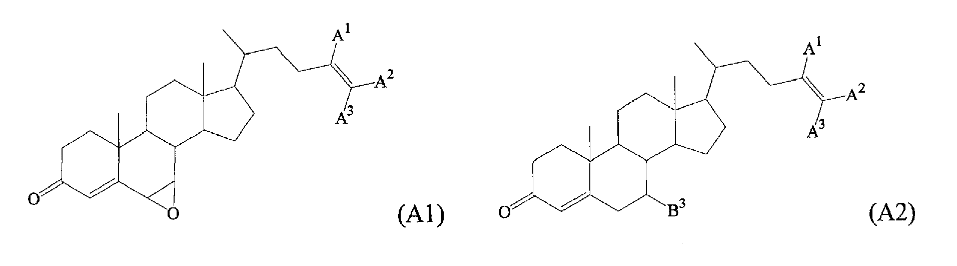 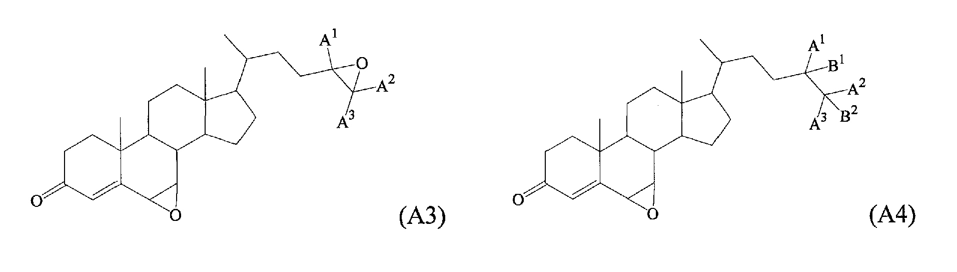 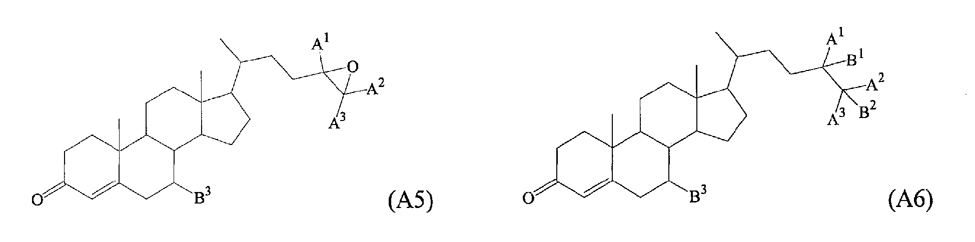 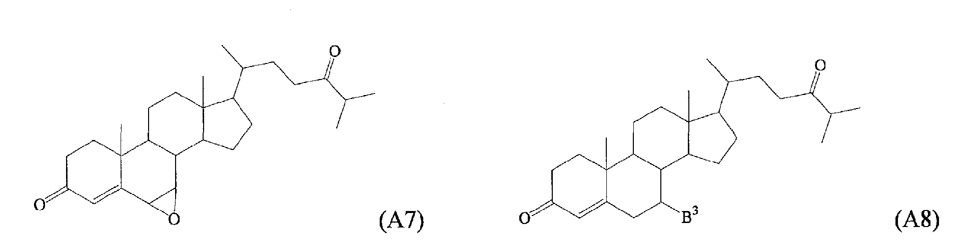 wherein A¹ represents a hydrogen atom or an isopropyl group; each of A² and A³ independently represents a methyl group when A¹ is a hydrogen atom, or a hydrogen atom or a methyl group when A¹ is an isopropyl group; and each of B¹, B² and B³ independently represents a hydroxyl group or a protected hydroxyl group.
(3) A method for producing 5β-3,7-dioxocholanic acid (8), ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (8), (32a), (32b), (32c) or (32d), or an ester derivative thereof: 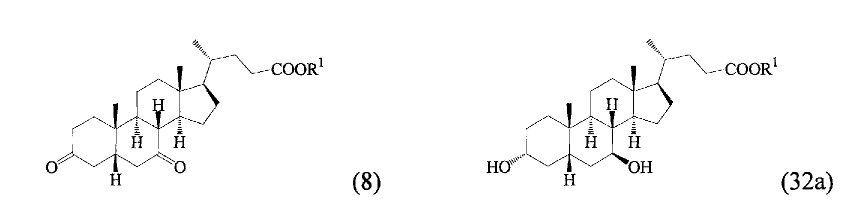 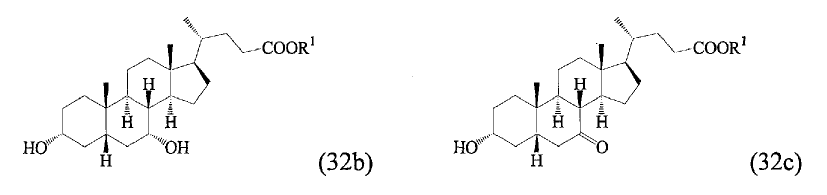 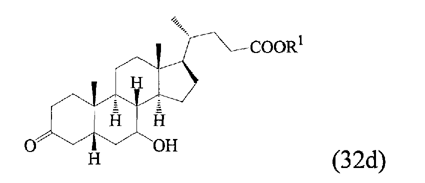 wherein R¹ represents a hydrogen atom or an alkyl group containing 1 to 6 carbon atoms,
   wherein a 5β configuration is constructed by reductive saturation of a double bond at position 4 in a steroid compound represented by the following formula (A1), (A2), (A3), (A4), (A5), (A6), (A7), (A8), (A9) or (A10): 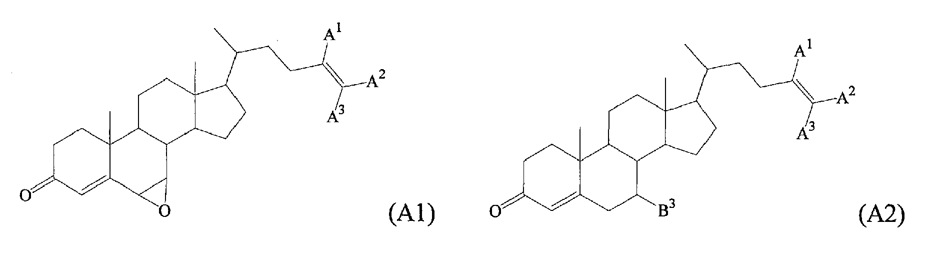 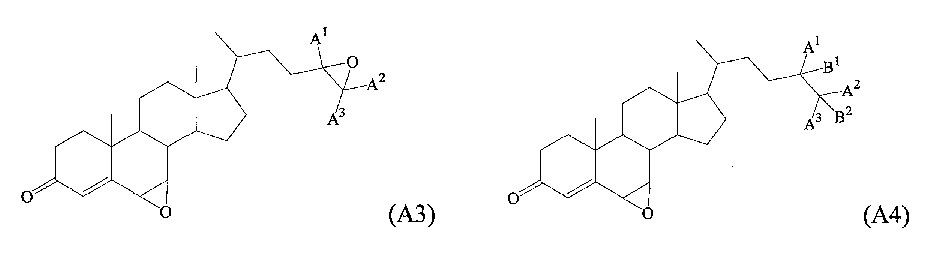 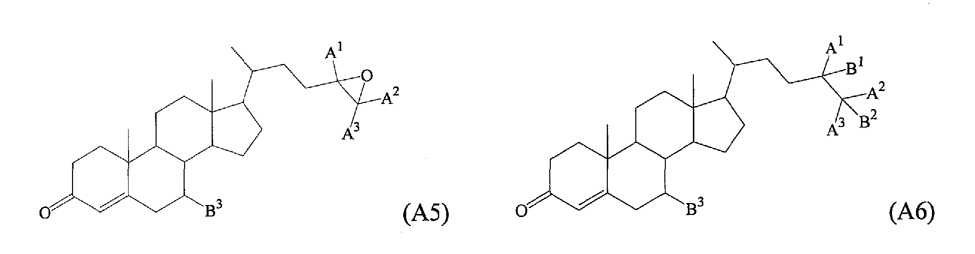 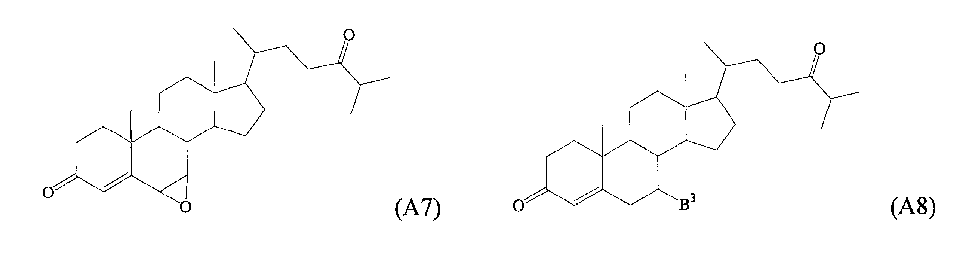 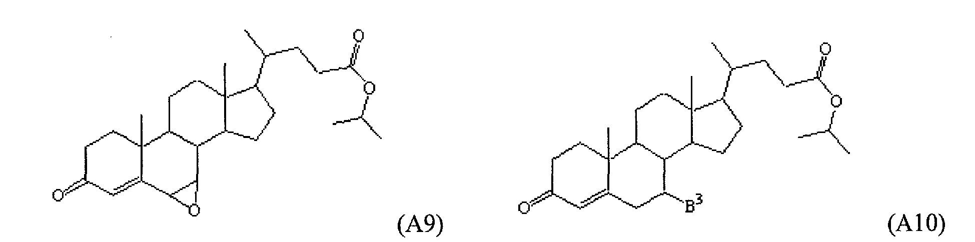 wherein A¹ represents a hydrogen atom or an isopropyl group; each of A² and A³ independently represents a methyl group when A¹ is a hydrogen atom, or a hydrogen atom or a methyl group when A¹ is an isopropyl group; and each of B¹, B² and B³ independently represents a hydroxyl group or a protected hydroxyl group,
   wherein said steroid compound is induced from a sterol compound represented by the following formula (1): 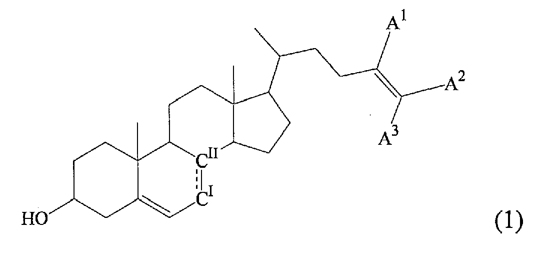 wherein A¹ represents a hydrogen atom or an isopropyl group; each of A² and A³ independently represents a methyl group when A¹ is a hydrogen atom, or a hydrogen atom or a methyl group when A¹ is an isopropyl group; and the bond between C^{I} and C^{II} represents a single bond or a double bond.
(4) A method for producing 5β-3,7-dioxocholanic acid (8), ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (8), (32a), (32b), (32c) or (32d), or an ester derivative thereof: 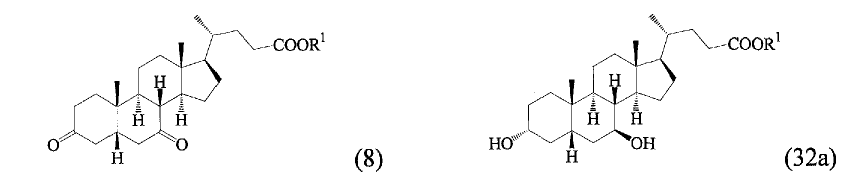 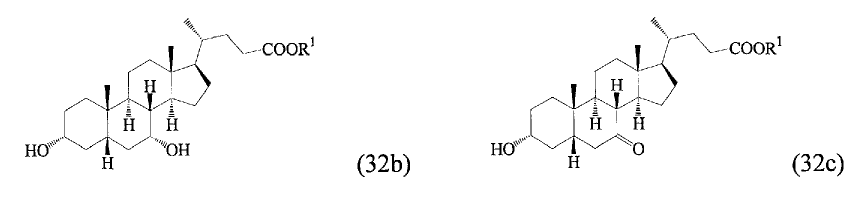 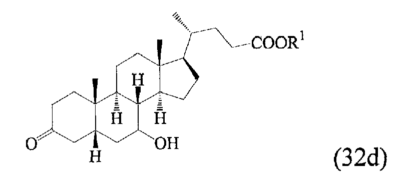 wherein R¹ represents a hydrogen atom or an alkyl group containing 1 to 6 carbon atoms,
   wherein a sterol compound represented by the following formula (1) is used as a raw material: 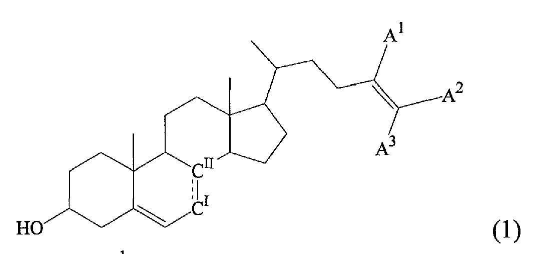 wherein A¹ represents a hydrogen atom or an isopropyl group; each of A² and A³ independently represents a methyl group when A¹ is a hydrogen atom, or a hydrogen atom or a methyl group when A¹ is an isopropyl group; and the bond between C^{I} and C^{II} represents a single bond or a double bond, and
   said production method comprises the following steps:
   (I) a step involving oxidation of a hydroxyl group at position 3 and isomerization of a double bond at position 5 to position 4;
   (II) a step involving the oxidative cleavage of a side chain to convert position 24 to a carboxyl group or an ester derivative thereof;
   (III) a step of introducing an oxygen functional group into position 7; and
   (IV) a step of constructing a 5β configuration by reductive saturation of a double bond at position 4.
(5) The method according to (4) above, wherein the sterol compound represented by the following formula (1) is cholesta-5,7,24-trien-3β-ol, ergosta-5,7,24(28)-trien-3β-ol, desmosterol, fucosterol, or ergosta-5,24(28)-dien-3β-ol: 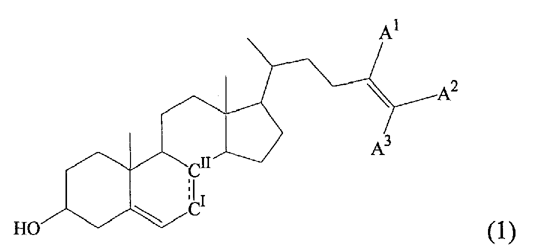 wherein A¹ represents a hydrogen atom or an isopropyl group; each of A² and A³ independently represents a methyl group when A¹ is a hydrogen atom, or a hydrogen atom or a methyl group
   when A¹ is an isopropyl group; and the bond between C^{I} and C^{II} represents a single bond or a double bond.
(6) The method according to (4) above, wherein the sterol compound represented by the following formula (1) is cholesta-5,7,24-trien-3β-ol: 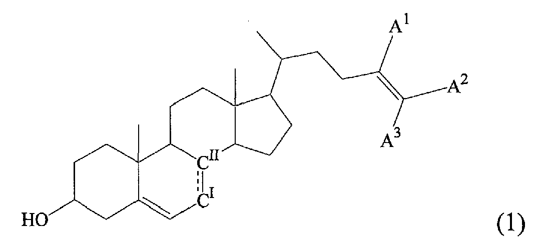 wherein A¹ represents a hydrogen atom or an isopropyl group; each of A² and A³ independently represents a methyl group when A¹ is a hydrogen atom, or a hydrogen atom or a methyl group
   when A¹ is an isopropyl group; and the bond between C^{I} and C^{II} represents a single bond or a double bond.
(7) A method for producing cholesta-4,6,24-trien-3-one represented by the following formula (4): 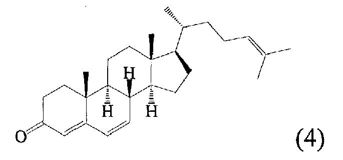 which comprises oxidizing cholesta-5,7,24-trien-3β-ol represented by the following formula (2) to obtain cholesta-4,7,24-trien-3-one represented by the following formula (3), and then isomerizing it: 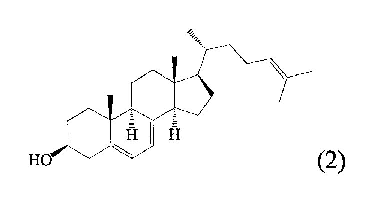 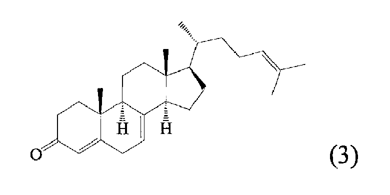
(8) The method according to (7) above, wherein the oxidation reaction is carried out in the presence of a ketone compound and a metal alkoxide.
(9) The method according to (8) above, wherein the oxidation reaction is carried out while oxygen is blocked.
(10) The method according to (8) above, wherein the ketone compound is represented by the formula R²(C=O)R³ wherein each of R² and R³ independently represents a chain or cyclic alkyl group containing 1 to 10 carbon atoms, or R² and R³ may bind to each other to form a ring structure containing 3 to 8 carbon atoms).
(11) The method according to (7) above, wherein the isomerization reaction is carried out in the presence of a basic compound.
(12) The method according to (11) above, wherein the basic compound is hydroxide, carbonate or alkoxide of alkaline metal or alkaline-earth metal.
(13) The method according to (11) above, wherein the isomerization reaction is carried out while oxygen is blocked.
(14) A method for producing 3-oxo-4,7-diene steroid compound, wherein a method of oxidizing a 3-hydroxy-5,7-diene steroid compound represented by the following formula (2a), (2b), (2c), (2d) or (2e) to a compound represented by the following formula (3a), (3b), (3c), (3d) or (3e) is carried out while oxygen is blocked and in the presence of a ketone compound and a metal alkoxide: 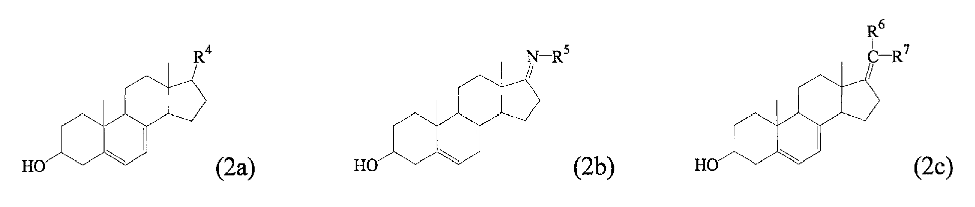 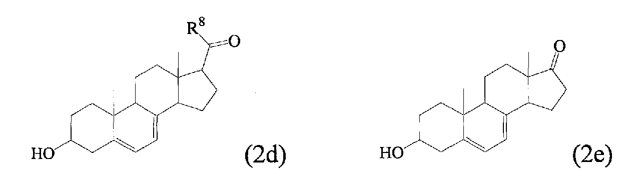 wherein each of R⁴ to R⁸ independently represents a hydrogen atom; a protected hydroxyl group; a halogen atom; or an alkyl group, alkenyl group or alkynyl group containing 1 to 10 carbon atoms that may be substituted with a carbonyl group, an ether group, a protected hydroxyl group, a halogen atom or a carboxyl group, 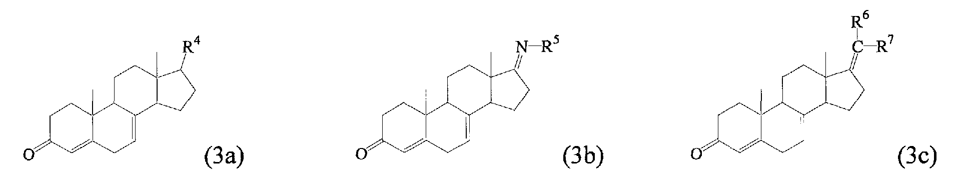 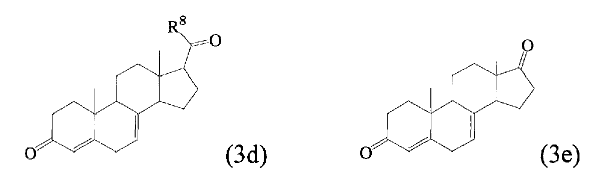 wherein each of R⁴ to R⁸ independently represents a hydrogen atom; a protected hydroxyl group; a halogen atom; or an alkyl group, alkenyl group or alkynyl group containing 1 to 10 carbon atoms that may be substituted with a carbonyl group, an ether group, a protected hydroxyl group, a halogen atom or a carboxyl group.
(15) A method for producing 3-oxo-4,6-diene steroid compound, which comprises isomerizing a 3-oxo-4,7-diene steroid compound represented by the following formula (3a), (3b), (3c), (3d) or (3e) to a compound represented by the following formula (4a), (4b), (4c), (4d) or (4e), using a base as a catalyst: 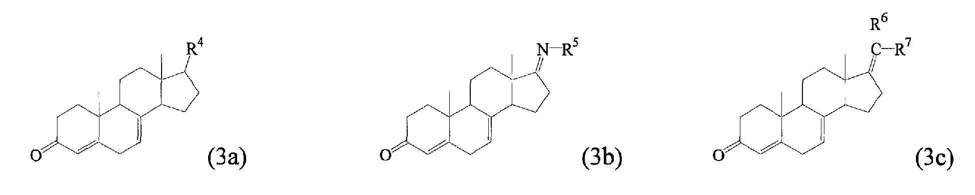 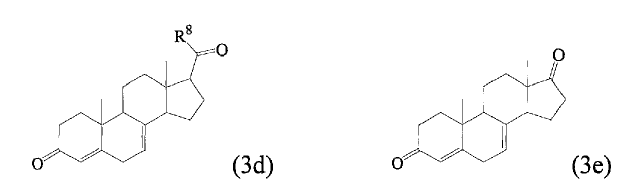 wherein each of R⁴ to R⁸ independently represents a hydrogen atom; a hydroxyl group; a protected hydroxyl group; a halogen atom; or an alkyl group, alkenyl group or alkynyl group containing 1 to 10 carbon atoms that may be substituted with a carbonyl group, an ether group, a hydroxyl group, a protected hydroxyl group, a halogen atom or a carboxyl group, 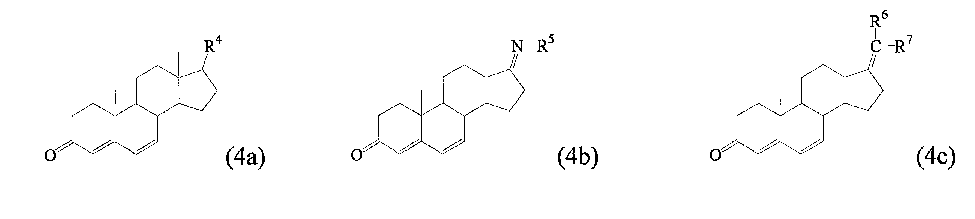 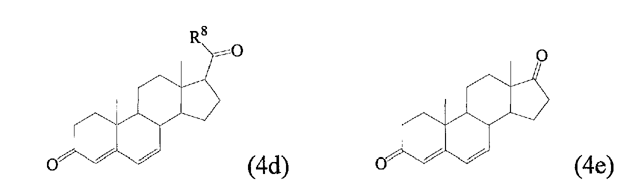 wherein each of R⁴ to R⁸ independently represents a hydrogen atom; a hydroxyl group; a protected hydroxyl group; a halogen atom; or an alkyl group, alkenyl group or alkynyl group containing 1 to 10 carbon atoms that may be substituted with a carbonyl group, an ether group, a hydroxyl group, a protected hydroxyl group, a halogen atom or a carboxyl group.
(16) A method for producing 5β-3,7-dioxocholanic acid represented by the following formula (8) or an ester derivative thereof: 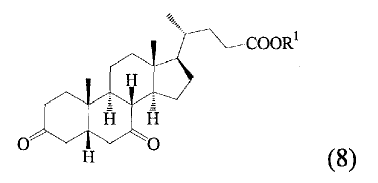 wherein R¹ represents a hydrogen atom or an alkyl group containing 1 to 6 carbon atoms,
   which comprises epoxidizing cholesta-4,6,24-trien-3-one represented by the following formula (4) to obtain 6,7:24,25-diepoxycholesta-4-en-3-one represented by the following formula (5), then hydrogenating it to obtain 5β-24,25-epoxycholesta-3-one-7-ol represented by the following formula (6), further hydrolyzing it to obtain 5β-cholesta-3-one-7,24,25-triol represented by the following formula (7), further oxidizing it, and in some cases, further esterifying it: 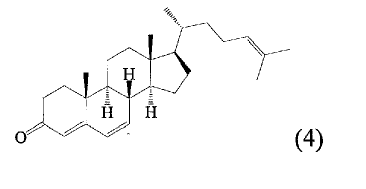 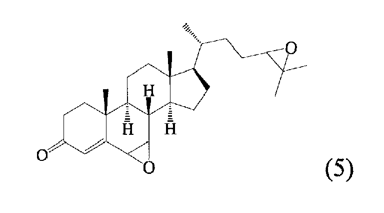 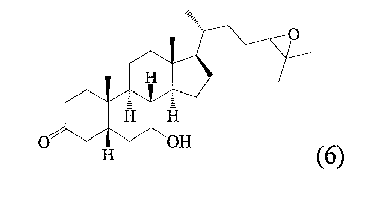 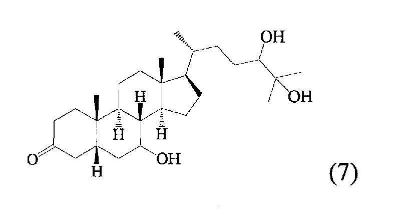
(17) A method for producing 5β-3,7-dioxocholanic acid represented by the following formula (8) or an ester derivative thereof: 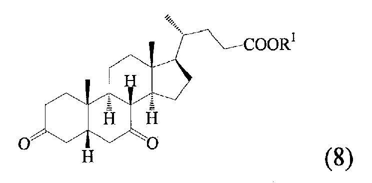 wherein R¹ represents a hydrogen atom or an alkyl group containing 1 to 6 carbon atoms,
   which comprises epoxidizing cholesta-4,6,24-trien-3-one represented by the following formula (4) to obtain 6,7:24,25-diepoxycholesta-4-en-3-one represented by the following formula (5), then hydrolyzing it to obtain 6,7-epoxycholesta-4-en-3-one-24,25-diol represented by the following formula (9), further hydrogenating it to obtain 5β-cholesta-3-one-7,24,25-triol represented by the following formula (7), further oxidizing it, and in some cases, further esterifying it: 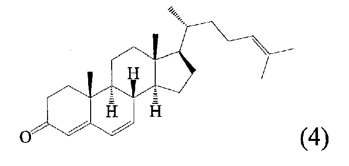 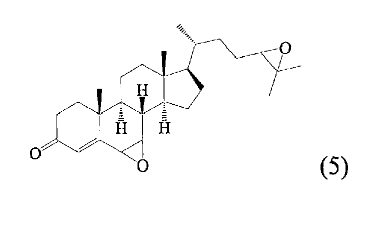 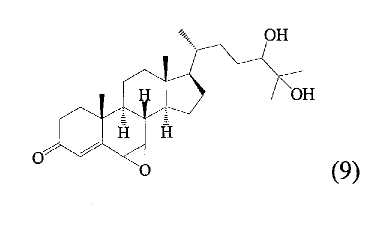 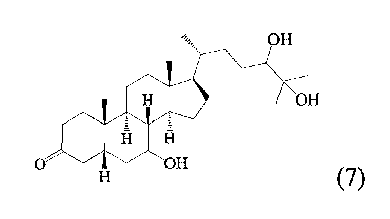
(18) A method for producing 5β-3,7-dioxocholanic acid represented by the following formula (8) or an ester derivative thereof: 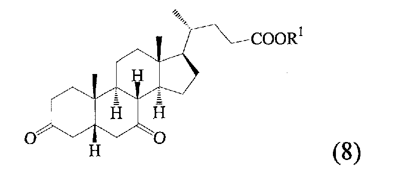 wherein R¹ represents a hydrogen atom or an alkyl group containing 1 to 6 carbon atoms,
   which comprises epoxidizing cholesta-4,6,24-trien-3-one represented by the following formula (4) to obtain 24,25-epoxycholesta-4,6-dien-3-one represented by the following formula (10), then hydrolyzing it to obtain cholesta-4,6-dien-3-one-24,25-diol represented by the following formula (11), then epoxidizing it to obtain 6,7-epoxycholesta-4-en-3-one-24,25-diol represented by the following formula (9), further hydrogenating it to obtain 5β-cholesta-3-one-7,24,25-triol represented by the following formula (7), further oxidizing it, and in some cases, further esterifying it: 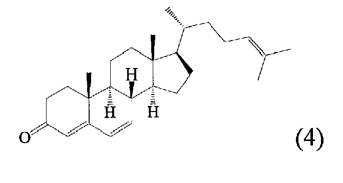 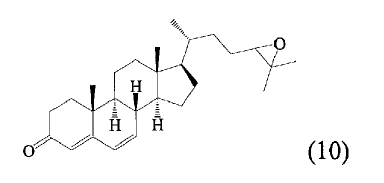 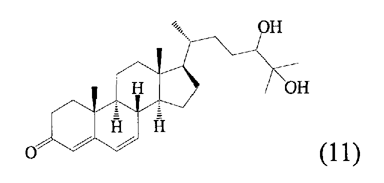 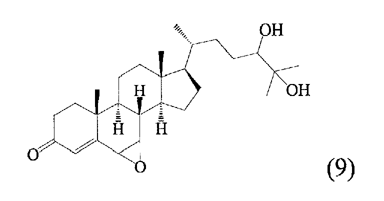 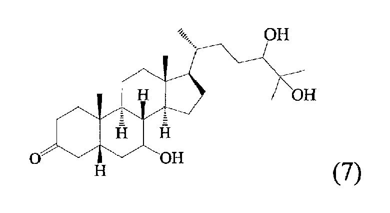
(19) A method for producing 5β-3,7-dioxocholanic acid represented by the following formula (8) or an ester derivative thereof: 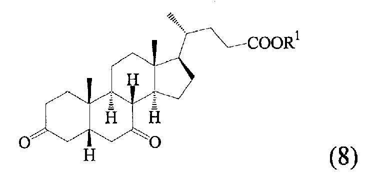 wherein R¹ represents a hydrogen atom or an alkyl group containing 1 to 6 carbon atoms,
   which comprises epoxidizing cholesta-4,6,24-trien-3-one represented by the following formula (4) to obtain 6,7:24,25-diepoxycholesta-4-en-3-one represented by the following formula (5), then hydrogenating it to obtain 5β-24,25-epoxycholesta-3-one-7-ol represented by the following formula (6), further oxidizing it to obtain 5β-24,25-epoxycholesta-3,7-dione represented by the following formula (12), then hydrolyzing it to obtain 5β-cholesta-3,7-dion-24,25-diol represented by the following formula (13), further oxidizing it, and in some cases, further esterifying it: 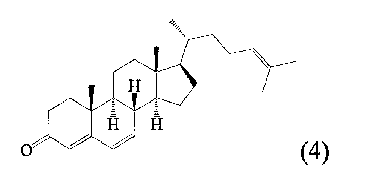 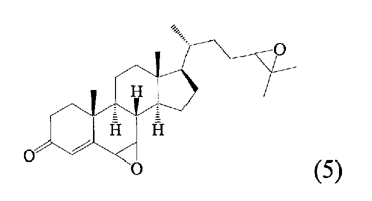 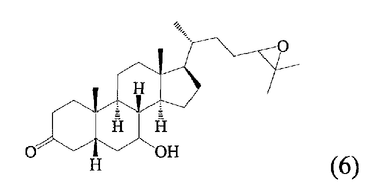 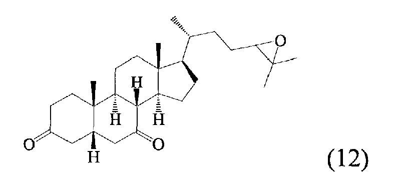 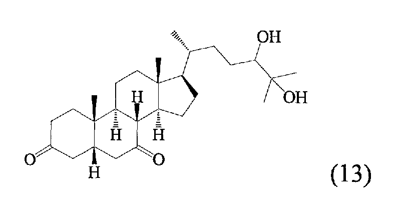
(20) A method for producing 5β-3,7-dioxocholanic acid represented by the following formula (8) or an ester derivative thereof: 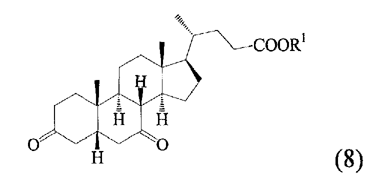 wherein R¹ represents a hydrogen atom or an alkyl group containing 1 to 6 carbon atoms,
   which comprises epoxidizing cholesta-4,6,24-trien-3-one represented by the following formula (4) to obtain 6,7:24,25-diepoxycholesta-4-en-3-one represented by the following formula (5), then hydrogenating it to obtain 5β-24,25-epoxycholesta-3-one-7-ol represented by the following formula (6), further reducing it to obtain 5β-24,25-epoxycholesta-3,7-diol represented by the following formula (14), further protecting a hydroxyl group thereof to obtain a 5β-24,25-epoxycholesta-3,7-dion-24,25-diol derivative represented by the following formula (15), then isomerizing the epoxy to obtain a 5β-cholesta-24-one-3,7-diol derivative represented by the following formula (16), then oxidizing it to obtain a 5β-3,7-dihydroxycholanic acid isopropyl ester derivative represented by the following formula (17), then performing deprotection and oxidation thereon, and in some cases, further esterifying it: 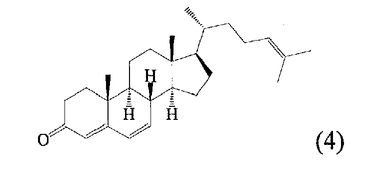 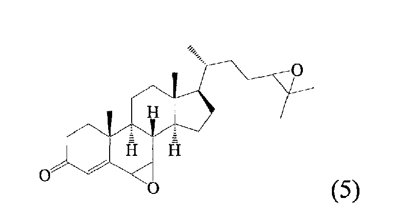 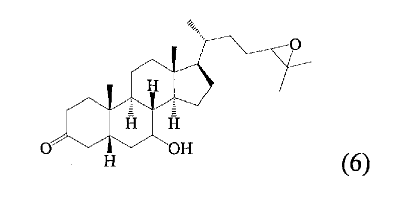 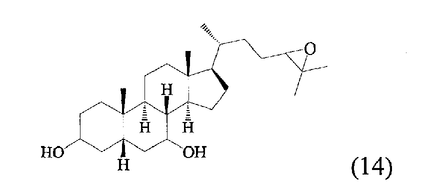 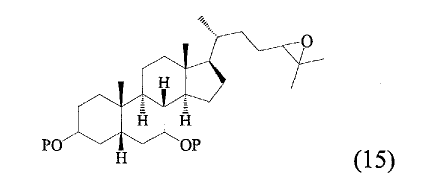 wherein P represents a protecting group for a hydroxyl group 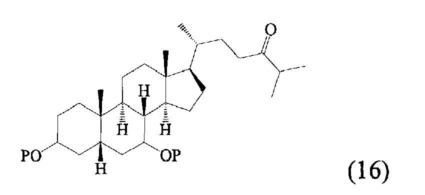 wherein P represents a protecting group for a hydroxyl group; 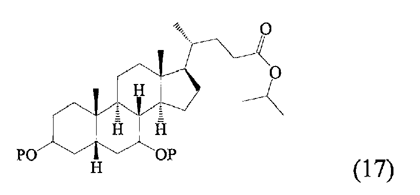 wherein P represents a protecting group for a hydroxyl group.
(21) A method for producing 5β-3,7-dioxocholanic acid represented by the following formula (8) or an ester derivative thereof: 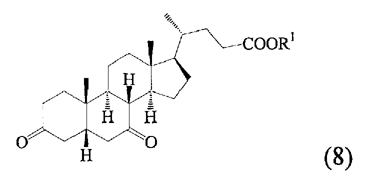 wherein R¹ represents a hydrogen atom or an alkyl group containing 1 to 6 carbon atoms,
   which comprises epoxidizing cholesta-4,6,24-trien-3-one represented by the following formula (4) to obtain 24,25-epoxycholesta-4,6-dien-3-one represented by the following formula (10), then isomerizing it to obtain cholesta-4,6-dien-3,24-dione represented by the following formula (18), then epoxidizing it to obtain 6,7-epoxycholesta-4-en-3,24-dione represented by the following formula (19), further hydrogenating it to obtain 5β-cholesta-3,24-dion-7-ol represented by the following formula (20), further reducing it to obtain 5β-cholesta-24-one-3,7-diol represented by the following formula (21), further protecting a hydroxyl group thereof to obtain a 5β-cholesta-24-one-3,7-diol derivative represented by the following formula (16), further oxidizing it to obtain a 5β-3,7-dihydroxycholanic acid isopropyl ester derivative represented by the following formula (17), then performing deprotection and oxidation thereon, and in some cases, further esterifying it: 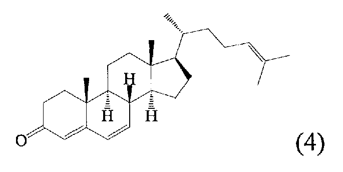 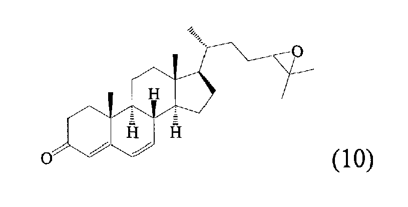 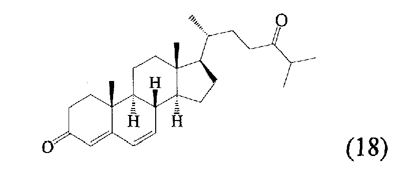 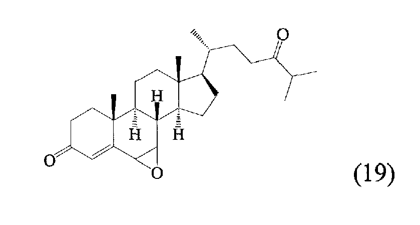 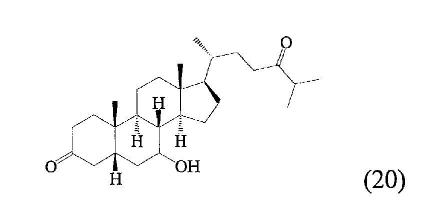 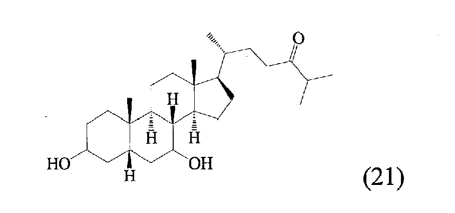 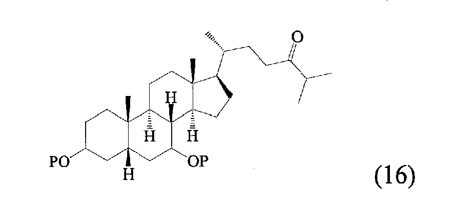 wherein P represents a protecting group for a hydroxyl group; 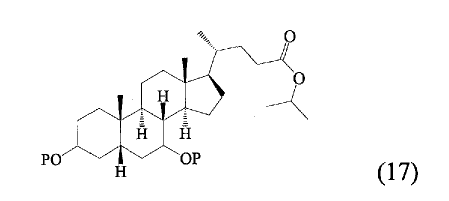 wherein P represents a protecting group for a hydroxyl group.
(22) A method for producing 5β-3,7-dioxocholanic acid represented by the following formula (8) or an ester derivative thereof: 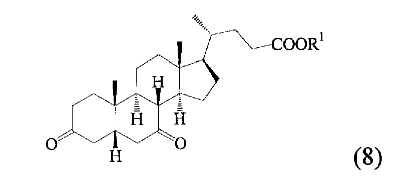 wherein R¹ represents a hydrogen atom or an alkyl group containing 1 to 6 carbon atoms,
   which comprises epoxidizing cholesta-4,6,24-trien-3-one represented by the following formula (4) to obtain 24,25-epoxycholesta-4,6-dien-3-one represented by the following formula (10), then hydrolyzing it to obtain cholesta-4,6-dien-3-one-24,25-diol represented by the following formula (11), then oxidizing it to obtain 3-oxochola-4,6-dien-24-al represented by the following formula (22), further oxidizing it to obtain 3-oxochola-4,6-dienoic acid represented by the following formula (23), further esterifying it to obtain a 3-oxochola-4,6-dienoic acid ester derivative represented by the following formula (24), further epoxidizing it to obtain a 6,7-epoxy-3-oxochola-4-enoic acid ester derivative represented by the following formula (25), further hydrogenating it, further hydrolyzing the ester thereof in some cases to obtain a 5β-7-hydroxy-3-ketocholanic acid (32d) derivative represented by the following formula (32d), and further oxidizing it: 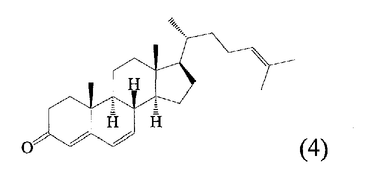 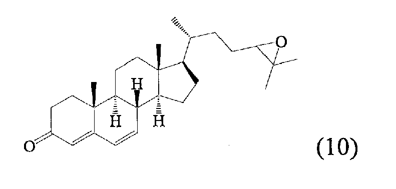 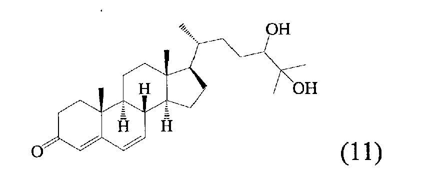 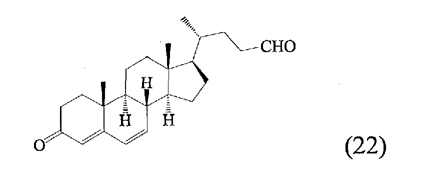 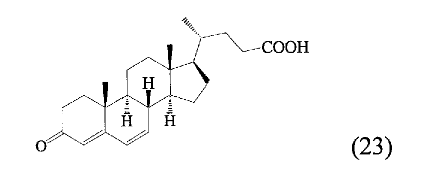 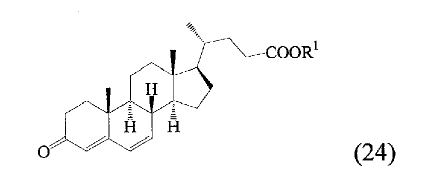 wherein R¹ represents an alkyl group containing 1 to 6 carbon atoms; 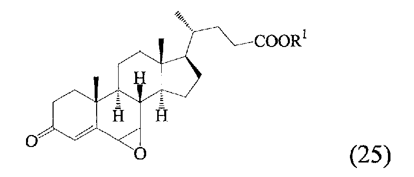 wherein R¹ represents an alkyl group containing 1 to 6 carbon atoms; 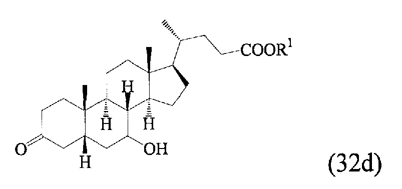 wherein R¹ represents a hydrogen atom or an alkyl group containing 1 to 6 carbon atoms.
(23) The method according to any one of (16) to (22) above, wherein an organic peroxide is used as an epoxidizing agent.
(24) The method according to (23) above, wherein percarboxylic acid represented by the formula A⁴CO₃H wherein A⁴ represents a hydrogen atom, an alkyl group containing 1 to 20 carbon atoms that may be substituted with a halogen atom, or an aryl group that may have a substituent, periminocarboxylic acid represented by the formula A⁵(C=NH)OOH wherein A⁵ represents a hydrogen atom, an alkyl group containing 1 to 20 carbon atoms that may be substituted with a halogen atom, or an aryl group that may have a substituent, or a dioxolane derivative represented by the following formula (26) is used as an organic peroxide: 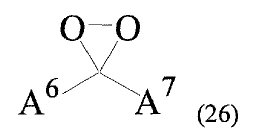 wherein each of A⁶ and A⁷ independently represents an alkyl group containing 1 to 20 carbon atoms that may be substituted with a halogen atom, or A⁶ and A⁷ may bind to each other to form a ring structure containing 3 to 8 carbon atoms.
(25) The method according to (24) above, wherein perbenzoic acid or 2-methylperbenzoic acid is used as an organic peroxide.
(26) The method according to (25) above, wherein water is added in the epoxidation reaction.
(27) The method according to (25) above, wherein the concentration of peracid and the concentration of carboxylic acid are maintained at 0.3 M or lower in the epoxidation reaction.
(28) The method according to (16), (17), (19) or (20) above, which comprises haloesterifying cholesta-4,6,24-trien-3-one represented by the following formula (4) to obtain a 7,24-dihalo-cholesta-4-en-3-one-6,25-diol diester represented by the following formula (27), and then performing the alkaline hydrolysis of the ester and cyclization to obtain 6,7:24,25-diepoxycholesta-4-en-3-one represented by the following formula (5): 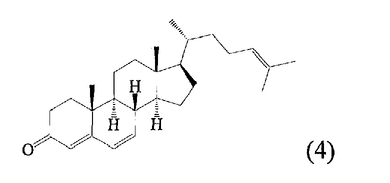 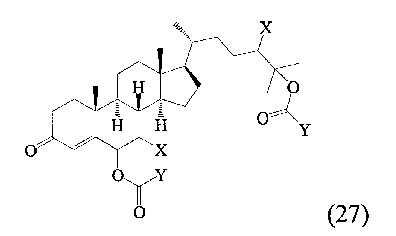 wherein X represents a halogen atom, and Y represents a hydrogen atom or an alkyl group containing 1 to 10 carbon atoms that may be substituted with a halogen atom; 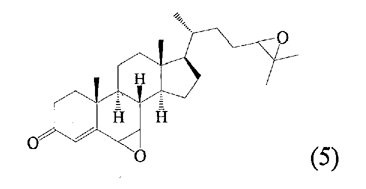
(29) The method according to (28) above, wherein organic carboxylic acid and a halocation generator represented by the formula Z-X wherein X represents a halogen atom, and Z represents succinimide, phthalimide, acetamide, hydantoin, or a t-butoxy group are used loesterification agent.
(30) The method according to any one of (16) to (22) and (28) above, wherein the hydrogenation reaction is carried out in the presence of a noble metal catalyst.
(31) The method according to (30) above, wherein powdery palladium, or one or two or more types of metal palladium selected from the group consisting of activated carbon-supported palladium, aluminum oxide-supported palladium, barium carbonate-supported palladium, barium sulfate-supported palladium and calcium carbonate-supported palladium with a palladium content of 0.5% to 50% by weight, is used as a noble metal catalyst.
(32) The method according to (30) or (31) above, wherein a base is allowed to coexist in the hydrogenation reaction in the presence of a noble metal catalyst.
(33) The method according to (32) above, wherein amine is used as a base.
(34) The method according to any one of (16) to (19), (22) and (28) above, wherein the epoxy hydrolysis reaction is carried out in the presence of silica gel or protonic acid.
(35) The method according to (34) above, wherein hydrochloric acid, sulfuric acid, nitric acid, perchloric acid, phosphoric acid, phosphorous acid, hypophosphorous acid, organic carboxylic acid or organic sulfonic acid is used as protonic acid.
(36) The method according to any one of (16) to (22) and (28), wherein halogen acid or a salt thereof, molecular halogen, permanganic acid, dichromic acid, or chromic acid is used as an oxidizing agent in the oxidation reaction.
(37) The method according to (20) or (21) above, wherein Lewis acid, protonic acid, or a salt thereof is used as a catalyst in the isomerization reaction of the side chain 24,25-epoxy group to 24-ketone.
(38) The method according to (37) above, wherein zinc chloride (II), zinc bromide (II) or zinc iodide (II) is used as Lewis acid.
(39) The method according to (37) above, wherein halogen acid or a salt thereof is used as protonic acid or a salt thereof.
(40) The method according to (20) or (21) above, wherein an organic peroxide is used in the oxidation reaction of the side chain ketone at position 24 to an isopropyl ester.
(41) The method according to (40) above, wherein monoperphthalic acid or m-chloroperbenzoic acid is used as an organic peroxide.
(42) The method according to (20) or (21) above, wherein hydrogen is used in the presence of a transition metal catalyst or hydride reduction is carried out as a means for reducing the ketone at position 3.
(43) The method according to (42) above, wherein platinum oxide or Raney nickel is used as a transition metal catalyst.
(44) The method according to any one of (16) to (22) and (28) above, wherein a compound obtained by isomerization of cholesta-4,7,24-trien-3-one represented by the following formula (3) is used as cholesta-4,6,24-trien-3-one represented by the following formula (4): 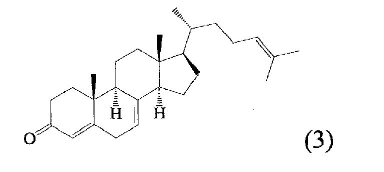 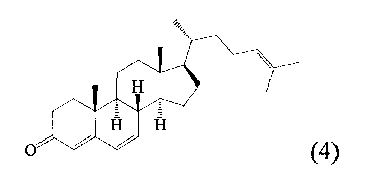
(45) The method according to (44) above, wherein a compound obtained by oxidation of cholesta-5,7,24-trien-3β-ol represented by the following formula (2) is used as cholesta-4,7,24-trien-3-one represented by the following formula (3): 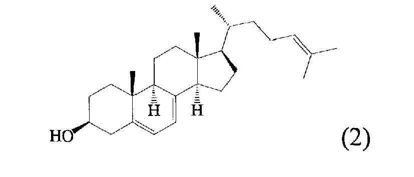 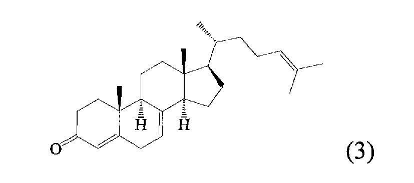
(46) A method for producing a vicinal diol compound represented by the following formula (29): 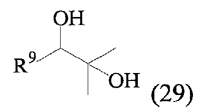 wherein R⁹ represents an alkyl group, alkenyl group or alkynyl group containing 1 to 20 carbon atoms that may be substituted with a hydroxyl group, a protected hydroxyl group, a carboxyl group, an ester group, a carbonyl group, a cyano group, an amino group or a halogen atom,
   which comprises hydrolyzing an epoxy compound represented by the following formula (28), using silica gel as a catalyst: 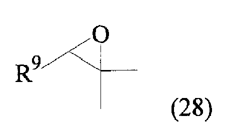 wherein R⁹ represents an alkyl group, alkenyl group or alkynyl group containing 1 to 20 carbon atoms that may be substituted with a hydroxyl group, a protected hydroxyl group, a carboxyl group, an ester group, a carbonyl group, a cyano group, an amino group or a halogen atom.
(47) A method for producing a vicinal diol compound represented by the following formula (31): 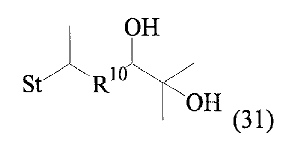 wherein St represents a steroid skeleton consisting of ring A, ring B, ring C, and ring D, wherein with regard to the aforementioned steroid skeleton, (1) it binds to a side chain shown in the formula at position C 17, (2) it may have a hydroxyl group, a protected hydroxyl group, a keto group, or an epoxy group on the ring A, ring B, ring C, and ring D, (3) C-C bonds at one or more positions selected from the group consisting of positions C1 to C8 may have double bonds, and (4) one or more positions selected from the group consisting of positions C4, C10, C13 and C14 may be substituted with methyl groups; and R¹⁰ represents an alkylene group, alkenylene group or alkynylene group containing 1 to 20 carbon atoms that may be substituted with a hydroxyl group, a protected hydroxyl group, a carboxyl group, an ester group, a carbonyl group, a cyano group, an amino group or a halogen atom,
   which comprises hydrolyzing a steroid epoxy compound represented by the following formula (30), using silica gel as a catalyst: 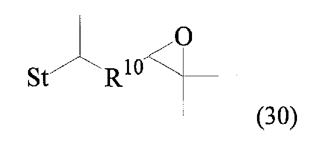 wherein St represents a steroid skeleton consisting of ring A, ring B, ring C, and ring D, wherein with regard to the aforementioned steroid skeleton, (1) it binds to a side chain shown in the formula at position C17, (2) it may have a hydroxyl group, a protected hydroxyl group, a keto group, or an epoxy group on the ring A, ring B, ring C, and ring D, (3) C-C bonds at one or more positions selected from the group consisting of positions C1 to C8 may have double bonds, and (4) one or more positions selected from the group consisting of positions C4, C10, C13 and C14 may be substituted with methyl groups; and R¹⁰ represents an alkylene group, alkenylene group or alkynylene group containing 1 to 20 carbon atoms that may be substituted with a hydroxyl group, a protected hydroxyl group, a carboxyl group, an ester group, a carbonyl group, a cyano group, an amino group or a halogen atom.
(48) 5β-24,25-epoxycholesta-3,7-diol represented by the following formula (14): 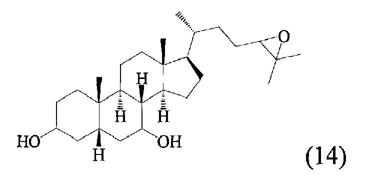
(49) A 5β-24,25-epoxycholesta-3,7-diol derivative represented by the following formula (15): 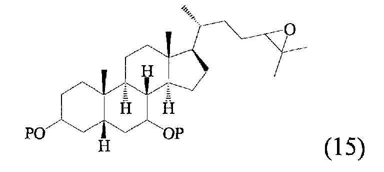 wherein P represents a protecting group for a hydroxyl group.
(50) A 5β-cholesta-24-one-3,7-diol derivative represented by the following formula (16): 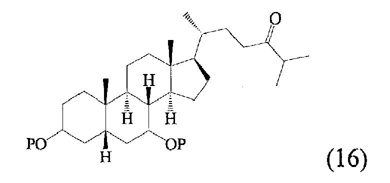 wherein P represents a protecting group for a hydroxyl group.
(51) Cholesta-4,6-dien-3,24-dione represented by the following formula (18): 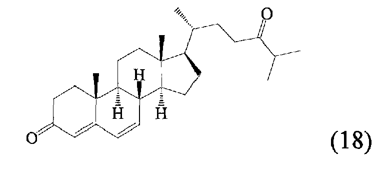
(52) 6,7-epoxycholesta-4-en-3,24-dione represented by the following formula (19): 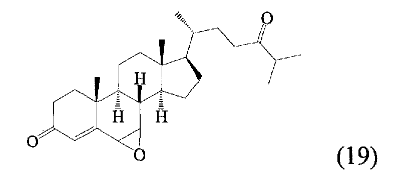
(53) 5β-cholesta-3,24-dion-7-ol represented by the following formula (20): 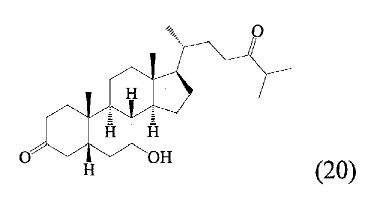
(54) 5β-cholesta-24-one-3,7-diol represented by the following formula (21): 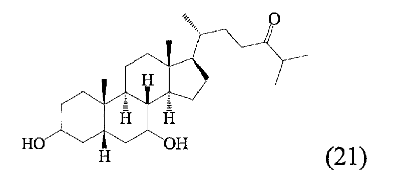
(55) 3-oxochola-4,6-dien-24-al represented by the following formula (22): 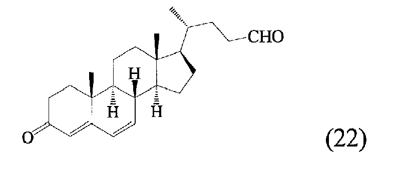
(56) A method for producing ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (32a), (32b), (32c) or (32d), or an ester derivative thereof: 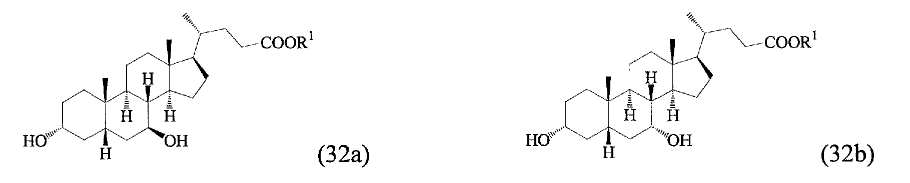 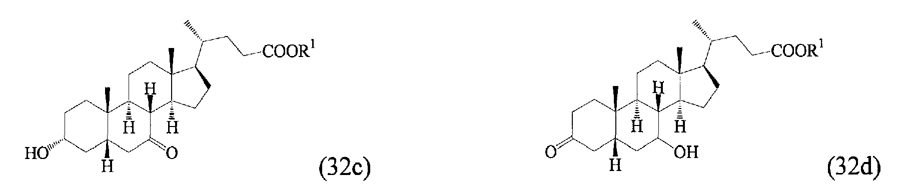 wherein R¹ represents a hydrogen atom or an alkyl group containing 1 to 6 carbon atoms,
   which comprises reducing 5β-3,7-dioxocholanic acid represented by the following formula (8) or an ester derivative thereof, which is produced by the method according to any one of (16) to (22), (28), (44) and (45) above, and then, in some cases, reoxidizing it: 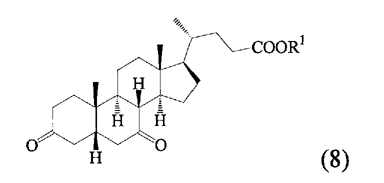 wherein R¹ represents a hydrogen atom or an alkyl group containing 1 to 6 carbon atoms.

### BEST MODE FOR CARRYING OUT THE INVENTION

The embodiments of the present invention will be described more in detail below.

The following figure of reaction shows the entire scheme comprising the reaction steps of the present invention.

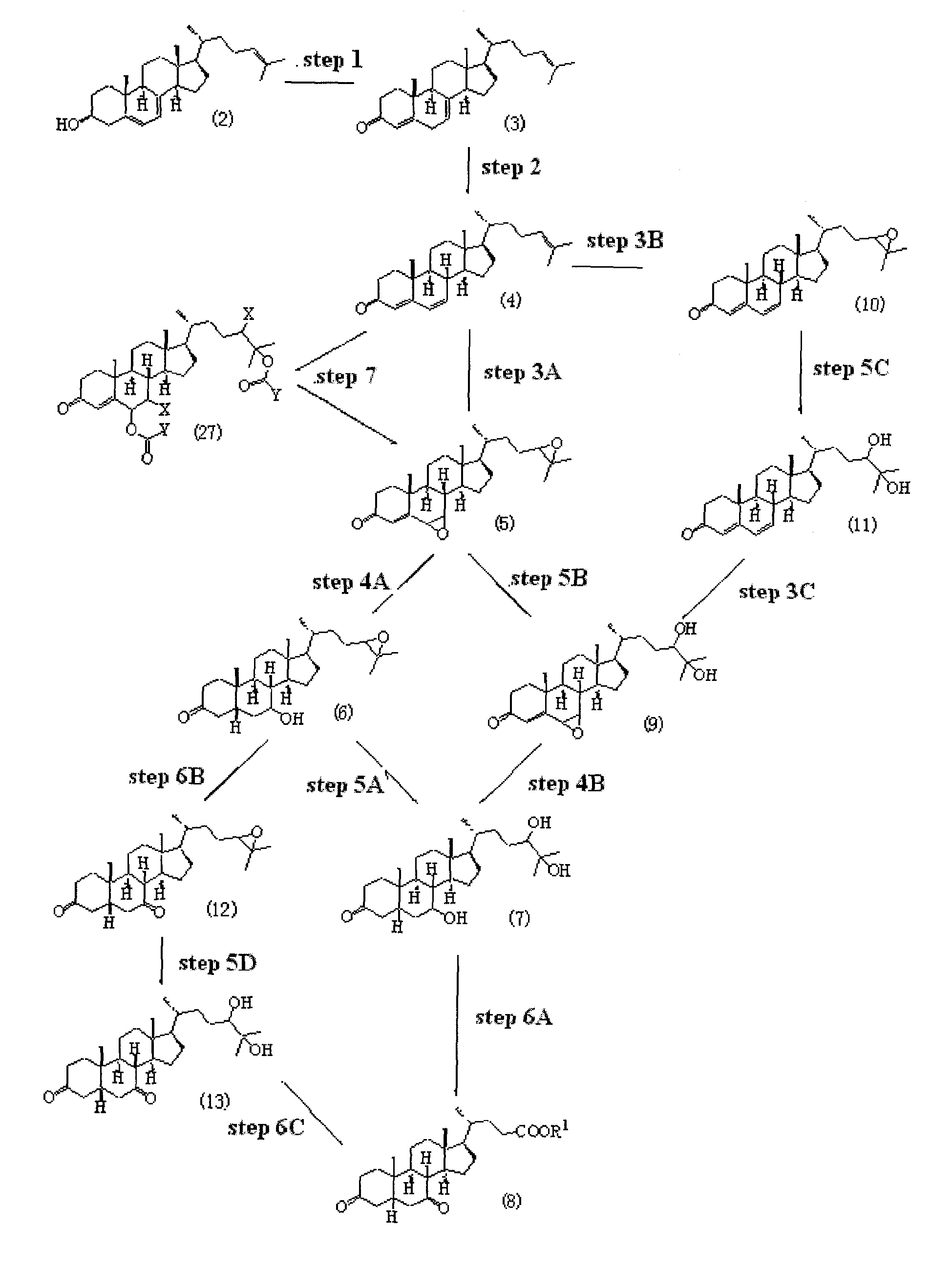

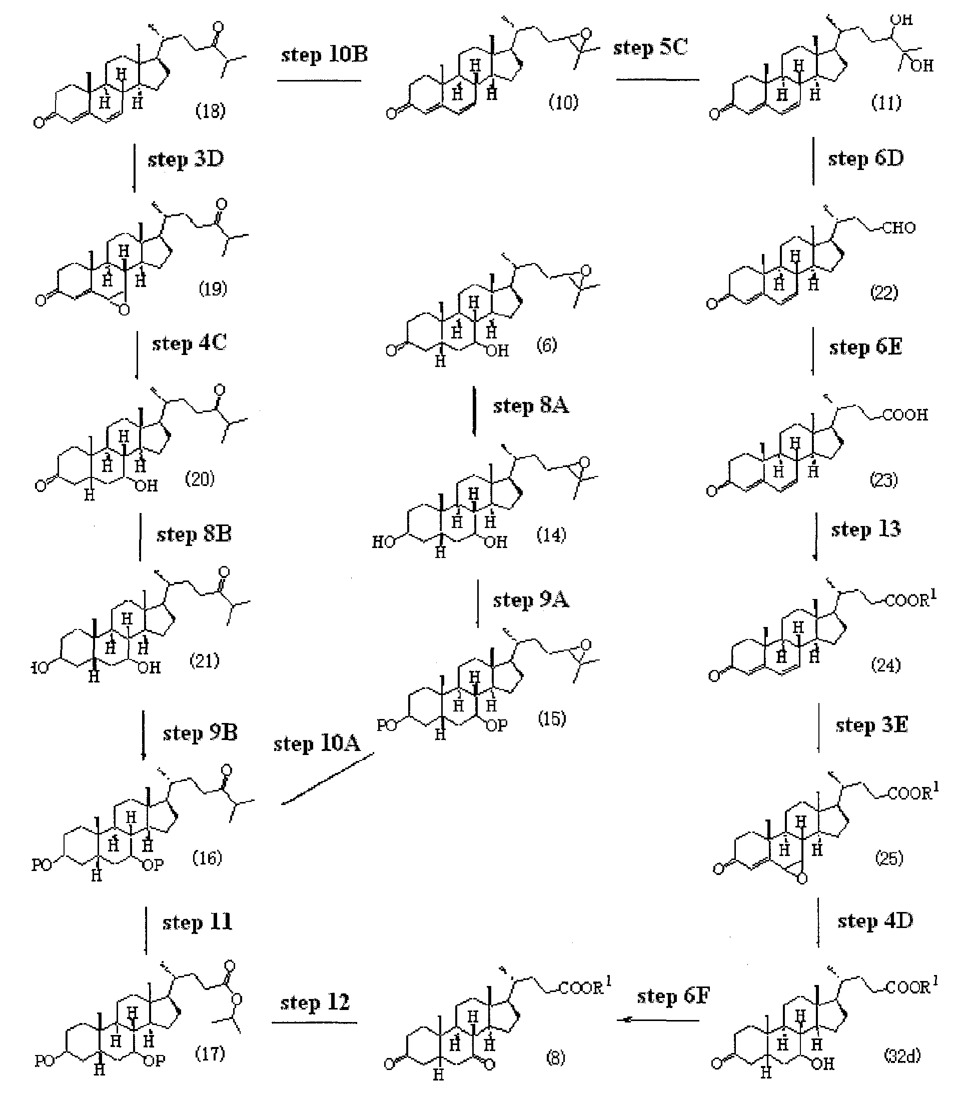

Hereinafter, the present invention will be described with reference to compound Nos. (2) to (25), (27) and (32d) as shown in the above scheme.

Cholesta-5,7,24-trien-3β-ol used as a raw material in the production method of the present invention is a known substance. Such cholesta-5,7,24-trien-3β-ol can be produced by modifying Eumycetes that produce ergosterol via zymosterol in a metabolic engineering manner, so as to produce a mutant strain, culturing the mutant strain, and then collecting cholesta-5,7,24-trien-3β-ol from the culture, for example, see the methods described in JP Patent Publication (Kokai) No. 5-192184 A (1993) and JP Patent Publication (Kokai) No. 2004-141125 A for the details of this production method.

### <Step 1> Step of producing cholesta-4,7,24-trien-3-one represented by the following formula (3) from cholesta-5,7,24-trien-3β-ol represented by the following formula (2)

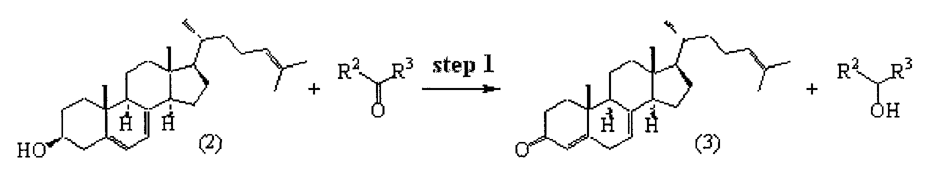

As is clear from the above reaction formula, step 1 of the present invention involves a method of simultaneously carrying out oxidation of a hydroxyl group at position 3 of cholesta-5,7,24-trien-3β-ol (hereinafter abbreviated as "compound 2"at times) and isomerization of a double bond at position 5 to position 4. This has been known as a method of converting ergosterol to ergosteron, and it is called "Oppenauer oxidation."

This oxidation reaction is carried out using a metal alkoxide as a catalyst and using a ketone compound as a hydrogen acceptor. Examples of a metal alkoxide may include aluminum isopropoxide, aluminum-t-butoxide, magnesium ethoxide, magnesium propoxide, and titanium propoxide. A preferred example of a ketone compound may be a compound represented by the formula R ²(C=O)R ³ wherein each of R ² and R ³ independently represents a chain or cyclic alkyl group containing 1 to 10 carbon atoms, or R ² and R ³ may bind to each other, so as to form a cyclic structure containing 3 to 8 carbon atoms. Specific examples of such a ketone compound may include: chain ketones such as acetone or methyl isobutyl ketone; and cyclic ketones such as cyclohexanone or cyclopentanone. As a particularly preferred catalyst, aluminum isopropoxide is used. As a particularly preferred ketone compound, cyclohexanone or methyl isobutyl ketone is used. Such a catalyst is used at a molar ratio generally between 0.1 : 1 and 20 : 1, and preferably between 0.2 : 1 and 0.5 : 1, with respect to the "compound 2." The reaction hardly progresses with no catalysts. On the other hand, if the amount of a catalyst is too large, side reactions frequently occur. Thus, the amount of a catalyst is determined within the aforementioned range. A hydrogen acceptor is used at a molar ratio generally between 1 : 1 and 50 : 1, and preferably between 2 : 1 and 10 : 1, with respect to the "compound 2."

The present reaction can be carried out with no solvents. However, a solvent may also be used. Examples of a solvent used herein may include: aliphatic hydrocarbons such as hexane; aromatic hydrocarbons such as toluene; halogen solvents such as dichloromethane; ethers such as diethyl ether or tetrahydrofuran; and aprotic polar solvents such as dimethyl sulfoxide or dimethyl formamide. Aprotic solvents are preferably used, and toluene or heptane is more preferably used. The reaction temperature is set generally between 90°C and 130°C, and preferably between 100°C and 120°C. The reaction time is approximately between 1 and 3 hours. After completion of the reaction, the reaction product is cooled to room temperature, and water is then added thereto, so as to inactivate the catalyst. The deposited precipitate is filtrated, and the filtrate is then concentrated, so as to obtain a product of interest, cholesta-4,7,24-trien-3-one (compound 3). The compound 3 can be isolated and purified by methods such as silica gel column chromatography or crystallization.

In addition, from the viewpoint of the stability of a product, the present reaction is preferably carried out while oxygen is blocked. For example, a solvent or a ketone compound has previously been subjected to a deoxygenation treatment, and the reaction is then carried out in a nitrogen or argon atmosphere. Specifically, a method of deaerating a solvent and a ketone compound under a reduced pressure for nitrogen substitution, or a method of heating to reflux in a nitrogen atmosphere for nitrogen substitution, is applied, and thereafter, the reaction is carried out in a nitrogen atmosphere.

The isomerization reaction of the present invention can also be applied to analogous compounds of the compound 2, that are, 3-hydroxy-5,7-diene steroid compounds represented by the following formulas (2a), (2b), (2c), (2d) and (2e): 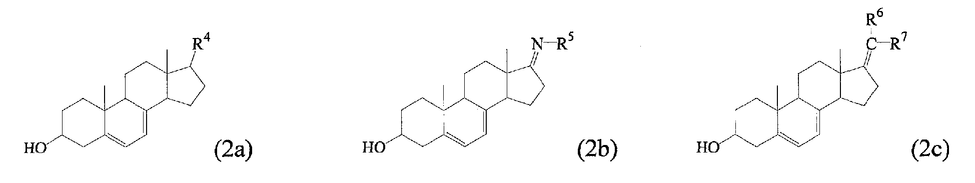 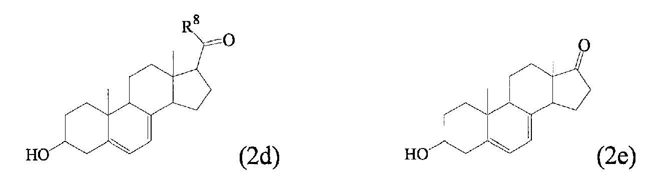 wherein each of R ⁴ to R ⁸ independently represents a hydrogen atom; a protected hydroxyl group;
or a halogen atom; or an alkyl, alkenyl or alkynyl group containing 1 to 10 carbon atoms that may be substituted with a carbonyl group, an ether group, a protected hydroxyl group, a halogen atom or a carboxyl group.

That is to say, these 3-hydroxy-5,7-diene steroid compounds are allowed to react in the presence of a ketone compound and a metal alkoxide, while oxygen is blocked, so that they are oxidized to compounds represented by the following formulas (3a), (3b), (3c), (3d) and (3e), respectively, at high yields: 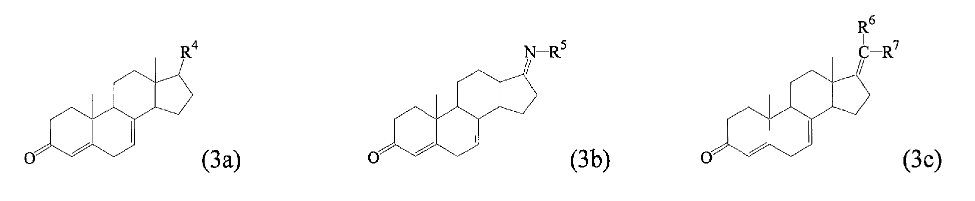 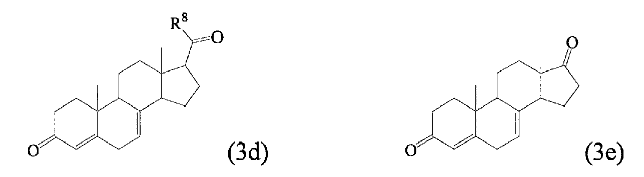 wherein each of R ⁴ to R ⁸ independently represents a hydrogen atom; a protected hydroxyl group; or a halogen atom; or an alkyl, alkenyl or alkynyl group containing 1 to 10 carbon atoms that may be substituted with a carbonyl group, an ether group, a protected hydroxyl group, a halogen atom or a carboxyl group.

Specific examples of the aforementioned compound (2a) may include cholesta-5,7,24-trien-3β-ol and ergosterol.

### <Step 2> Step of producing cholesta-4,6,24-trien-3-one represented by the following formula (4) from cholesta-4,7,24-trien-3-one represented by the following formula 3

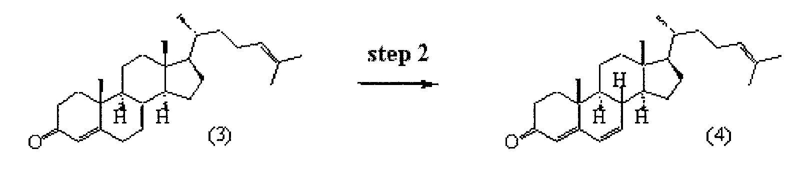

As is clear from the above reaction formula, step 2 of the present invention involves a reaction of isomerizing a double bond at position 7 of cholesta-4,7,24-trien-3-one (hereinafter abbreviated as "compound 3"" at times) to position 6.

The isomerization reaction is carried out using a basic compound as a catalyst. Examples of such a basic compound may include alkaline metal hydroxide, alkaline-earth metal hydroxide, alkaline metal carbonate, alkaline-earth metal carbonate, alkaline metal hydrogencarbonate, alkaline metal acetate, alkaline-earth metal acetate, alkaline metal alkoxide, and alkaline-earth metal alkoxide. Of these, alkaline metal hydroxide is preferably used, and potassium hydroxide and sodium hydroxide are more preferably used. Such a basic compound is used at a molar ratio generally between 1 : 1 and 20 : 1, and preferably between 2 : 1 and 10 : 1, with respect to the "compound 3."

The type of a reaction solvent used herein is not particularly limited. Examples of a reaction solvent may include: aliphatic hydrocarbons such as hexane; aromatic hydrocarbons such as toluene; halogen solvents such as dichloromethane; ethers such as diethyl ether or tetrahydrofuran; alcohols such as methanol or ethanol; and aprotic polar solvents such as dimethyl sulfoxide or dimethyl formamide. Preferably, methanol is used. The reaction is carried out generally between 40°C and 80°C, and preferably between 50°C and 70°C, for approximately 5 to 10 hours. After completion of the reaction for a certain period of time, acid is added to the reaction solution to neutralize the base used as a catalyst, and the reaction is terminated. The type of acid used herein is not particularly limited. Examples of acid used herein may include: mineral acids such as hydrochloric acid or sulfuric acid; organic carboxylic acids such as formic acid or acetic acid; and organic sulfonic acids such as p-toluenesulfonic acid. After completion of neutralization, the solvent is distilled away under a reduced pressure, so as to obtain a product of interest, cholesta-4,6,24-trien-3-one (hereinafter abbreviated as "compound 4" at times). According to circumstances, water may be added to the reaction solution obtained after the neutralization treatment, so as to crystallize the compound 4. Otherwise, an organic solvent may be added thereto for extraction, and the organic solvent layer may be washed with water, dried, and concentrated, followed by isolation and purification by silica gel column chromatography or other methods.

In addition, from the viewpoint of the stability of a substrate, the present reaction is preferably carried out while oxygen is blocked. For example, a solvent has previously been subjected to a deoxygenation treatment, and the reaction is then carried out in a nitrogen or argon atmosphere. Specifically, a method of deaerating a solvent under a reduced pressure for nitrogen substitution, or a method of heating to reflux in a nitrogen atmosphere for nitrogen substitution, is first applied, and thereafter, the reaction is carried out in a nitrogen atmosphere.

The isomerization reaction of the present invention can also be applied to analogous compounds of the compound 3, that are, 3-oxo-4,7-diene steroid compounds represented by the following formulas (3a), (3b), (3c), (3d) and (3e): 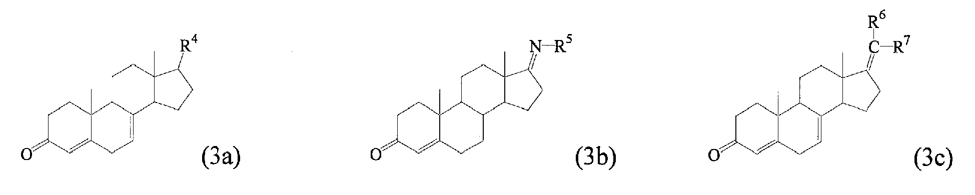 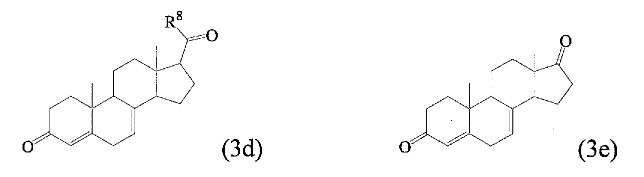 wherein each of R ⁴ to R ⁸ independently represents a hydrogen atom; a hydroxyl group; a protected hydroxyl group; or a halogen atom; or an alkyl, alkenyl or alkynyl group containing 1 to 10 carbon atoms that may be substituted with a carbonyl group, an ether group, a hydroxyl group, a protected hydroxyl group, a halogen atom or a carboxyl group.

That is to say, these 3-oxo-4,7-diene steroid compounds can be isomerized to 3-oxo-4,6-diene steroid compounds represented by the following formulas (4a), (4b), (4c), (4d), and (4e), respectively, using a base as a catalyst: 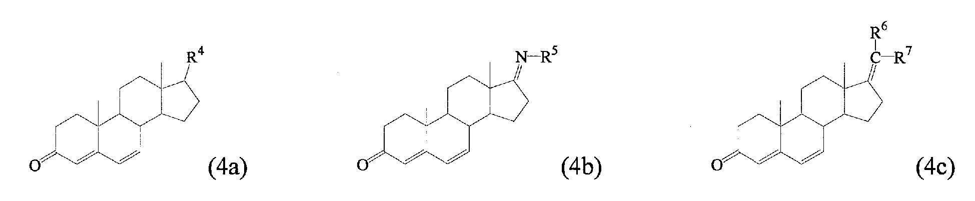 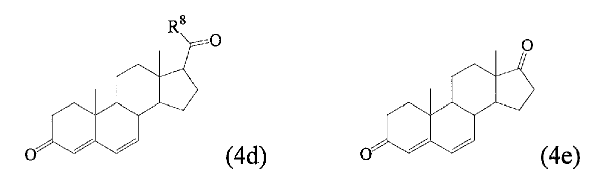 wherein each of R ⁴ to R ⁸ independently represents a hydrogen atom; a hydroxyl group; a protected hydroxyl group; or a halogen atom; or an alkyl, alkenyl or alkynyl group containing 1 to 10 carbon atoms that may be substituted with a carbonyl group, an ether group, a hydroxyl group, a protected hydroxyl group, a halogen atom or a carboxyl group.

An example of the aforementioned protected hydroxyl group may be a hydroxyl group protected with an ether-type protecting group.

### <Step 3A> Step of producing 6,7:24,25-diepoxycholesta-4-en-3-one represented by the following formula (5) from cholesta-4,6,24-trien-3-one represented by the following formula (4)

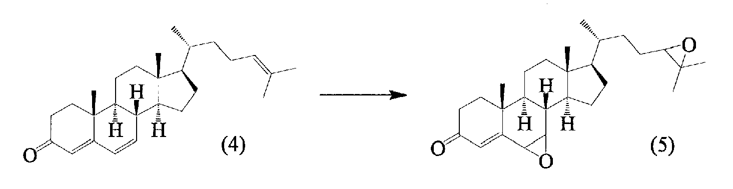

As is clear from the above reaction formula, step 3A of the present invention involves a reaction of epoxidizing double bonds at position 6 and at position 24 of cholesta-4,6,24-trien-3-one (hereinafter abbreviated as "compound 4" at times). As an epoxidizing agent, an organic peroxide is generally used.

Examples of an organic peroxide used herein may include: percarboxylic acid represented by the formula A ⁴ CO ₃ H wherein A ⁴ represents a hydrogen atom, an alkyl group containing 1 to 20 carbon atoms that may be substituted with a halogen atom, or an aryl group that may have a substituent; periminocarboxylic acid represented by the formula A⁵ (C=NH)OOH wherein A⁵ represents a hydrogen atom, an alkyl group containing 1 to 20 carbon atoms that may be substituted with a halogen atom, or an aryl group that may have a substituent; and a dioxirane derivative represented by the following formula (26): 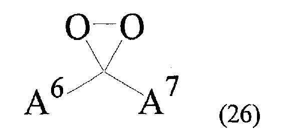 wherein each of A ⁶ and A ⁷ independently represents an alkyl group containing 1 to 20 carbon atoms that may be substituted with a halogen atom, or A ⁶ and A ⁷ may bind to each other, so as to form a cyclic structure containing 3 to 8 carbon atoms. Specific examples of percarboxylic acid may include performic acid, peracetic acid, perpropionic acid, perbenzoic acid, 2-methylperbenzoic acid, and monoperphthalic acid. A specific example of periminocarboxylic acid may be CH ₃ C(=NH)OOH (peroxyacetimidic acid). Specific examples of a dioxirane derivative may include dimethyldioxirane (acetone peroxide) and methyl ethyl dioxirane (methyl ethyl ketone peroxide).

From the viewpoint of reaction selectivity, perbenzoic acid and 2-methylperbenzoic acid are particularly preferably used. Such an organic peroxide is used at an equivalent molar ratio generally between 2 : 1 and 10 : 1, and preferably between 2 : 1 and 3 : 1, with respect to the "compound 4." The temperature applied for epoxidation is set generally between 0°C and 100°C, and preferably between 40°C and 90°C.

The type of a reaction solvent used herein is not particularly limited. Examples of a reaction solvent may include: aliphatic hydrocarbons such as hexane; aromatic hydrocarbons such as toluene; halogen solvents such as dichloromethane; ethers such as diethyl ether or tetrahydrofuran; esters such as ethyl acetate or butyl acetate; nitriles such as acetonitrile; alcohols such as methanol or ethanol; aprotic polar solvents such as dimethyl sulfoxide or dimethyl formamide; and water. Of these, esters are preferably used. When percarboxylic acid is used as an oxidizing agent, reaction selectivity is significantly improved by adding water, or by maintaining the concentration of peracid and that of carboxylic acid at low in a reaction solution.

The obtained 6,7:24,25-diepoxycholesta-4-en-3-one (compound 5) can be isolated and purified by methods such as silica gel column chromatography or crystallization.

### <Step 3B> Step of producing 24,25-epoxycholesta-4,6-dien-3-one represented by the following formula (10) from cholesta-4,6,24-trien-3-one represented by the following formula (4)

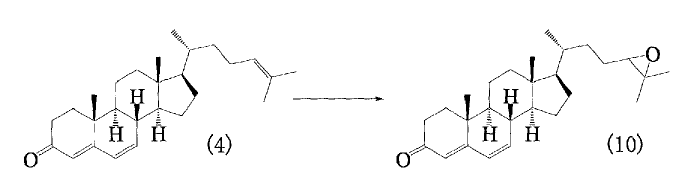

As is clear from the above reaction formula, step 3B of the present invention involves a reaction of epoxidizing a double bond at position 24 of cholesta-4,6,24-trien-3-one (compound 4). In the epoxidation reaction of step 3A, the double bond at position 24 is preferentially epoxidized, and the double bond at position 6 is then epoxidized. Thus, if the amount of an epoxidizing agent used is small, or if the reaction temperature is low, only the double bond at position 24 represented by formula (4) is epoxidized, so as to obtain a monoepoxy compound represented by formula (10). Accordingly, it may be adequate that an epoxidizing agent be used at a molar ratio of 1 : 1 with respect to the "compound 4," and that the reaction be carried out around room temperature for 1 to 3 hours. Other reaction conditions are the same as those in step 3A.

### <Step 3C> Step of producing 6,7-epoxycholesta-4-en-3-one-24,25-diol represented by the following formula (9) from cholesta-4,6-dien-3-one-24,25-diol represented by the following formula (11)

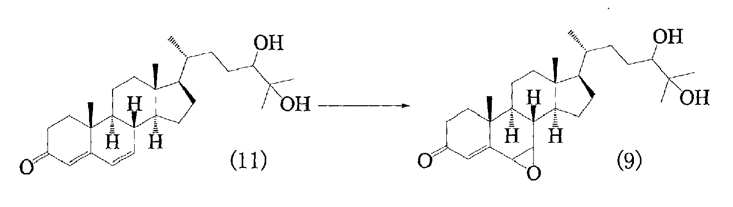

As is clear from the above reaction formula, step 3C of the present invention involves a reaction of epoxidizing a double bond at position 6 of cholesta-4,6-dien-3-one-24,25-diol (compound 11).

The present reaction is the same as that in the aforementioned step 3 A in that it is a reaction of epoxidizing a double bond at position 6 of a 3-keto-4,6-diene steroid compound. Accordingly, the same oxidizing agent and solvent as those in the case of step 3A can be used. Reaction conditions are also the same as those in step 3A. An epoxidizing agent is preferably used at an equivalent molar ratio between 1 : 1 and 2 : 1 with respect to the "compound 11."

### <Step 7> Step of producing 6,7:24,25-diepoxycholesta-4-en-3-one represented by the following formula (5) from cholesta-4,6,24-trien-3-one represented by the following formula (4)

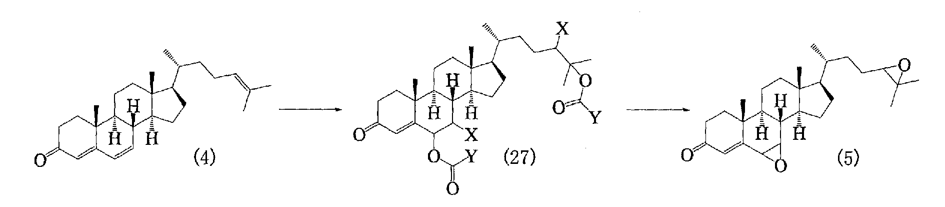

wherein X represents a halogen atom; and Y represents a hydrogen atom, or an alkyl group containing 1 to 10 carbon atoms that may be substituted with a halogen atom.

As shown in the above formula, as an alternative method of obtaining the aforementioned diepoxy compound (5), it is also possible to apply a method involving the combination of the hydrolysis of an ester with cyclization to an epoxy group, wherein the reaction is performed via a haloester. For such halo-esterification, organic carboxylic acid and a halocation generator represented by the formula Z-X wherein X represents a halogen atom, and Z represents succinimide, phthalimide, acetamide, hydantoin, or a t-butoxy group are used. Preferably, formic acid is used as organic carboxylic acid, and t-butyl hypochloride is used as a halocation generator.

Such organic carboxylic acid is used at a molar ratio generally between 2 : 1 and 50 : 1, and preferably between 2 : 1 and 10 : 1, with respect to the "compound 4." Such a halocation generator is used at a molar ratio generally between 2 : 1 and 10 : 1, and preferably between 2 : 1 and 5:1, with respect to the "compound 4."

The reaction temperature is generally between 0°C and 50°C, and preferably between 0°C and 30°C. Examples of a reaction solvent used herein may include: aliphatic hydrocarbons such as hexane; aromatic hydrocarbons such as toluene; halogen solvents such as dichloromethane; ethers such as diethyl ether or tetrahydrofuran; esters such as ethyl acetate or butyl acetate; nitriles such as acetonitrile; ketones such as acetone; and organic carboxylic acids such as acetic acid or formic acid.

Moreover, for the hydrolysis of an ester in the obtained haloester and the cyclization to an epoxy group, a base is used. Examples of a base used herein may include hydroxide, carbonate and alkoxide of an alkaline metal or alkaline-earth metal. Such a base is used at a molar ratio generally between 2 : 1 and 50 : 1, and preferably between 4 : 1 and 10 : 1, with respect to the "compound 27." The reaction temperature is generally between 0°C and 50°C, and preferably between 0°C and 30°C. The type of a reaction solvent used herein is not particularly limited. Preferred examples of a reaction solvent may include: alcohols such as methanol or ethanol; ketones such as acetone; nitriles such as acetonitrile; and water.

### <Step 4A> Step of producing 5β-24,25-epoxycholesta-3-one-7-ol represented by the following formula (6) from 6,7:24,25-diepoxycholesta-4-en-3-one represented by the following formula (5)

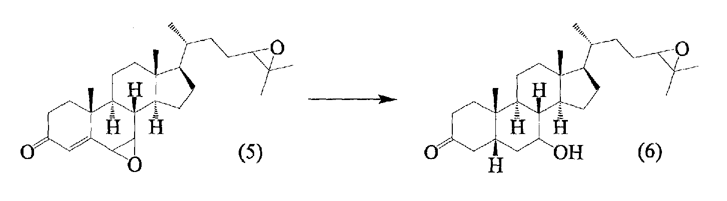

As is clear from the above reaction formula, step 4A of the present invention involves hydrogenation (reduction) of a double bond at position 4 of 6,7:24,25-diepoxycholesta-4-en-3-one (compound 5) and the reductive cleavage of a carbon-oxygen bond at position 6. Hydrogenation is carried out using hydrogen in the presence of a noble metal catalyst such as palladium, platinum, or ruthenium. Examples of a palladium catalyst used herein may include powdery palladium, and activated carbon-supporting palladium, aluminum oxide-supporting palladium, barium carbonate-supporting palladium, barium sulfate-supporting palladium, and calcium carbonate-supporting palladium, each of which contains 0.5% to 50% by weight of palladium. A noble metal catalyst is used at a molar ratio generally between 0.005 : 1 and 0.5 : 1 with respect to the "compound 5." A hydrogen pressure is not particularly limited. The reaction is generally carried out under a pressure of 1 MPa or less. Examples of a solvent used herein may include alcohols, ethers, esters, and aliphatic and aromatic hydrocarbons, but examples are not limited thereto. From the viewpoint of selectivity, it is preferable that a base be allowed to coexist in the present reaction. Preferred examples of a base used herein may include pyridine, and amines such as triethylamine, tetramethylethylenediamine or diisopropylamine. Such a base is used at a molar ratio generally between 0.1 : 1 and 100 : 1 with respect to the "compound 5." The reaction temperature is generally between 0°C and 50°C, and preferably between 0°C and 20°C.

After filtration of the catalyst, the obtained 5β-24,25-epoxycholesta-3-one-7-ol (compound 6) can be isolated and purified by methods such as silica gel column chromatography or crystallization.

### <Step 5A> Step of producing 5β-cholesta-3-one-7,24,25-triol represented by the following formula (7) from 5β-24,25-epoxycholesta-3-one-7-ol represented by the following formula (6)

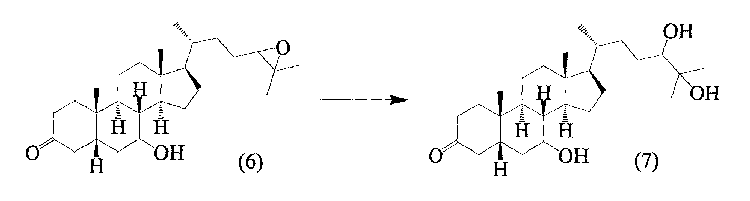

As is clear from the above reaction formula, step 5A of the present invention involves the hydrolysis of the 24,25-epoxy group of 5β-24,25-epoxycholesta-3-one-7-ol (compound 6) to 24,25-vicinal diol.

The hydrolysis reaction is carried out by allowing water to react with the compound in the presence of a catalyst. As such a catalyst, protonic acid or silica gel can be used. Examples of such protonic acid may include hydrochloric acid, sulfuric acid, nitric acid, perchloric acid, phosphoric acid, phosphorous acid, hypophosphorous acid, organic carboxylic acids, and organic sulfonic acids. The reaction temperature is generally between 10°C and 60°C, and particularly preferably room temperature. The reaction mixture is stirred for approximately 1 to 24 hours, and if necessary alkaline neutralization is carried out, so as to separate a product of interest.

The type of a reaction solvent is not particularly limited. Esters, ethers, nitriles, and other solvents can be used. Preferred examples of a solvent used herein may include ethyl acetate, tetrahydrofuran, and acetonitrile. When protonic acid is used as a catalyst, the catalyst is used at a molar ratio between approximately 0.01 : 1 and 2 : 1 with respect to the "compound 6." It is also possible to obtain the compound 7 wherein the 24,25-epoxy group of the compound 6 is hydrolyzed by supplying an organic solvent solution of the compound 6 to gel-state silica, so as to allow the compound 6 to adsorb on it, and then by supplying the organic solvent again.

The obtained 5β-cholesta-3-one-7,24,25-triol (compound 7) can be isolated and purified by methods such as silica gel column chromatography or crystallization.

### <Step 5B> Step of producing 6,7-epoxycholesta-4-en-3-one-24,25-diol represented by the following formula (9) from 6,7:24,25-diepoxycholesta-4-en-3-one represented by the following formula (5)

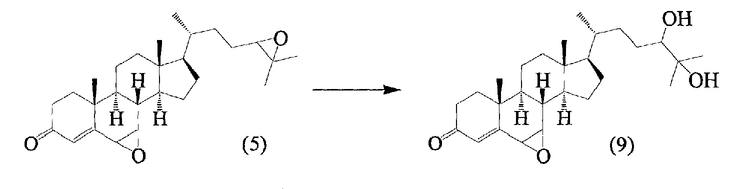

As is clear from the above reaction formula, step 5B of the present invention involves the hydrolysis of the 24,25-epoxy group of 6,7:24,25-diepoxycholesta-4-en-3-one (compound 5) to 24,25-vicinal diol.

The present reaction is the same as that in the aforementioned step 5A in that it is the hydrolysis reaction of a side chain epoxy group of a steroid compound. Accordingly, the same catalyst and solvent as those in the case of step 5A can be used, and reaction conditions are also the same.

### <Step 5C> Step of producing cholesta-4,6-dien-3-one-24,25-diol represented by the following formula (11) from 24,25-epoxycholesta-4,6-dien-3-one represented by the following formula (10)

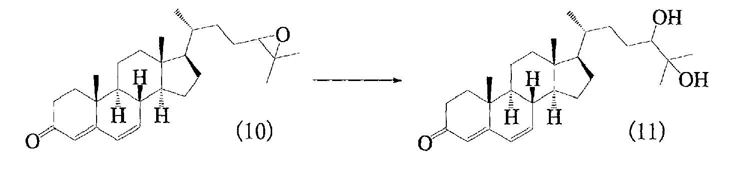

As is clear from the above reaction formula, step 5C of the present invention involves the hydrolysis of the 24,25-epoxy group of 24,25-epoxycholesta-4,6-dien-3-one (compound 10) to 24,25-vicinal diol.

The present reaction is the same as that in the aforementioned step 5A in that it is the hydrolysis reaction of a side chain epoxy group of a steroid compound. Accordingly, the same catalyst and solvent as those in the case of step 5A can be used, and reaction conditions are also the same.

### <Step 5D> Step of producing 5β-cholesta-3,7-dion-24,25-diol represented by the following formula (13) from 5β-24,25-epoxycholesta-3,7-dione represented by the following formula (12)

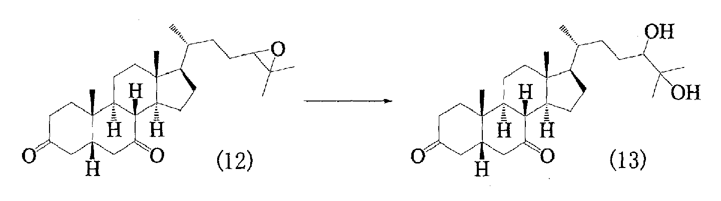

As is clear from the above reaction formula, step 5D of the present invention involves the hydrolysis of the 24,25-epoxy group of 5β-24,25-epoxycholesta-3,7-dione (compound 12) to 24,25-vicinal diol.

The present reaction is the same as that in the aforementioned step 5A in that it is the hydrolysis reaction of a side chain epoxy group of a steroid compound. Accordingly, the same catalyst and solvent as those in the case of step 5A can be used, and reaction conditions are also the same.

### <Step 4B> Step of producing 5β-cholesta-3-one-7,24,25-triol represented by the following formula (7) from 6,7-epoxycholesta-4-en-3-one-24,25-diol represented by the following formula (9)

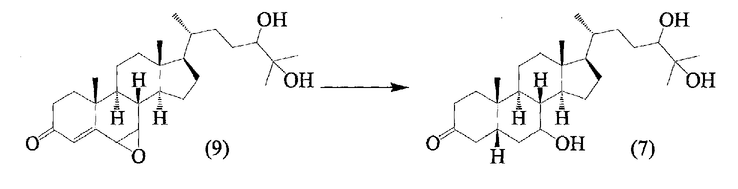

As is clear from the above reaction formula, step 4B of the present invention involves hydrogenation (reduction) of a double bond at position 4 of 6,7-epoxycholesta-4-en-3-one-24,25-diol (compound 9) and the reductive cleavage of a carbon-oxygen bond at position 6 thereof.

The present reaction is the same as that in the aforementioned step 4A in that it is the hydrogenation reaction of the 6,7-epoxy group of steroid ring B. Accordingly, the same noble metal catalyst, base, and solvent as those in the case of step 4A can be used, and reaction conditions are also the same.

### <Step 6A> Step of producing 5β-3,7-dioxocholanic acid represented by the formula (8) or an ester derivative thereof from 5β-cholesta-3-one-7,24,25-triol represented by the following formula (7)

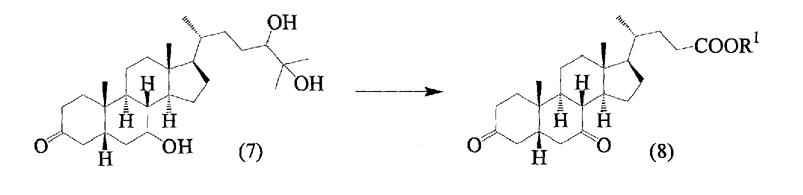

As is clear from the above reaction formula, step 6A of the present invention involves the oxidative cleavage of a 24,25-diol portion of 5β-cholesta-3-one-7,24,25-triol and oxidation of a hydroxyl group at position 7, and an esterification reaction if necessary.

Examples of an oxidizing agent used herein may include halogen acids or salts thereof, molecular halogen, permanganic acids, dichromic acids, and chromic acids. Examples of such halogen acids may include hypohalogenous acid, halogenous acid, halogenic acid and perhalogenic acid of chlorine, bromine and iodine. Examples of salts of such halogen acids may include: salts of alkaline metal such as lithium, potassium or sodium; and salts of alkaline-earth metal such as calcium or magnesium. Preferably, hypohalogenous acid or a salt thereof is used. More preferably, calcium hypochlorite or sodium hypochlorite is used. In addition, examples of an oxidizing agent used herein may include molecular halogen such as chlorine gas or bromine gas.
In some cases, halogen acids or salts thereof can be used with the combination of molecular halogen. As permanganic acids, potassium permanganate is used. As dichromic acids, dichromic acid or a pyridine salt thereof is used. As chromic acids, chromic acid or a pyridine salt thereof is used.

This oxidation reaction can be carried out, using an oxidizing agent at an equivalent molar ratio between 3 : 1 and 20 : 1, and preferably between 3 : 1 and 6 : 1, with respect to the "compound 7," in the presence of a solvent such as ketones, esters, nitriles, ethers, halogenated aliphatic hydrocarbons, or halogenated aromatic hydrocarbons, at a temperature between 0°C and 100°C, and preferably between room temperature and 30°C.

5β-3,7-dioxocholanic acid (compound 8 wherein R is a hydrogen atom) obtained by the present oxidation method can be isolated and purified by methods such as silica gel column chromatography or crystallization.

Moreover, the carboxylic acid at position 24 is then esterified by a known method, so as to induce the above carboxylic acid to an ester derivative thereof (compound 8 wherein R is an alkyl group containing 1 to 6 carbon atoms), and it can be then isolated and purified by methods such as silica gel column chromatography or crystallization, as described above. Examples of alcohol used for esterification may include linear, branched, or cyclic alcohols, such as methanol, ethanol, n-butanol, t-butanol, or cyclohexanol. Of these, methanol is preferable. The reaction can easily be carried out by heating in alcohol in the presence of an acid catalyst such as sulfuric acid or p-toluenesulfonic acid. Otherwise, the compound may also be esterified in an organic solvent other than alcohol, using dialkyl sulfate and a base (for example, potassium carbonate).

### <Step 6B> Step of producing 5β-24,25-epoxycholesta-3,7-dione represented by the following formula (12) from 5β-24,25-epoxycholesta-3-one-7-ol represented by the following formula (6)

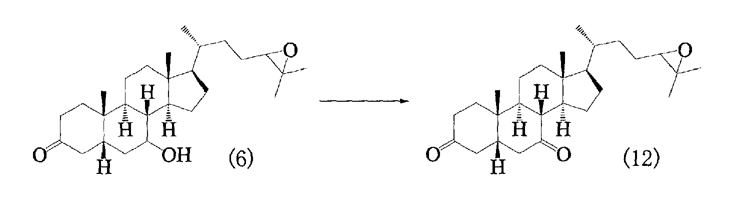

As is clear from the above reaction formula, step 6B of the present invention involves oxidation of a hydroxyl group at position 7 of 5β-24,25-epoxycholesta-3-one-7-ol (compound 6) to ketone.

The present reaction is the same as that in the aforementioned step 6A in that it is a reaction of oxidizing a hydroxyl group at position 7 of a steroid compound to ketone.
Accordingly, the same oxidizing agent and solvent as those in the case of step 6A can be used, and reaction conditions are also the same. The necessary amount of an oxidizing agent is at an equivalent molar ratio between 1 : 1 and 5 : 1, and preferably between 1 : 1 and 2 : 1, with respect to the "compound 6."

### <Step 6C> Step of producing 5β-3,7-dioxocholanic acid represented by the formula (8) or an ester derivative thereof from 5β-cholesta-3,7-dion-24,25-diol represented by the following formula (13)

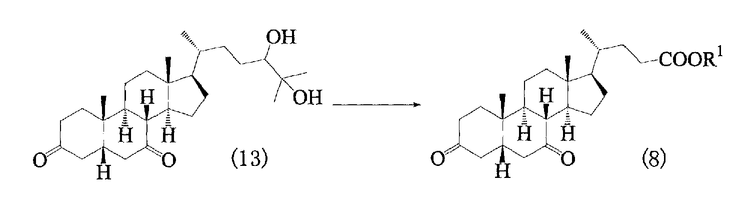

As is clear from the above reaction formula, step 6C of the present invention involves the oxidative cleavage of a 24,25-diol portion of 5β-cholesta-3,7-dion-24,25-diol (compound 13).

The present reaction is the same as that in the aforementioned step 6A in that it is an oxidative cleavage reaction of a 24,25-diol portion of a steroid compound. Accordingly, the same oxidizing agent and solvent as those in the case of step 6A can be used, and reaction conditions are also the same. The necessary amount of an oxidizing agent is at an equivalent molar ratio between 3 : 1 and 10 : 1, and preferably between 2 : 1 and 3 : 1, with respect to the "compound 13."

### <Step 8A> Step of producing 5β-24,25-epoxycholesta-3,7-diol represented by the following formula (14) from 5β-24,25-epoxycholesta-3-one-7-ol represented by the following formula (6)

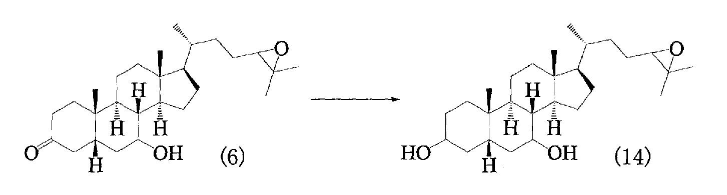

As is clear from the above reaction formula, step 8A of the present invention involves reduction of ketone at position 3 of 5β-24,25-epoxycholesta-3-one-7-ol (compound 6) to a hydroxyl group.

As a reduction method, a method using a hydride reducing agent or a method using hydrogen in the presence of a transition metal catalyst can be used.

Examples of a hydride reducing agent used herein may include an alkaline metal hydride and an alkaline-earth metal hydride. Preferably, sodium borohydride is used. Alcohols or ethers are preferably used as solvents. More preferably, alcohols are used. The reaction temperature is generally between 0°C and 50°C, and preferably between 0°C and 30°C.

Examples of a transition metal catalyst used in hydrogenation may be transition metal catalysts such as palladium, platinum, ruthenium or nickel. Preferably, platinum oxide or Raney nickel is used. Such a catalyst is used at a molar ratio generally between 0.005 : 1 and 0.5 : 1, with respect to the "compound 6." A hydrogen pressure is not particularly limited. The reaction is generally carried out under a pressure of 1 MPa or less. Examples of a solvent used herein may include alcohols, ethers, esters, and aliphatic and aromatic hydrocarbons, but examples are not limited thereto. The reaction temperature is generally between 0°C and 50°C, and preferably between 0°C and 30°C.

### <Step 9A> Step of producing a 5β-24,25-epoxycholesta-3,7-diol derivative represented by the following formula (15) comprising a protected hydroxyl group from 5β-24,25-epoxycholesta-3,7-diol represented by the following formula (14)

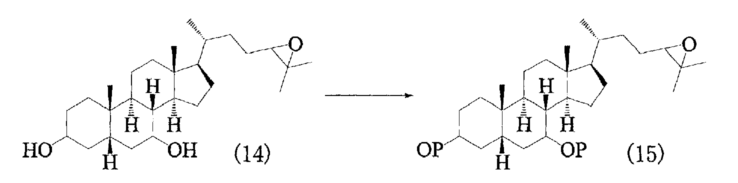

wherein P represents a protecting group for a hydroxyl group

As is clear from the above reaction formula, step 9A of the present invention involves a reaction of protecting the 3,7-hydroxyl group of 5β-24,25-epoxycholesta-3,7-diol (compound 14).

As a protecting group, an ether protecting group or an ester protecting group can be used. The type of such an ether protecting group is not particularly limited. Preferred examples of an ether protecting group used herein may include: substituted alkyl ether groups such as a methoxymethyl group or a benzyl group; and silyl ether groups such as a trimethylsilyl group or a t-butyldimethylsilyl group. The type of such an ester protecting group is not particularly limited, either. An acetyl group and a benzoyl group are preferably used. In this case, an aprotic organic solvent is used as a solvent. The aforementioned compound is allowed to react with acetic anhydride or an acylating agent such as acetyl chloride or benzoyl chloride in the presence of a base such as triethylamine, pyridine or N,N-dimethylaminopyridine, so as to obtain an ester body of interest.

### <Step 10A> Step of producing a 5β-cholesta-3,7-diol-24-one derivative represented by the following formula (16) comprising a protected hydroxyl group from a 5β-24,25-epoxycholesta-3,7-diol derivative represented by the following formula (15) comprising a protected hydroxyl group

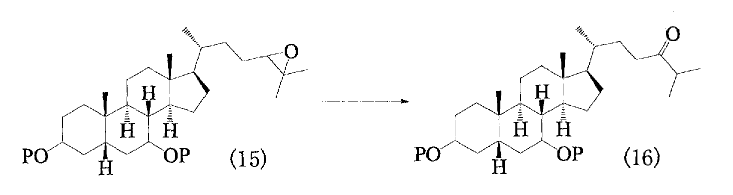

wherein P represents a protecting group for a hydroxyl group

As is clear from the above reaction formula, step 10A of the present invention involves isomerization of the 24,25-epoxy of 5β-24,25-epoxycholesta-3,7-diol derivative (compound 15) (wherein P represents a protecting group for a hydroxyl group) to ketone.

As a catalyst used in isomerization, Lewis acid, protonic acid or a salt thereof can be used.

The type of Lewis acid is not particularly limited. Examples of Lewis acid used herein may include zinc chloride, zinc bromide, zinc iodide, copper chloride (II), boron trifluoride, tin chloride (IV), tin fluoride (IV), antimony trifluoride, antimony pentafluoride, bis(4-bromo-2,6-di-t-butylphenoxide)methylammonium, and magnesium bromide. Preferably, zinc chloride is used. Examples of a solvent used herein may include: ketones such as acetone or methyl isobutyl ketone; esters such as ethyl acetate or butyl acetate; nitriles such as acetonitrile; ethers such as diethyl ether or tetrahydrofuran; aliphatic and aromatic hydrocarbons such as hexane, benzene or toluene; halogenated aliphatic hydrocarbons such as methylene chloride; and aprotic polar solvents such as N,N-dimethylformamide. Preferred examples of such a solvent used herein may include ethyl acetate, butyl acetate, tetrahydrofuran, diethyl ether, benzene, toluene, and methylene chloride. A catalyst is used at an equivalent molar ratio generally between 1 : 1 and 20 : 1, and preferably between 1 : 1 and 5 : 1, with respect to the "compound 15." The reaction temperature is generally between 0°C and 100°C, and preferably between 0°C and 30°C.

Preferred examples of protonic acid or a salt thereof used herein may include formic acid, p-toluenesulfonic acid, perchloric acids and salts thereof. More preferably, perchloric acid, lithium perchlorate, or sodium perchlorate is used. Examples of a solvent used herein may include: ketones such as acetone or methyl isobutyl ketone; esters such as ethyl acetate or butyl acetate; nitriles such as acetonitrile; ethers such as diethyl ether or tetrahydrofuran; aliphatic and aromatic hydrocarbons such as hexane, benzene or toluene; halogenated aliphatic hydrocarbons such as methylene chloride; and aprotic polar solvents such as N,N-dimethylformamide. Preferred examples of such a solvent used herein may include esters, aromatic hydrocarbons, and halogenated aliphatic hydrocarbons. The obtained 5β-cholesta-24-one-3,7-diol derivative comprising a protected hydroxyl group (in the formula, P represents a protecting group) can be isolated and purified by methods such as silica gel column chromatography.

### <Step 11> Step of producing a 5β-cholesta-3,7-dihydroxycholanic acid isopropyl ester derivative represented by the following formula (17) comprising a protected hydroxyl group from a 5β-cholesta-24-one-3,7-diol derivative represented by the following formula (16) comprising a protected hydroxyl group

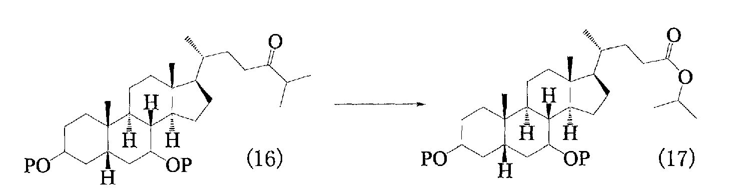

wherein P represents a protecting group for a hydroxyl group.

As is clear from the above reaction formula, step 11 of the present invention involves insertion of an oxygen atom into the side chain ketone at position 24 of the 5β-cholesta -24-one-3,7-diol derivative comprising a protected hydroxyl group (compound 16) (wherein P represents a protecting group for a hydroxyl group) by an oxidation reaction.

As an oxidizing agent, an organic peroxide is used, and organic percarboxylic acid is preferably used. The type of percarboxylic acid is not particularly limited. The organic percarboxylic acid described as an epoxidizing agent in step 3A can be used. In terms of reactivity, in particular, m-chloroperbenzoic acid or monoperphthalic acid is preferably used. Such organic percarboxylic acid is used at an equivalent molar ratio generally between 1 : 1 and 20 : 1, and preferably between 5 : 1 and 10 : 1, with respect to the "compound 16." The reaction temperature is generally between 0°C and 100°C, and preferably between 30°C and 50°C. The type of a reaction solvent is not particularly limited. Examples of a reaction solvent may include: aliphatic hydrocarbons such as hexane; aromatic hydrocarbons such as toluene; halogen solvents such as dichloromethane; ethers such as diethyl ether or tetrahydrofuran; esters such as ethyl acetate or butyl acetate; nitriles such as acetonitrile; alcohols such as methanol or ethanol; aprotic polar solvents such as dimethyl sulfoxide or dimethyl formamide; and water. Of these, esters are preferably used. The obtained 5β-3,7-dihydroxycholanic acid isopropyl ester derivative comprising a protected hydroxyl group (in the formula, P represents a protecting group) can be isolated and purified by methods such as silica gel column chromatography.

### <Step 12> Step of producing 5β-3,7-dioxocholanic acid represented by the following formula (8) or an ester derivative thereof from a 5β-3,7-dihydroxycholanic acid isopropyl ester derivative comprising a protected hydroxyl group represented by the following formula (17)

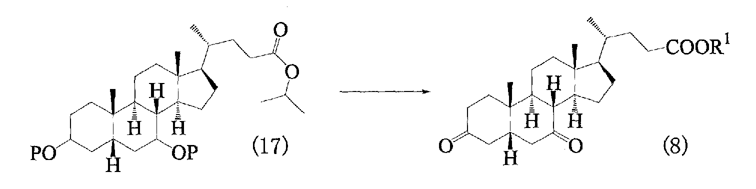

wherein P represents a protecting group for a hydroxyl group, and R¹ represents a hydrogen atom or an alkyl group containing 1 to 6 carbon atoms)

As is clear from the above reaction formula, step 12 of the present invention involves deprotection of the 5β-3,7-dihydroxycholanic acid isopropyl ester derivative comprising a protected hydroxyl group (compound 17) and further oxidation of a hydroxyl group.

When an ether protecting group is used as a protecting group that protects a hydroxyl group, generally used acid catalyst hydrolysis, or in the case of benzyl protection, catalytic hydrogenation using a palladium catalyst, is carried out for deprotection, so as to obtain a 5β-3,7-dihydroxycholanic acid isopropyl ester (which is the compound 17 wherein P is a hydrogen atom). Thereafter, a side chain ester thereof is subjected to acid or alkaline hydrolysis, as necessary, so as to induce the compound to 5β-3,7-dihydroxycholanic acid. Thereafter, a hydroxyl group thereof is oxidized, so as to obtain 5β-3,7-dioxocholanic acid (compound 8).

On the other hand, when an ester protecting group is used as a protecting group that protects a hydroxyl group, acid or alkaline hydrolysis is performed to obtain 5β-3,7-dihydroxycholanic acid, and a hydroxyl group thereof is then oxidized, so as to obtain 5β-3,7-dioxocholanic acid (compound 8).

Examples of an oxidizing agent used herein may include halogen acids or salts thereof, molecular halogen, permanganic acids, dichromic acids, and chromic acids. Examples of such halogen acids may include hypohalogenous acid, halogenous acid, halogenic acid and perhalogenic acid of chlorine, bromine and iodine. Examples of salts of such halogen acids may include: salts of alkaline metal such as lithium, potassium or sodium; and salts of alkaline-earth metal such as calcium or magnesium. Preferably, hypohalogenous acid or a salt thereof is used. More preferably, calcium hypochlorite or sodium hypochlorite is used. In addition, examples of an oxidizing agent used herein may include molecular halogen such as chlorine gas or bromine gas. In some cases, halogen acids or salts thereof can be used with the combination of molecular halogen. As permanganic acids, potassium permanganate is used. As dichromic acids, dichromic acid or a pyridine salt thereof is used. As chromic acids, chromic acid or a pyridine salt thereof is used.

This oxidation reaction can be carried out, using an oxidizing agent at an equivalent molar ratio between 3 : 1 and 20 : 1, and preferably between 2 : 1 and 6 : 1, with respect to the "5β-3,7-dihydroxycholanic acid," in the presence of a solvent such as ketones, esters, nitriles, ethers, halogenated aliphatic hydrocarbons, or halogenated aromatic hydrocarbons, at a temperature between 0°C and 100°C, and preferably between 0°C and 30°C.

5β-3,7-dioxocholanic acid (compound 8 wherein R¹ is a hydrogen atom) obtained by the present oxidation method can be isolated and purified by methods such as silica gel column chromatography or crystallization.

Moreover, the carboxylic acid at position 24 is then esterified by a known method, so as to induce the above carboxylic acid to an ester derivative thereof (compound 8 wherein R is an alkyl group containing 1 to 6 carbon atoms), and it can be then isolated and purified by methods such as silica gel column chromatography or crystallization, as described above. Examples of alcohol used for esterification may include linear, branched, or cyclic alcohols, such as methanol, ethanol, n-butanol, t-butanol, or cyclohexanol. Of these, methanol is preferable. The reaction can easily be carried out by heating in alcohol in the presence of an acid catalyst such as sulfuric acid or p-toluenesulfonic acid. Otherwise, the compound may also be esterified in an organic solvent other than alcohol, using dialkyl sulfate and a base (for example, potassium carbonate).

### <Step 10B> Step of producing cholesta-4,6-dien-3,24-dione represented by the following formula (18) from 24,25-epoxycholesta-4,6-dien-3-one represented by the following formula (10)

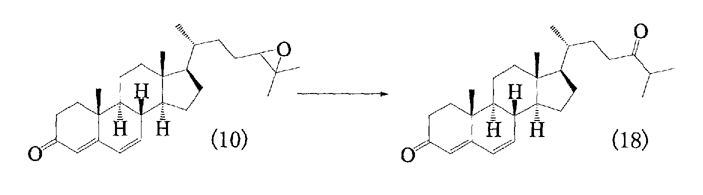

As is clear from the above reaction formula, step 10B of the present invention involves isomerization of the 24,25-epoxy of 24,25-epoxycholesta-4,6-dien-3-one (compound 10) to ketone.

The present reaction is the same as that in the aforementioned step 10A in that it is a reaction of isomerizing the epoxy at position 24 of a steroid compound to ketone. Accordingly, the same reagent and solvent as those in the case of step 10A can be used. Reaction conditions are also the same as those in step 10A.

### <Step 3D> Step of producing 6,7-epoxycholesta-4-en-3,24-dione represented by the following formula (19) from cholesta-4,6-dien-3,24-dione represented by the following formula (18)

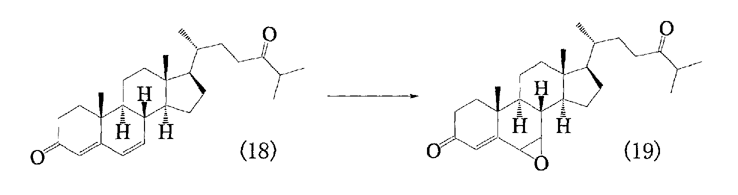

As is clear from the above reaction formula, step 3D of the present invention involves a reaction of epoxidizing a double bond at position 6 of cholesta-4,6-dien-3,24-dione represented by the following formula (18).

The present reaction is the same as that in the aforementioned step 3A in that it is a reaction of epoxidizing a double bond at position 6 of a 3-keto-4,6-dien-steroid compound. Accordingly, the same oxidizing agent and solvent as those in the case of step 3A can be used. Reaction conditions are also the same as those in step 3A. Such an epoxidizing agent is preferably used at an equivalent molar ratio between 1 : 1 and 2 : 1 with respect to the "compound 18."

### <Step 4C> Step of producing 5β-cholesta-3,24-dion-7-ol represented by the following formula (20) from 6,7-epoxycholesta-4-en-3,24-dione represented by the following formula (19)

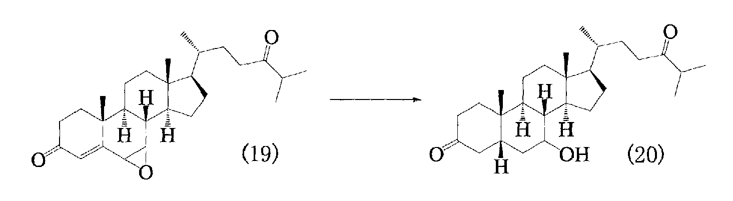

As is clear from the above reaction formula, step 4C of the present invention involves hydrogenation (reduction) of a double bond at position 4 of 6,7-epoxycholesta-4-en-3,24-dione represented by the following formula (19) and the reductive cleavage of a carbon-oxygen bond at position 6 thereof.

The present reaction is the same as that in the aforementioned step 4A in that it is a reaction of hydrogenating the 6,7-epoxy group of steroid ring B. Accordingly, the same noble metal catalyst, base and solvent as those in the case of step 4A can be used. Reaction conditions are also the same as those in step 4A.

### <Step 8B> Step of producing 5β-cholesta-24-one-3,7-diol represented by the following formula (21) from 5β-cholesta-3,24-dion-7-ol represented by the following formula (20)

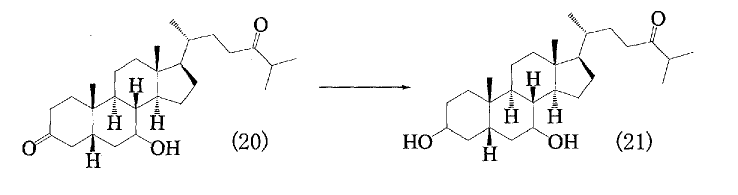

As is clear from the above reaction formula, step 8B of the present invention involves a reduction reaction of a hydroxyl group at position 3 of 5β-cholesta-3,24-dion-7-ol (compound 20).

As a reduction method, hydrogenation using a transition metal catalyst is used. Examples of such a transition metal catalyst may be transition metal catalysts such as palladium, platinum, ruthenium or nickel. Preferably, platinum oxide or Raney nickel is used. Such a catalyst is used at a molar ratio generally between 0.005 : 1 and 0.5 : 1 with respect to the "compound 20." A hydrogen pressure is not particularly limited. The reaction is generally carried out under a pressure of 1 MPa or less. Examples of a solvent used herein may include alcohols, ethers, esters, and aliphatic and aromatic hydrocarbons, but examples are not particularly limited thereto. The reaction temperature is generally between 0°C and 50°C, and preferably between 0°C and 30°C.

### <Step 9B> Step of producing a 5β-cholesta-24-one-3,7-diol derivative comprising a protected hydroxyl group represented by the following formula (16) from 5β-cholesta-24-one-3,7-diol represented by the following formula (21)

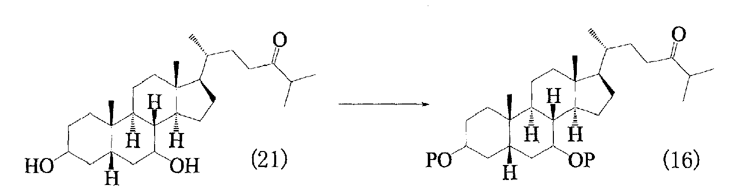

wherein P represents a protecting group for a hydroxyl group.

As is clear from the above reaction formula, step 9B of the present invention involves a reaction of protecting the 3,7-hydroxyl group of 5β-cholesta-24-one-3,7-diol (compound 21).

The present reaction is the same as that in the aforementioned step 9A in that it is a reaction of protecting the 3,7-hydroxyl group of a steroid compound. Accordingly, the same reagent and solvent as those in the case of step 9A can be used. Reaction conditions are also the same as those in step 9A.

### <Step 6D> Step of producing 3-oxochola-4,6-dien-24-al represented by the following formula (22) from cholesta-4,6-dien-3-one-24,25-diol represented by the following formula (11)

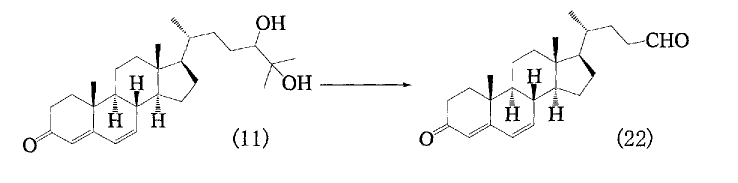

As is clear from the above reaction formula, step 6D of the present invention involves an oxidative cleavage reaction of a 24,25-diol portion of cholesta-4,6-dien-3-one-24,25-diol (compound 11).

As an oxidizing agent, periodic acids or salts thereof, dichromic acids, or chromic acids can be used. As a salt, salts of alkaline metals such as lithium, potassium or sodium, or salts of alkaline-earth metals such as calcium or magnesium are used. Preferably, periodic acid or sodium periodate is used. As dichromic acids, dichromic acid or a pyridine salt thereof is used. As chromic acids, chromic acid or a pyridine salt thereof is used. This oxidation reaction can be carried out, using an oxidizing agent at an equivalent molar ratio between 1 : 1 and 10 : 1, and preferably between 2 : 1 and 3 : 1, with respect to the "compound 11," in the presence of a solvent such as ketones, esters, nitriles, ethers, halogenated aliphatic hydrocarbons, or halogenated aromatic hydrocarbons, or water, at a temperature between 0°C and 100°C, and preferably between 0°C and 30°C.

### <Step 6E> Step of producing 3-oxochola-4,6-dienoic acid represented by the following formula (23) from 3-oxochola-4,6-dien-24-al represented by the following formula (22)

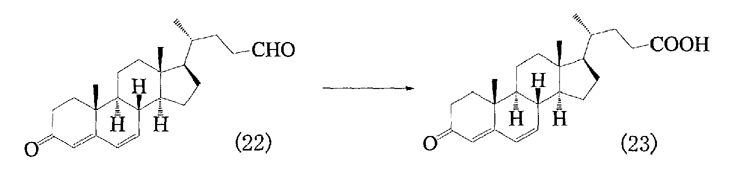

As is clear from the above reaction, step 6E of the present invention involves oxidation of the side chain aldehyde of 3-oxochola-4,6-dien-24-al (compound 22) to carboxylic acid.

As an oxidizing agent, halogen acids or salts thereof, dichromic acids, or chromic acids can be used.

Examples of an oxidizing agent used herein may include halogen acids or salts thereof, dichromic acids, and chromic acids. Examples of such halogen acids may include hypohalogenous acid, halogenic acid and perhalogenic acid of chlorine, bromine and iodine. Examples of salts of such halogen acids may include: salts of alkaline metal such as lithium, potassium or sodium; and salts of alkaline-earth metal such as calcium or magnesium. Preferably, dichromic acid or chromic acid is used. More preferably, sodium dichromate is used.

This oxidation reaction can be carried out, using an oxidizing agent at an equivalent molar ratio between 1 : 1 and 5 : 1, and preferably between 1 : 1 and 2 : 1, with respect to the "compound 22," in the presence of a solvent such as ketones, esters, nitriles, ethers, halogenated aliphatic hydrocarbons, or halogenated aromatic hydrocarbons, or water, at a temperature between 0°C and 100°C, and preferably between 0°C and 30°C.

### <Step 13> Step of producing a 3-oxochola-4,6-dienoic acid ester derivative represented by the following formula (24) from 3-oxochola-4,6-dienoic acid represented by the following formula (23)

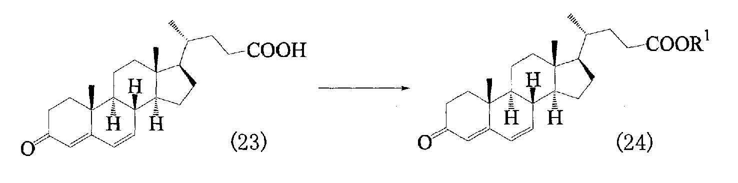

wherein R¹ represents an alkyl group containing 1 to 6 carbon atoms.

As is clear from the above reaction formula, step 13 of the present invention involves an esterification reaction of the side chain carboxylic acid of 3-oxochola-4,6-dienoic acid (compound 23).

For such a reaction, a method using an alkylating agent such as dialkyl sulfate or halogenated alkyl under basic conditions, or a method of dehydrating and condensing the aforementioned compound with alcohol in the presence of an acid catalyst is used.

When the reaction is carried out under basic conditions, as a base, hydroxide, bicarbonate or carbonate of alkaline metal or alkaline-earth metal is used. As a salt, salts of alkaline metals such as lithium, potassium or sodium, or salts of alkaline-earth metals such as calcium or magnesium are used. Preferably, carbonate or bicarbonate is used, and more preferably, potassium carbonate or sodium carbonate is used. As halogenated alkyl, methyl iodide is preferably used, and as dialkyl sulfate, dimethyl sulfate is preferably used. As a reaction solvent, ketones, ethers, nitriles, or the like can be used. Of these, acetone, tetrahydrofuran, acetonitrile, or the like is preferably used. This reaction can be carried out, using a base at an equivalent molar ratio between 1 : 1 and 20 : 1 and preferably between 3 : 1 and 10 : 1, and also using dialkyl sulfate or halogenated alkyl at an equivalent molar ratio between 1 : 1 and 20 : 1 and preferably between 1 : 1 and 10 : 1, with respect to the "compound 23," at a temperature between 0°C and 100°C, and preferably between room temperature and 70°C.

When the compound is subjected to dehydration and condensation together with alcohol in the presence of an acid catalyst, examples of such an acid catalyst may include hydrochloric acid, sulfuric acid, and organic sulfonic acid. Preferably, sulfuric acid or hydrochloric acid is used. Alcohol is preferably used as a solvent. The type of alcohol is not particularly limited. Methanol or ethanol is preferably used.

### <Step 3E> Step of producing a 6,7-epoxy-3-oxochola-4-enoic acid ester derivative represented by the following formula (25) from a 3-oxochola-4,6-dienoic acid ester derivative represented by the following formula (24)

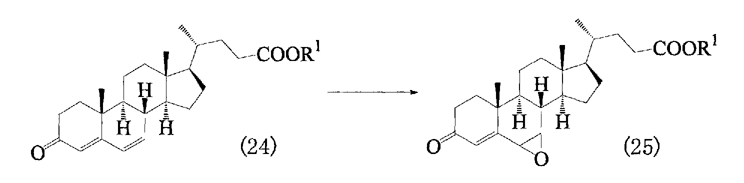

wherein R¹ represents an alkyl group containing 1 to 6 carbon atoms.

As is clear from the above reaction formula, step 3E of the present invention involves a reaction of epoxidizing a double bond at position 6 of the 3-oxochola-4,6-dienoic acid ester derivative (compound 24).

The present reaction is the same as that in the aforementioned step 3A in that it is a reaction of epoxidizing a double bond at position 6 of a 3-keto-4,6-diene steroid compound. Accordingly, the same oxidizing agent and solvent as those in the case of step 3A can be used. Reaction conditions are also the same as those in step 3A. An epoxidizing agent is preferably used at an equivalent molar ratio between 1 : 1 and 2 : 1 with respect to the "compound 24."

### <Step 4D> Step of producing a 5β-7-hydroxy-3-oxocholanic acid ester derivative represented by the following formula (32d) from a 6,7-epoxy-3-oxochola-4-enoic acid ester derivative represented by the following formula (25)

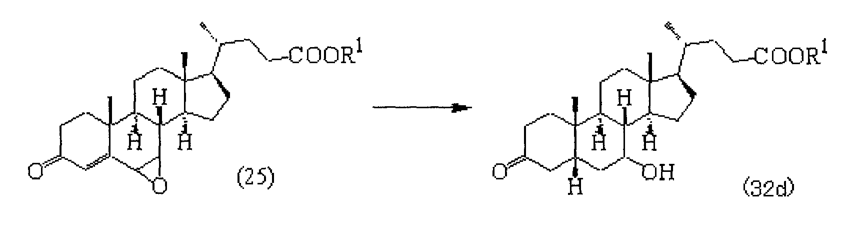

wherein R¹ represents an alkyl group containing 1 to 6 carbon atoms.

As is clear from the above reaction formula, step 4D of the present invention involves hydrogenation (reduction) of a double bond at position 4 of the 6,7-epoxy-3-oxochola-4-enoic acid ester derivative (compound 25) and the reductive cleavage of a carbon-oxygen bond at position 6 thereof.

The present reaction is the same as that in the aforementioned step 4A in that it is a hydrogenation reaction of the 6,7-epoxy group of steroid ring B. Accordingly, the same noble metal catalyst, base, and solvent as those in the case of step 4A can be used, and reaction conditions are also the same.

### <Step 6F> Step of producing 5β-3,7-dioxocholanic acid represented by the following formula (8) or an ester derivative thereof from a 5β-7-hydroxy-3-oxocholanic acid ester derivative represented by the following formula (32d)

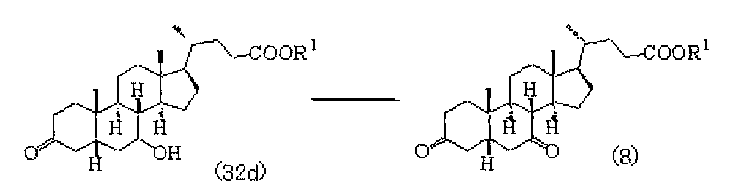

wherein R¹ represents an alkyl group containing 1 to 6 carbon atoms.

As is clear from the above reaction formula, step 6F of the present invention involves oxidation of a hydroxyl group at position 7 of the 5β-7-hydroxy-3-oxocholanic acid ester derivative (compound 32d) to ketone.

The present reaction is the same as that in the aforementioned step 6A in that it is a reaction of oxidizing a hydroxyl group at position 7 of a steroid compound to ketone.
Accordingly, the same oxidizing agent and solvent as those in the case of step 6A can be used, and reaction conditions are also the same. The necessary amount of an oxidizing agent is at an equivalent molar ratio between 1 : 1 and 5 : 1, and preferably between 1 : 1 and 2 : 1, with respect to the "compound 32d."

The method for producing 5β-3,7-dioxocholanic acid or an ester derivative thereof of the present invention is as described above.

Moreover, the reaction of hydrolyzing epoxy of the present invention is extremely effective for conversion of compound 6 to compound 7 described in step 5A, conversion of compound 5 to compound 9 described in step 5B, conversion of compound 10 to compound 11 described in step 5C, and conversion of compound 12 to compound 13 described in step 5D. Furthermore, this reaction can also be applied to an epoxy compound represented by the following formula (28) and a steroid epoxy compound represented by the following formula (30): 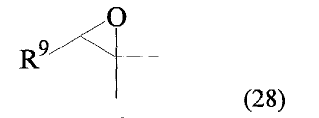 wherein R⁹ represents an alkyl group, alkenyl group or alkynyl group containing 1 to 20 carbon atoms that may be substituted with a hydroxyl group, a protected hydroxyl group, a carboxyl group, an ester group, a carbonyl group, a cyano group, an amino group or a halogen atom; and 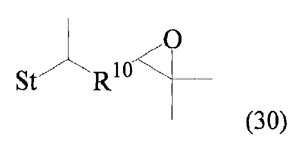 wherein St represents a steroid skeleton consisting of ring A, ring B, ring C, and ring D, wherein with regard to the aforementioned steroid skeleton, (1) it binds to a side chain shown in the formula at position C17, (2) it may have a hydroxyl group, a protected hydroxyl group, a keto group, or an epoxy group on the ring A, ring B, ring C, and ring D, (3) C-C bonds at one or more positions selected from the group consisting of positions C1 to C8 may have double bonds, and (4) one or more positions selected from the group consisting of positions C4, C10, C13 and C14 may be substituted with methyl groups; and R¹⁰ represents an alkylene group, alkenylene group or alkynylene group containing 1 to 20 carbon atoms that may be substituted with a hydroxyl group, a protected hydroxyl group, a carboxyl group, an ester group, a carbonyl group, a cyano group, an amino group or a halogen atom.

That is to say, the aforementioned epoxy compound can be converted to a vicinal diol compound represented by the following formula (29): 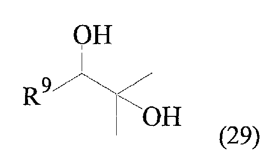 wherein R⁹ represents an alkyl group, alkenyl group or alkynyl group containing 1 to 20 carbon atoms that may be substituted with a hydroxyl group, a protected hydroxyl group, a carboxyl group, an ester group, a carbonyl group, a cyano group, an amino group or a halogen atom,
and the aforementioned steroid epoxy compound can be converted to a vicinal diol compound represented by the following formula (31): 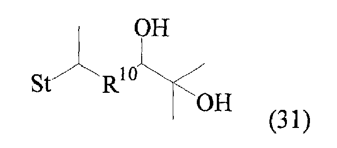 (wherein St represents a steroid skeleton consisting of ring A, ring B, ring C, and ring D, wherein with regard to the aforementioned steroid skeleton, (1) it binds to a side chain shown in the formula at position C17, (2) it may have a hydroxyl group, a protected hydroxyl group, a keto group, or an epoxy group on the ring A, ring B, ring C, and ring D, (3) C-C bonds at one or more positions selected from the group consisting of positions C1 to C8 may have double bonds, and (4) one or more positions selected from the group consisting of positions C4, C10, C13 and C14 may be substituted with methyl groups; and R¹⁰ represents an alkylene group, alkenylene group or alkynylene group containing 1 to 20 carbon atoms that may be substituted with a hydroxyl group, a protected hydroxyl group, a carboxyl group, an ester group, a carbonyl group, a cyano group, an amino group or a halogen atom.

An example of the epoxy compound represented by the above formula (28) may be an epoxy compound induced from sitnerol. Examples of the steroid epoxy compound represented by the above formula (30) may include 24,25-epoxycholesta-4,6-dien-3-one and 24,25-epoxycholesta-4-en-3-one. The method of the present invention is applied to these epoxy compounds, so as to advantageously produce vicinal diols.

The 5β-3,7-dioxocholanic acid or an ester derivative thereof (compound 8) obtained by the method of the present invention is an intermediate of steroid medicaments. By reducing the compound 8 by a known method, the compound can be converted to ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (32a), (32b), (32c) or (32d), or an ester derivative thereof: 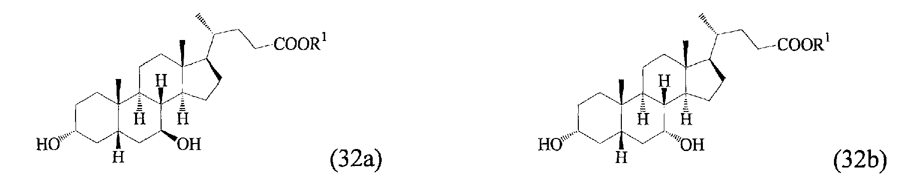 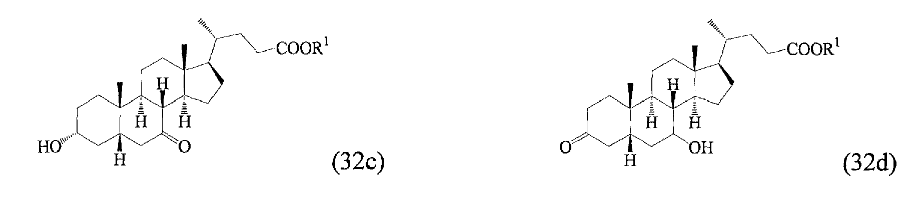 wherein R¹ represents a hydrogen atom or an alkyl group containing 1 to 6 carbon atoms.

Examples of a reduction method may include: a method of allowing the compound to react with hydrogen using water, methanol, ethanol, tetrahydrofuran or the like as a solvent, in the presence of a catalyst such as nickel (in particular, Raney nickel), cobalt, copper or chromium, and preferably in the presence of alkali such as sodium hydroxide (catalytic hydrogenation method); and a method of allowing the compound to react with alkaline metal in alcohol (metal reduction method). Moreover, a method using a specific organic boron compound at an extremely low temperature around -45°C in the presence of tetrahydrofuran as a solvent (hydride reduction method using K-selectride) is also applicable.

The following reaction steps can be used, for example.
(1) 5β-3,7-dioxocholanic acid → (metal reduction) → ursodeoxycholanic acid
(2) 5β-3,7-dioxocholanic acid → (catalytic hydrogenation) → 5β-3α-hydroxy-7-ketocholanic acid
(3) 5β-3α-hydroxy-7-ketocholanic acid → (metal reduction) → ursodeoxycholanic acid
(4) 5β-3α-hydroxy-7-ketocholanic acid → (hydride reduction) → chenodeoxycholanic acid
(5) chenodeoxycholanic acid → (silver carbonate oxidation) → 5β-3α-hydroxy-7-ketocholanic acid

See JP Patent Publication (Kokai) Nos. 52-78863 A (1977), 52-78864 A (1977) and 60-228500 A (1985), Tetrahedron (1984) Vol. 40, No. 5, p. 851, and the like, for the aforementioned methods.

Moreover, with regard to conversion of 5β-3,7-dioxocholanic acid or an ester derivative thereof (compound 8) to ursodeoxycholic acid or an ester derivative thereof (compound 32a), see JP Patent Publication (Kokai) No. 60-228500 A (1985) and JP Patent Publication (Kokai) Nos. 5-32692 A (1993). Furthermore, with regard to conversion of 5β-3,7-dioxocholanic acid or an ester derivative thereof (compound 8) to 5β-3α-hydroxy-7-ketocholanic acid or an ester derivative thereof (compound 32c), see JP Patent Publication (Kokai) Nos. 52-78863 A (1977) and JP Patent Publication (Kokai) No. 52-78864 A (1977). Still further, with regard to conversion of 5β-3α-hydroxy-7-ketocholanic acid or an ester derivative thereof (compound 32c) to ursodeoxycholic acid or an ester derivative thereof (compound 32a), see JP Patent Publication (Kokai) Nos. 52-78863 A (1977), 52-78864 A (1977) and 5-32692 A (1993).

Next, the embodiment of the above schematic view 1 will be described more in detail.

In the method for producing 5β-3,7-dioxocholanic acid or an ester derivative thereof, using, as a raw material, a sterol having double bonds at position 5 and at position 24, such as cholesta-5,7,24-trien-3β-ol, ergosta-5,7,24(28)-trien-3β-ol, desmosterol, fucosterol, or ergosta-5,24(28)-dien-3β-ol, via the following 4 steps:
(I) a step involving oxidation of a hydroxyl group at position 3 and isomerization of a double bond at position 5 to position 4;
(II) a step involving the oxidative cleavage of a side chain to convert position 24 to a carboxyl group or an ester derivative thereof;
(III) a step of introducing an oxygen functional group into position 7; and
(IV) a step of constructing a 5β configuration by reductive saturation of a double bond at position 4, with regard to the above step (I), both a sterol having a double bond at position 5 and a sterol having a double bond at position 5,7 can be treated by the same method as described in the above step 1.

Moreover, with regard to the above step (III), a steroid substrate having a double bond at position 6 can be treated by the same method as described in the above steps 3A, 3B, 3C, 3D, 3E and 7, for example, and a steroid substrate that does not have a double bond at position 6 can be treated by the method described in Appl. Environ. Microbiol., 1986, Vol. 51, p. 946, J. Chem. Res., Synop., 1986, No. 2, p. 48, or Appl. Environ. Microbiol., 1982, Vol. 44, p. 6, for example.

Furthermore, with regard to the above step (IV), the substance can be treated by the same method as described in the above steps 4A, 4B, 4C or 4D, for example.

Further, the above step (II) will be described in detail below based on the above schematic view 2.

In the case of sterols including cholesta-5,7,24-trien-3β-ol and desmosterol as typical examples, a double bond at position 24 can be epoxidized by the same method as described in the above steps 3A and 3B, for example. Subsequently, the epoxy can be hydrolyzed to glycol by the same method as described in the above steps 5A, 5B, 5C and 5D. Thereafter, the glycol can be subjected to oxidative cleavage so as to convert it to a carboxyl group at position 24 by the same method as described in the above steps 6A, 6C, 6D and 6E.

On the other hand, in the case of sterols including ergosta-5,7,24(28)-trien-3β-ol, fucosterol and ergosta-5,24(28)-dien-3β-ol as typical examples, a double bond at position 24(28) can be epoxidized by the same method as described in the above steps 3A and 3B, for example. Subsequently, the epoxy can be hydrolyzed to glycol by the same method as described in the above steps 5A, 5B, 5C and 5D. Thereafter, the glycol can be subjected to oxidative cleavage so as to induce it to a ketone body at position 24 by the same method as described in the above steps 6A, 6C, 6D and 6E. The Baeyer-Villiger oxidation method can be applied to the ketone body at position 24, so as to induce it to a carboxyl body or isopropyl ester body at position 24. The same method as described in the above steps 3A, 3B and 11 can be applied for such induction, for example.

In addition, in the case of sterols including cholesta-5,7,24-trien-3β-ol and desmosterol as typical examples as well, after epoxidation of the double bond at position 24, the epoxy can be isomerized to the ketone body at position 24 by the same method as described in the above steps 10A and 10B, for example. Thereafter, the ketone body can be induced to the carboxyl body or isopropyl ester body at position 24 by the same method as described in the above steps 3A, 3B and 11, for example.

Moreover, in all cases of the aforementioned substrates, the double bond at position 24 can be directly subjected to ozone oxidation for oxidative cleavage. For example, the double bond at position 24 is subjected to oxidative cleavage by the method described in J. Am. Chem. Soc., 1995, Vol. 77, p. 1212, so as to convert it to an aldehyde body at position 24 or a ketone body at position 24. Thereafter, the former can be induced to a carboxyl body at position 24 or an ester body at position 24 by the method described in J. Am. Chem. Soc., 1952, Vol. 74, p. 3627 or Tetrahedron Lett., 1978, No. 19, p. 1627, for example. The latter can be induced to a carboxyl body or isopropyl ester body at position 24 by the same method as described in the above steps 3A, 3B and 11, for example.

Furthermore, examples of a steroid compound containing 22 or more carbon atoms generated from carbohydrate by a fermentation method may include zymosterol, cholesta-7,24-dien-3β-ol, cholesta-5,7,24-trien-3β-ol, desmosterol, fucosterol, episterol, ergosta-5,7,24(28)-trien-3-ol, ergosta-5,7,22,24(28)-tetraen-3-ol and ergosterol. These compounds can be subjected to a step of constructing a 5β configuration by reductive saturation of a double bond at position 4 by the same method as described in the above 4A, 4B, 4C, and 4D, for example, and as necessary, can also be subjected to the combination of (I) a step involving oxidation of a hydroxyl group at position 3 and isomerization of a double bond at position 5 to position 4, (II) a step involving the oxidative cleavage of a side chain to convert position 24 to a carboxyl group or an ester derivative thereof, and (III) a step of introducing an oxygen functional group into position 7, so as to produce 5β-3,7-dioxocholanic acid (8), ursodeoxycholic acid (32a), chenodeoxycholic acid (32b), 5β-3α-hydroxy-7-ketocholanic acid (32c) or 5β-7-hydroxy-3-ketocholanic acid (32d), represented by the above formula (8), (32a), (32b), (32c) or (32d) (wherein R¹ represents a hydrogen atom or an alkyl group containing 1 to 6 carbon atoms), or an ester derivative thereof.

### EXAMPLES

The present invention will be more specifically described in the following examples. However, these examples are not intended to limit the scope of the present invention. The structures of the generated products were confirmed by ¹H-NMR (300 or 400 MHz, TMS/CDCl₃).

### [Example 1]

### <Production of cholesta-4,7,24-trien-3-one (compound 3)>

4.38 g (11.47 mmol) of cholesta-5,7,24-trien-3β -ol (compound 2) and 11.24 g (114.66 mmol) of cyclohexanone were dissolved in 44 ml of toluene, and the mixture was subjected to deaeration under a reduced pressure and nitrogen substitution at room temperature. This treatment was repeated several times. Thereafter, 1.17 g (5.74 mmol) of aluminum isopropoxide was added to the reaction solution at room temperature, and the obtained mixture was then stirred in a nitrogen atmosphere at 112°C for 2 hours. After completion of the reaction, the reaction solution was cooled to room temperature, and 2.3 ml of water was then added thereto. The obtained mixture was stirred at room temperature for 1 hour. Thereafter, the deposited precipitate was filtrated, and the filtrate was then concentrated. The concentrate was isolated and purified by silica gel column chromatography, so as to obtain 4.01 g of cholesta-4,7,24-trien-3-one (compound 3). The yield of the isolated and purified compound 3 was found to be 92%. The NMR shift value (δ ppm) is shown below.
δ : 0.60 (s, 3H, 18-H), 0.96 (d, 6.5Hz, 3H, 21-H), 1.18 (s, 3H, 19-H), 1.61 (s, 3H, 26-H), 1.69 (s, 3H, 27-H) , 2.22 (m, 1H), 2.30-2.40 (m, 3H) , 2.63-2.72 (m, 1H, 6-H), 3.10-3.20 (m, 1H, 6-H), 5.09 (m, 1H, 24-H), 5.18 (m, 1H, 7-H), 5.80 (s, 1H, 4-H)

### [Example 2]

### <Production of cholesta-4,6,24-trien-3-one (compound 4)>

3.49 g (9.18 mmol) of the cholesta-4,7,24-trien-3-one (compound 3) obtained by the method described in Example 1 was dissolved in 70 ml of methanol, and thereafter, deaeration under a reduced pressure and nitrogen substitution were repeated several times at room temperature. Thereafter, 2.53 g (38.40 mmol) of 85% granurated potassium hydroxide was added to the reaction solution, and the obtained mixture was then stirred in a nitrogen atmosphere at 64°C for 7 hours. After completion of the reaction, the reaction solution was cooled to room temperature, and 2.37 g of acetic acid was then added thereto, followed by stirring the obtained mixture at room temperature for 0.5 hours. Subsequently, the methanol was distilled away under a reduced pressure, and water was then added thereto, followed by extraction with ethyl acetate. The organic phase was washed with water, dried, and then concentrated. The obtained concentrate was isolated and purified by silica gel column chromatography, so as to obtain 3.13 g of cholesta-4,6,24-trien-3-one (compound 4). The yield of the isolated and purified compound 4 was found to be 90%. The NMR shift value (δ ppm) is shown below.
δ :0.76 (s, 3H, 18-H), 0.95 (d, 6.5Hz, 3H, 21-H) , 1.12 (s, 3H, 19-H), 1.61 (s, 3H, 26-H), 1.69 (s, 3H, 27-H), 2.19 (m, 1H), 2.39-2.65 (m, 2H), 5.09 (m, 1H, 24-H), 5.67 (s, 1H, 4-H), 6.12 (m, 2H, 6-H and 7-H)

### [Example 3]

### <Production of ergosta-4,6,24-trien-3-one>

0.20 g (0.50 mmol) of ergosta-4,7,24-trien-3-one was dissolved in 10 ml of methanol. Thereafter, deaeration under a reduced pressure and nitrogen substitution were repeated several times at room temperature. Thereafter, 0.10 g (1.52 mmol) of 85% granurated potassium hydroxide was added to the reaction solution, and the obtained mixture was stirred in a nitrogen atmosphere at 65°C for 12 hours. After completion of the reaction, the reaction solution was cooled to room temperature, and 0.18 g of acetic acid was then added thereto, followed by stirring the obtained mixture at room temperature for 0.5 hours. Subsequently, the methanol was distilled away under a reduced pressure, and water was then added thereto, followed by extraction with ethyl acetate. The organic phase was washed with water, dried, and then concentrated. The obtained concentrate was isolated and purified by silica gel column chromatography, so as to obtain 0.20 g of ergosta-4,6,24-trien-3-one. The yield was found to be 100%. The NMR shift value (δ ppm) is shown below.
δ :0.77 (s, 3H), 0.81 (d, 7.3Hz, 3H), 0.83 (d, 7.3Hz, 3H) , 0.91 (d, 7.3Hz, 3H), 1.01 (d, 7.3Hz, 3H), 1.12 (s, 3H), 2.20 (m, 1H), 2.38-2.66 (m, 2H), 5.11-5.27 (m, 2H), 5.67 (s, 1H), 6.06-6.17 (m, 2H)

The reaction formula of the present example is shown below.

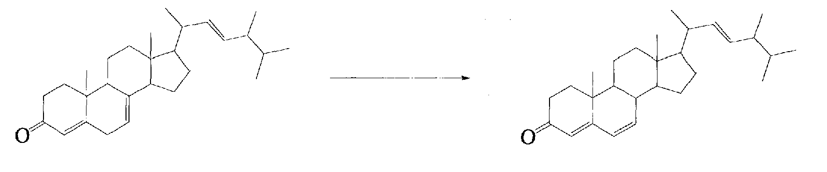

### [Comparative example 1]

### <Production of cholesta-4,7,24-trien-3-one (compound 3)>

0.20 g (0.5 mmol) of cholesta-5,7,24-trien-3β-ol (compound 2) and 0.26 g (2.6 mmol) of cyclohexanone were dissolved in 2 ml of heptane. Thereafter, 0.02 g (0.1 mmol) of aluminum isopropoxide was added thereto at room temperature, and the obtained mixture was heated to reflux for 4 hours in an open system. After completion of the reaction, the reaction solution was cooled to room temperature, and water was added thereto. The obtained mixture was stirred at room temperature for 1 hour. The deposited precipitate was filtrated, and the filtrate was then concentrated. The concentrate was calibrated by HPLC. As a result, the yield of compound 3 was found to be 85%.

### [Comparative example 2]

### <Production of cholesta-4,6,24-trien-3-one (compound 4)>

0.3 g (0.8 mmol) of the cholesta-4,7,24-trien-3-one (compound 3) obtained by the method described in Example 1 was dissolved in 5.3 ml of methanol. Thereafter, 0.09 g (1.4 mmol) of 85% granurated potassium hydroxide was added thereto, and the obtained mixture was heated to reflux for 4 hours in an open system. After completion of the reaction, the reaction solution was cooled to room temperature, and 2.37 g of acetic acid was then added thereto. The obtained mixture was stirred at room temperature for 0.5 hours. Thereafter, the methanol was distilled away under a reduced pressure. Water was added thereto, followed by extraction with ethyl acetate. The organic phase was washed with water, dried, and then concentrated. The concentrate was calibrated by HPLC. As a result, the yield of compound 4 was found to be 52%.

### [Example 4-1]

### <Step 3A: production of 6,7:24,25-diepoxycholesta-4-en-3-one (compound 5)>

0.10 g (0.26 mmol) of cholesta-4,6,24-trien-3-one was dissolved in 3.5 ml (1 mmol) of a 0.3 M perbenzoic acid/ethyl acetate solution, and the obtained mixture was then stirred at 45°C for 8 hours. After completion of the reaction, an aqueous 10% sodium sulfite solution was added to the reaction solution to decompose the residual peroxide, and the resultant was then extracted with ethyl acetate. Subsequently, the organic phase was washed with an aqueous saturated potassium bicarbonate solution, dried, and then concentrated, so as to obtain 0.138 g of a crude compound, 6,7:24,25-diepoxycholesta-4-en-3-one. The yield was found to be 70%. The NMR shift value (δ ppm) is shown below.
δ :0.75 (s, 3H, 18-H), 0.95 (d, 5.7Hz, 3H, 21-H), 1.10 (s, 3H, 19-H), 1.27 (s, 3H, 26-H), 1.30 (s, 3H, 27-H), 2.4-2.6 (m, 2H), 2.69 (t, 5.5Hz, 1H, 24-H), 3.3-3.4 (m, 1H, 7-H), 3.47 (d, 3.3Hz, 1H, 6-H), 6.12 (s, 1H, 4-H)

### [Example 4-2]

### <Step 3A: production of 6,7:24,25-diepoxycholesta-4-en-3-one (compound 5)>

1.00 g (2.63 mmol) of cholesta-4,6,24-trien-3-one was dissolved in 11.7 ml of n-butyl acetate, and 4 ml of water was then added thereto. Thereafter, 2.53 ml (2.63 mmol) of a 1.04 M 2-methylperbenzoic acid/n-butyl acetate solution (hereinafter abbreviated as a peracid solution) was added dropwise to the above mixed solution at 78°C, and the obtained mixture was then stirred at 78°C for 1 hour. Thereafter, the water phase was separated and eliminated, and the resultant was then washed with a saturated sodium bicarbonate solution and then with water. Thereafter, 4 ml of water was added to the resultant. (* 1) 0.50 ml (0.53 mmol) of a peracid solution was added to the mixed solution at 78°C, and the obtained mixture was then stirred at 78°C for 0.5 hours. Thereafter, 0.50 ml (0.53 mmol) of a peracid solution was further added to the reaction solution, and the obtained mixture was then stirred at 78°C for 0.5 hours. Thereafter, the water phase was separated and eliminated, and the resultant was then washed with a saturated sodium bicarbonate solution and then with water. Thereafter, 4 ml of water was added to the resultant. The same operations as described in (*1) were repeated twice on the above mixed solution. Thereafter, 0.50 ml (0.53 mmol) of a peracid solution was added thereto at 78°C, and the mixture was then stirred at 78°C for 0.5 hours. Thereafter, 0.50 ml (0.53 mmol) of a peracid solution was further added thereto, and the mixture was then stirred at 78°C for 0.5 hours. Thereafter, the reaction solution was cooled to room temperature, and sodium sulfite was added thereto to decompose the residual peroxide, followed by extraction with ethyl acetate. Subsequently, the organic phase was washed with a saturated sodium bicarbonate solution, dried, and then concentrated, so as to obtain 1.33 g of a crude compound, 6,7:24,25-diepoxycholesta-4-en-3-one. The yield was found to be 82%.

The obtained crude compound, 6,7:24,25-diepoxycholesta-4-en-3-one, was subjected to thin-layer chromatography, so as to separate 6α-, 7α-, and 6β-, 7β-forms. The NMR shift values (δ ppm) thereof are shown below.
6α, 7α ; 24, 25-diepoxycholesta-4-en-3-one,
δ :0.75 (s, 3H, 18-H), 0.94 (d, 6.8Hz, 3H, 21-H), 1.09 (s, 3H, 19-H), 1.27 and 1.31 (s, 6H, 26-H and 27-H), 2.42-2.60 (m, 2H), 2.69 (t, 6.0Hz, 1H, 24-H), 3.35 (d, 3.6Hz, 1H, 7-H), 3.46 (d, 4.0Hz, 1H, 6-H), 6.11 (s, 1H, 4-H)
6β, 7β; 24, 25-diepoxycholesta-4-en-3-one,
δ :0.75 (s, 3H, 18-H), 0.95 (d, 6.5Hz, 3H, 21-H), 1.21 (s, 3H, 19-H), 1.27 and 1.31 (s, 6H, 26-H and 27-H) , 2.36-2.64 (m, 2H), 2.69 (t, 5.5Hz, 1H, 24-H), 3.37 (s, 2H, 6-H and 7-H), 6.15 (s, 1H, 4-H)

### [Example 5-1]

### <Step 4A: production of 5β-24,25-epoxycholesta-3-one-7-ol (compound 6)>

0.128 g of the crude compound, 6,7:24,25-diepoxycholesta-4-en-3-one, which was obtained in the aforementioned Example 4-1, was dissolved in 1.8 ml of ethyl acetate. Thereafter, 0.5 ml of triethylamine and 7 mg of 10% palladium carbon were added thereto, and the obtained mixture was stirred in a hydrogen atmosphere of 1 atm at room temperature for 15 hours. After completion of the reaction, the catalyst was filtrated, and the filtrate was then concentrated. The concentrate was subjected to short silica gel column chromatography for concentration, so as to obtain 0.077 g of a crude compound, 5β-24,25-epoxycholesta-3-one-7-ol. The yield was found to be 87%. The NMR shift value (δ ppm) is shown below.
δ :0.70 (s, 3H, 18-H), 0.95 (d, 5.5Hz, 3H, 21-H), 1.00 (s, 3H, 19-H), 1.27 (s, 3H, 26-H), 1.30 (s, 3H, 27-H), 2.15-2.25 (m, 2H), 2.3-2.5 (m, 1H), 2.69 (t, 5.5Hz, 1H, 24-H), 3.40 (t, 13.3Hz, 1H, 4-H), 3.92 (m, 1H, 7-H)

The obtained crude compound, 5β-24,25-epoxycholesta-3-one-7-ol, was subjected to thin-layer chromatography, so as to separate 7α- and 7β-forms and a reaction intermediate. The NMR shift values (δ ppm) thereof are shown below.
5β-24, 25-epoxycholesta-3-one-7α-ol
δ: 0.71 (s, 3H, 18-H), 0.96 (d, 6.0Hz, 3H, 21-H), 1.01 (s, 3H, 19-H), 1.27 and 1.31 (s, 6H, 26-H and 27-H), 2.12-2.23 (m, 2H), 2.41 (dt, 4Hz and 4.8Hz, 1H), 2.69 (t, 6.0Hz, 1H, 24-H), 3.39 (t, 13.2Hz, 1H, 4-H), 3.93 (m, 1H, 7-H)
5β-24, 25-epoxycholesta-3-one-7β-ol
δ: 0.73 (s, 3H, 18-H), 0.96 (d, 6.6Hz, 3H, 21-H), 1.06 (s, 3H, 19-H), 1.27 and 1.31 (s, 6H, 26-H and 27-H), 2.15-2.35 (m, 3H), 2.52 (t, 11.0Hz, 1H), 2.69 (t, 6.1Hz, 1H, 24-H), 3.56-3.68 (m, 1H, 7-H)
24, 25-epoxycholesta-4-ene-3-one-7 α-ol
δ :0.73 (s, 3H, 18-H), 0.94 (d, 6.8Hz, 3H, 21-H), 1.19 (s, 3H, 19-H), 1.27 and 1.31 (s, 6H, 26-H and 27-H), 2.33-2.46 (m, 3H), 2.60-2.64 (m, 1H), 2.69 (t, 6.4Hz, 1H, 24-H), 3.97 (m, 1H, 7-H), 5.81(d, 1.6Hz, 1H, 4-H)
24, 25-epoxycholesta-4-ene-3-one-7β-ol
δ: 0.74 (s, 3H, 18-H), 0.95 (d, 6.8Hz, 3H, 21-H), 1.21 (s, 3H, 19-H), 1.27 and 1.31 (s, 6H, 26-H and 27-H), 2.31-2.56 (m, 4H), 2.69 (t, 6.0Hz, 1H, 24-H), 3.42-3.50 (m, 1H, 7-H), 5.76 (d, 1.2Hz, 1H, 4-H)

### [Example 5-2]

### <Step 4A: production of 5β-24,25-epoxycholesta-3-one-7-ol (compound 6)>

2.6 mg of 5% palladium carbon was suspended in 1,060 µl of methanol, and 25 µl (0.165 mmol) of tetramethylethylenediamine was then added thereto. The obtained mixture was stirred in a nitrogen atmosphere at 50°C for 3 hours. Subsequently, the reaction solution was cooled to room temperature, and 206 µl of water was then added thereto. The inside of the reaction system was substituted with hydrogen of 1 atm, and the reaction solution was then stirred at room temperature for 1 hour. Subsequently, the pre-treated catalyst suspension was cooled to 5°C. Thereafter, 310 µl of an n-butyl acetate solution containing 100 mg (0.242 mmol) of the crude 6,7:24,25-diepoxycholesta-4-en-3-one that had been obtained in the aforementioned Example 4-2 and had been then purified with a silica gel column and 424 µl of methanol were added thereto in a hydrogen atmosphere. The obtained mixture was stirred in a hydrogen atmosphere of 1 atm at 5°C for 49 hours. After completion of the reaction, the catalyst was filtrated, and the filtrate was then concentrated. The concentrate was concentrated by short silica gel column chromatography, so as to obtain 115 mg of a crude compound, 5β-24,25-epoxycholesta-3-one-7-ol. The yield was found to be 96%.

### [Example 6-1]

### <Step 5A: production of 5β-cholesta-3-one-7,24,25-triol (compound 7)>

0.077 g (0.19 mmol) of the crude compound, 5β-24,25-epoxycholesta-3-one-7-ol, which was obtained in the aforementioned Example 5-1, was adsorbed on a silica gel column, and it was then eluted with a mixed solution of hexane and ethyl acetate, so as to obtain 0.080 g of 5β-cholesta-3-one-7,24,25-triol. The yield was found to be 100%. The NMR shift value (δ ppm) thereof is shown below.
δ: 0.71 (s, 3H, 18-H), 0.95 and 0.96 (d, 6.8Hz, 3H, 21-H), 1.01 (s, 3H, 19-H), 1.17 and 1.21 (s, 6H, 26-H and 27-H), 2.15-2.23 (m, 2H), 2.35-2.45 (m, 1H), 3.27-3.33 (m, 1H, 24-H), 3.39 (t, 15.6Hz, 1H, 4-H), 3.93 (m, 1H, 7-H)

### [Example 6-2]

### <Step 5A: production of 5β-cholesta-3-one-7,24,25-triol (compound 7)>

145 mg (0.35 mmol) of the 5β-24,25-epoxycholesta-3-one-7-ol which was obtained in the aforementioned Example 5-2 was dissolved in a mixed solution of 1 ml of acetonitrile and 1 ml of water. Thereafter, 73 mg (0.35 mmol) of citric acid monohydrate was added thereto, and the obtained mixture was then stirred at room temperature for 9 hours. After completion of the reaction, sodium bicarbonate was added to the reaction solution, so as to neutralize citric acid. Thereafter, the acetonitrile was distilled away under a reduced pressure, followed by extraction with ethyl acetate. The extract was concentrated, so as to obtain 150 mg of a crude compound, 5β-cholesta-3-one-7,24,25-triol. The yield was found to be 98%.

### [Example 7]

### <Step 6A: production of 5β-3,7-dioxocholanic acid (compound 8)>

93 mg (0.22 mmol) of 5β-cholesta-3-one-7,24,25-triol was dissolved in 1.5 ml of acetonitrile and 1.1 ml of water. Thereafter, 0.17 g (0.44 mmol) of 60% calcium hypochlorite was added thereto at room temperature, and 0.12 g of acetic acid was further added to the obtained mixture, followed by stirring the obtained mixture at room temperature for 40 minutes. After completion of the reaction, an aqueous 10% sodium sulfite solution was added to the reaction solution to decompose an excessive oxidizing agent. Thereafter, concentrated hydrochloric acid was added thereto, so that the pH thereof could be adjusted to pH 1. After the mixture had been subjected to vacuum concentration, the concentrate was isolated and purified by silica gel column chromatography, so as to obtain 75 mg of 5β-3,7-dioxocholanic acid. The yield was found to be 92%. The NMR shift value (δ ppm) thereof is shown below.
δ: 0.72 (s, 3H, 18-H), 0.95 (d, 6.3Hz, 3H, 21-H), 1.30 (s, 3H, 19-H), 2.50 (t, 10.7Hz, 1H), 2.88 (dd, 5.1Hz and 14.2Hz, 1H)

### [Example 8]

### <Step 5B: production of 6,7-epoxycholesta-4-en-3-one-24,25-diol (compound 9)>

0.120 g of the crude compound, 6,7:24,25-diepoxycholesta-4-en-3-one, which was synthesized by the same method as that in Example 4-1, was adsorbed on a silica gel column, and it was then eluted with a mixed solution of hexane and ethyl acetate, so as to obtain 0.080 g of a crude compound, 6,7-epoxycholesta-4-en-3-one-24,25-diol. The yield was found to be 80%. The NMR shift value (δ ppm) thereof is shown below.
δ: 0.75 (s, 3H, 18-H), 0.95 and 0.96 (d, 6.8Hz, 3H, 21-H), 1.10 (s, 3H, 19-H), 1.18 (s, 3H, 26-H), 1.23 (s, 3H, 27-H), 2.4-2.6 (m, 2H), 3.25-3.31 (m, 1H, 24-H), 3.33-3.38 (m, 1H, 7-H), 3.46 (d, 3.7Hz, 1H, 6-H), 6.12 (s, 1H, 4-H)

### [Example 9]

### <Step 4B: production of 5β-cholesta-3-one-7,24,25-triol (compound 7)>

The crude compound, 6,7-epoxycholesta-4-en-3-one-24,25-diol, which was obtained by the same method as that in the aforementioned Example 8, was purified with a silica gel column. 0.075 g of the thus purified product was reduced by the same method as that in Example 5-1, and it was then purified by silica gel column chromatography, so as to obtain 0.068 g of 5β-cholesta-3-one-7,24,25-triol. The yield was found to be 90%. In addition, it was confirmed that the NMR data thereof were identical to those in Example 6-1.

### [Example 10]

### <Step 3B: production of 24,25-epoxycholesta-4,6-dien-3-one (compound 10)>

0.10 g (0.27 mmol) of cholesta-4,6,24-trien-3-one was dissolved in 2 ml of ethyl acetate. Thereafter, 0.76 ml (0.32 mmol) of a 0.42 M monoperphthalic acid/ethyl acetate solution was added thereto, and the obtained mixture was then stirred at room temperature for 3 hours. After completion of the reaction, an aqueous 10% sodium sulfite solution was added to the reaction solution to decompose the residual peroxide, followed by extraction with ethyl acetate. Subsequently, the organic phase was washed with an aqueous saturated potassium bicarbonate solution, dried, and then concentrated, so as to obtain 0.11 g of a crude compound, 24,25-epoxycholesta-4,6-dien-3-one. The yield was found to be 100%. The NMR shift value (δ ppm) thereof is shown below.
δ: 0.77 (s, 3H, 18-H), 0.95 (d, 6.4Hz, 3H, 21-H), 1.11 (s, 3H, 19-H), 1.27 (s, 3H, 26-H), 1.31 (s, 3H, 27-H), 2.15-2.23 (m, 1H), 2.38-2.65 (m, 2H, 2-H), 2.69 (t, 5.5Hz, 1H, 24-H), 5.67(s, 1H, 4-H), 6.07-6.16 (m, 2H, 6-H and 7-H)

### [Example 11-1]

### <Step 5C: production of cholesta-4,6-dien-3-one-24,25-diol (compound 11)>

0.110 g of the crude compound, 24,25-epoxycholesta-4,6-dien-3-one (compound 10), which was synthesized in accordance with Example 10, was adsorbed on a silica gel column, and it was then eluted with a mixed solution of hexane and ethyl acetate, so as to obtain 0.070 g of cholesta-4,6-dien-3-one-24,25-diol. The yield was found to be 61 %. The NMR shift value (δ ppm) thereof is shown below.
δ: 0.76 and 0.77 (s, 3H, 18-H), 0.94 and 0.95 (d, 6.6Hz, 3H, 21-H), 1.11 (s, 3H, 19-H), 1.17 (s, 3H, 26-H), 1.21 (s, 3H, 27-H), 2.37-2.64 (m, 2H), 3.25-3.37 (m, 1H, 24-H), 5.67 (s, 1H, 4-H), 6.05-6.16 (m, 2H, 6-H and 7-H)

### [Example 11-2]

### <Step 5C: production of cholesta-4,6-dien-3-one-24,25-diol (compound 11)>

1.09 g of the crude compound, 24,25-epoxycholesta-4,6-dien-3-one (compound 10), which was synthesized in accordance with Example 10, was dissolved in a mixed solution of 15 ml of acetonitrile and 5 ml of water. 0.55 g of citric acid monohydrate was added thereto, and the obtained mixture was then stirred at room temperature for 23 hours. Thereafter, a saturated sodium bicarbonate solution was added to the reaction solution for neutralization, followed by extraction with ethyl acetate. Subsequently, the organic phase was washed with a saturated sodium bicarbonate solution, dried, and then concentrated. The obtained concentrate was purified by silica gel column chromatography, so as to obtain 0.82 g of cholesta-4,6-dien-3-one-24,25-diol (yield: 79%).

In addition, it was confirmed that the NMR data thereof were identical to those in Example 11-1.

### [Example 12]

### <Step 3C: production of 6,7-epoxycholesta-4-en-3-one-24,25-diol (compound 9)>

66 mg (0.159 mmol) of the cholesta-4,6-dien-3-one-24,25-diol (compound 11) which was synthesized in accordance with Example 11-1 was epoxidized by the same method as that in Example 4-2 (wherein the amount of peracid used was 0.254 mmol in total), and the resultant was then purified by silica gel column chromatography, so as to obtain 48 mg of 6,7-epoxycholesta-4-en-3-one-24,25-diol. The yield was found to be 70%. In addition, it was confirmed that the NMR data thereof were identical to those in Example 8.

### [Example 13]

### <Step 6B: production of 5β-24,25-epoxycholesta-3,7-dione (compound 12)>

667 mg of the crude compound, 5β-24,25-epoxycholesta-3-one-7-ol (compound 6), which was synthesized in accordance with Example 5-2, was dissolved in 5.4 ml of acetonitrile and 2.7 ml of water. Thereafter, 1.21 ml of acetic acid and 31 mg of NaBr were added thereto. Thereafter, 0.36 g (1.51 mmol) of 60% calcium hypochlorite was added to the mixture at 0°C, and the obtained mixture was then stirred at 0°C for 23 hours. After completion of the reaction, sodium sulfite was added to the reaction solution to decompose an excessive oxidizing agent, followed by extraction with ethyl acetate. Thereafter, the extract was washed with a saturated sodium bicarbonate solution and then with water. The resultant was dried and was then subjected to vacuum concentration, so as to obtain 576 mg of a crude compound, 5β-24,25-epoxycholesta-3,7-dione. The yield was found to be 96%. The NMR shift value (δ ppm) thereof is shown below.
δ: 0.70 (s, 3H, 18-H), 0.95 (d, 6.4Hz, 3H, 21-H), 1.27 and 1.31 (s, 6H, 26-H and 27-H), 1.31 (s, 3H, 19-H), 2.50 (t, 11.2Hz, 1H), 2.69 (t, 6.4Hz, 1H, 24-H), 2.89 (dd, 5.6Hz and 12.8Hz, 1H)

### [Example 14]

### <Step 5D: production of 5β-cholesta-3,7-dione-24,25-diol (compound 13)>

419 mg of the crude compound, 5β-24,25-epoxycholesta-3,7-dione (compound 12), which was obtained in Example 13, was hydrolyzed by the same method as that in Example 6-2, so as to obtain 410 mg of a crude compound, 5β-cholesta-3,7-dione-24,25-diol. The yield was found to be 97%. The NMR shift value (δ ppm) thereof is shown below.
δ :0.70 and 0.71 (s, 3H, 18-H), 0.94 and 0.95 (d, 6.0Hz, 3H, 21-H), 1.16 and 1.21 (s, 6H, 26-H and 27-H), 1.31 (s, 3H, 19-H), 2.51 (t, 11.6Hz, 1H), 2.89 (dd, 5.6Hz and 12.8Hz, 1H), 3.27-3.35 (m, 1H, 24-H)

### [Example 15]

### <Step 6C: production of 5β-3,7-dioxocholanic acid (compound 8)>

390 mg of the crude compound 5β-cholesta-3,7-dione-24,25-diol (compound 13), which was obtained in Example 14, was dissolved in 6 ml of acetonitrile and 2.7 ml of water. Thereafter, 0.17 ml of acetic acid was further added thereto. Thereafter, 294 mg (1.23 mmol) 60% calcium hypochlorite was added to the obtained mixture at 10°C, and the obtained mixture was then stirred at 10°C for 26.5 hours. After completion of the reaction, sodium sulfite was added to the reaction solution to decompose an excessive oxidizing agent. Thereafter, concentrated hydrochloric acid was added thereto, resulting in pH < 1. The reaction solution was extracted with ethyl acetate, and the extract was washed with water, dried, and then concentrated, so as to obtain 381 mg of a crude compound, 5β-3,7-dioxocholanic acid. The yield was found to be 84%. In addition, it was confirmed that the NMR data thereof were identical to those in Example 7.

### [Example 16]

### <Step 7: production of 6,7:24,25-diepoxycholesta-4-en-3-one (compound 5)>

547 mg (1.44 mmol) of cholesta-4,6,24-trien-3-one was dissolved in 10 ml of formic acid at 10°C, and 520 µl of t-butyl hypochloride was then added thereto. The obtained mixture was stirred at 10°C for 30 minutes. After completion of the reaction, water was added to the reaction solution, followed by extraction with ethyl acetate. The extract was washed with water and then with a saturated sodium bicarbonate solution. The resultant was dried and then concentrated, so as to obtain 870 mg of a crude compound, 7,24-dichloro-cholesta-4-en-3-one-6,25-diol diformyl ester (compound 27a). The yield was found to be approximately 70%. The NMR shift value (δ ppm) thereof is shown below.
δ: 0.77 (s, 3H, 18-H), 0.93 and 0.95 (d, 4.9Hz and 6.5Hz, 3H, 21-H), 1.28 (s, 3H, 19-H), 1.60 (s, 6H, 26-H and 27-H), 2.38-2.57 (m, 2H), 4.14 (t, 3.0Hz, 1H), 4.25 (t, 12.0Hz, 1H), 5.55 (d, 1.9Hz, 1H, 6-H), 6.04 (s, 1H, 4-H), 8.00 (s, 1H, Formyl), 8.05 (s, 1H, Formyl)

Subsequently, 870 mg of the thus obtained crude 7,24-dichloro-cholesta-4-en-3-one-6,25-diol diformyl ester (compound 27a) was dissolved in 15 ml of methanol, and 300 gm of KHC03 (3 mmol) was then added thereto. The obtained mixture was stirred at room temperature for 8 hours, so as to cleave the ester. Thereafter, 694 mg of K2CO3 (5 mmol) was added thereto, and the obtained mixture was then stirred at room temperature for 24 hours, so as to carry out the ring closure of an intermediate chlorohydrin to epoxy. After completion of the reaction, 1 ml of acetic acid was added to the reaction solution, and the methanol was then concentrated. Thereafter, water was added thereto, followed by extraction with ethyl acetate. The extract was dried, concentrated, and then purified by silica gel chromatography, so as to obtain 341 mg of a crude compound, 6,7:24,25-diepoxycholesta-4-en-3 one. The total yield from the two steps was found to be approximately 57%. In addition, the obtained crude 6,7:24,25-diepoxycholesta-4-en-3 one was only 6β,7β-epoxy.

### [Example 17]

### <Step 8A: production of 5β-24,25-epoxycholesta-3,7-diol (compound 14)>

449 mg (1.08 mmol) of 5β-24,25-epoxycholesta-3-one-7-ol (compound 6) which was obtained in accordance with Example 5-2 was dissolved in 10 ml of methanol, and 22 mg (0.54 mmol) of 95% sodium borohydride was then added thereto under cooling on ice. The obtained mixture was stirred for 1 hour, while cooling on ice. After completion of the reaction, water and acetic acid were added to the reaction solution, and methanol was then distilled away under a reduced pressure, followed by extraction with ethyl acetate. Subsequently, the organic phase was washed with water, dried, and then concentrated, so as to obtain 450 mg of a crude compound, 5β-24,25-epoxycholesta-3,7-diol. The yield of the obtained compound was found to be 99%. The NMR shift value (δ ppm) is shown below.
δ: 0.67 (s, 3H, 18-H), 0.91 (s, 3H, 19-H, 3α 7α OH), 0.94 (d, 6.8Hz, 21-H), 0.95 (s, 3H, 19-H, 3β 7α OH), 0.98 (dt, 3.6Hz and 14Hz, 1H), 1.28 (s, 3H, 26-H), 1.31 (s, 3H, 27-H), 2.0 (q, 11.6Hz, 1H), 2.68 (t, 6Hz, 1H, 24-H), 3.46 (m, 1H, 3-H, 3α 7α OH), 3.86 (m, 1H, 7-H), 4.07 (m, 1H, 3-H, 3β 7α OH)

### [Example 18]

### <Step 9A: production of 5β-24,25-epoxycholesta-3,7-diol diacetate (compound 15a)>

450 mg of the crude compound, 5β-24,25-epoxycholesta-3,7-diol (compound 14), which was obtained in Example 17, was dissolved in 10 ml of ethyl acetate, and 0.44 g of acetic anhydride, 0.45 g of triethylamine and a catalytyc amount of N,N-dimethylaminopyridine were then added thereto. The obtained mixture was stirred at room temperature for 16 hours. After completion of the reaction, water was added, and the obtained mixture was stirred at room temperature for several hours. Thereafter, the water phase was separated, and it was then washed with a saturated sodium bicarbonate solution. The resultant was dried and was then concentrated, so as to obtain 501 mg of a crude compound, 5β-24,25-epoxycholesta-3,7-diol diacetate. The yield was found to be 93%. The NMR shift value (δ ppm) is shown below.
δ: 0.66 (s, 3H, 18-H), 0.93 (s, 3H, 19-H, 3α 7α OAc), 0.94 (d, 5.6Hz, 3H, 21-H) , 0.97 (s, 3H, 19-H, 3β 7α OAc), 1.26 (s, 3H, 26-H), 1.30 (s, 3H, 27-H) , 2.03 (s, 3H, OAc, 3β 7α OAc), 2.04 (s, 3H, OAc, 3β 7α OAc), 2.05 (s, 3H, OAc, 3α 7α OAc), 2.06 (s, 3H, OAc, 3α 7α OAc), 2.67 (t, 6.4Hz, 1H, 24-H), 4.59 (m, 1H, 3-H, 3α 7α OAc), 4.88 (m, 1H, 7-H), 5.03 (m, 1H, 3-H, 3β 7α OAc)

### [Example 19]

### <Step 10A: production of 5β-cholesta-24-one-3,7-diol diacetate (compound 16a)>

500 mg (0.10 mmol) of the crude compound, 5β-24,25-epoxycholesta-3,7-diol diacetate (compound 15a), which was obtained in Example 18, was dissolved in 10 ml of ethyl acetate, and 0.41 g (2.99 mmol) of zinc chloride was then added thereto. The obtained mixture was stirred at room temperature for 20 hours. After completion of the reaction, the organic phase was washed with a saturated sodium bicarbonate solution. The resultant was dried, concentrated, and then purified by silica gel column chromatography, so as to obtain 365 mg of 5β-cholesta-24-one-3,7-diol diacetate. The yield was found to be 73%. The NMR shift value (δ ppm) is shown below.
δ: 0.65 (s, 3H, 18-H), 0.91 (d, 6.4Hz, 3H, 21-H), 0.93 (s, 3H, 19-H, 3α 7α OAc), 0.97 (s, 3H, 19-H, 3β 7α OAc), 1.09 (d, 6.8Hz, 3H, 26-H and 27-H), 2.03 (s, 3H, OAc, 3α 7α OAc), 2.05 (s, 3H, OAc, 3α 7α OAc), 2.04 (s, 3H, OAc, 3β 7α OAc), 2.04 (s, 3H, OAc, 3β 7α OAc), 2.32-2.50 (m, 2H, 23-H), 2.61 (m, 1H, 25-H), 4.59 (m, 1H, 3-H, 3α 7α OAc), 4.88 (m, 1H, 7-H), 5.03 (m, 1H, 3-H, 3β 7α OAc)

### [Example 20]

### <Step 10B: production of cholesta-4,6-dien-3,24-dione (compound 18)>

1.88 g (4.74 mmol) of the 24,25-epoxycholesta-4,6-dien-3-one (compound 10) which was obtained in accordance with Example 10 was dissolved in 38 ml of ethyl acetate, and 1.62 g (11.9 mmol) of zinc chloride was then added thereto. The obtained mixture was stirred at room temperature for 37 hours. After completion of the reaction, the organic phase was washed with a saturated sodium bicarbonate solution. The resultant was dried, concentrated, and then purified by silica gel column chromatography, so as to obtain 1.39 g of cholesta-4,6-dien-3,24-dione. The yield was found to be 74%. The NMR shift value (δ ppm) is shown below.
δ :0.75 (s, 3H, 18-H), 0.92 (d, 6.5Hz, 3H, 21-H), 1.11 (s, 3H, 19-H), 1.10 (d, 7Hz, 6H, 26-H and 27-H), 2.19 (t, 9.4Hz, 1H), 2.32-2.66 (m, 5H), 5.67 (s, 1H, 4-H), 6.08-6.15 (m, 2H, 6-H and 7-H)

### [Example 21]

### <Step 3D: production of 6,7-epoxycholesta-4-en-3,24-dione (compound 19)>

1.57 g (3.96 mmol) of the cholesta-4,6-dien-3,24-dione (compound 18) which was obtained in accordance with Example 20 was dissolved in 21.2 ml of butyl acetate, and 6.3 ml of water was then added thereto. Thereafter, (*1) 657 µl (0.79 mmol) of a 1.21 M 2-methylperbenzoic acid/n-butyl acetate solution (hereinafter abbreviated as a peracid solution) was added to the above mixed solution at 73°C, and the obtained mixture was then stirred at 73°C for 0.5 hours. Subsequently, 657 µl (0.79 mmol) of a peracid solution was further added to the reaction solution, and the obtained mixture was then stirred at 73°C for 1 hour. Thereafter, the water phase was separated and eliminated, and the resultant was then washed with a saturated sodium bicarbonate solution and then with water. Thereafter, 6.3 ml of water was added to the resultant. The same operation as that described in * 1 was repeated once on the mixed solution. Further, 657 µl (0.79 mmol) of a peracid solution was added to the reaction solution at 73°C, and the obtained mixture was then stirred at 73°C for 0.5 hours. Thereafter, 657 µl (0.79 mmol) of a peracid solution was further added to the reaction solution, and the obtained mixture was then stirred at 73°C for 2 hours. Thereafter, the resultant was cooled to room temperature, and sodium sulfite was added thereto to decompose the residual peroxide, followed by extraction with ethyl acetate. Subsequently, the organic phase was washed with a saturated sodium bicarbonate solution, dried, concentrated, and then purified by silica gel column chromatography, so as to obtain 1.09 g of 6,7-epoxycholesta-4-en-3,24-dione. The yield was found to be 67%. The NMR shift value (δ ppm) thereof is shown below.
δ: 0.73 (s, 3H, 18-H), 0.92 (d, 6.4Hz, 3H, 21-H), 1.09 (s, 3H, 19-H), 1.10 (d, 6.8Hz, 3H, 26-H and 27-H), 2.32-2.61 (m, 4H, 2-H and 23-H), 2.61 (m, 1H, 25-H), 3.34 (d, 3.6Hz, 1H, 7-H, α-epoxy), 3.36 (s, 2H, 6-H and 7-H, β-epoxy), 3.45 (d, 3.6Hz, 1H, 6-H, α-epoxy), 6.11 (s, 1H, 4-H, α-epoxy), 6.15 (s, 1H, 4-H, β-epoxy)

### [Example 22]

### <Step 4C: production of 5β-cholesta-3,24-dion-7-ol (compound 20)>

243 mg of 5% palladium carbon (water content: 55%) was suspended in 7.8 ml of methanol, and 1.03 ml (6.83 mmol) of tetramethylethylenediamine was then added thereto.The obtained mixture was stirred in a hydrogen atmosphere at 50°C for 1 hour. Subsequently, the reaction solution was cooled to room temperature, and 2.05 ml of water was then added thereto. The inside of the reaction system was substituted with hydrogen of 1 atm, and the reaction solution was then stirred at room temperature for 1 hour. Subsequently, the pre-treated catalyst suspension was cooled to 5°C. Thereafter, 7.2 ml of an ethyl acetate solution containing 1.05 g (2.55 mmol) of the 6,7-epoxycholesta-4-en-3,24-dione (compound 19) obtained in the aforementioned Example 21 and 10 ml of methanol were added thereto in a hydrogen atmosphere. The obtained mixture was stirred in a hydrogen atmosphere of 1 atm at 5°C for 27 hours. After completion of the reaction, the catalyst was filtrated, and the filtrate was then concentrated, followed by extraction with ethyl acetate. Subsequently, the organic phase was successively washed with diluted hydrochloric acid and then with a saturated sodium bicarbonate solution. It was dried and was then concentrated, so as to obtain 1.21 g of a crude compound, 5β-cholesta-3,24-dion-7-ol. The yield was found to be 96%. The NMR shift value (δ ppm) thereof is shown below.
δ: 0.70 (s, 3H, 18-H, 7α OH), 0.72 (s, 3H, 18-H, 7β OH), 0.93 (d, 6.8Hz, 3H, 21-H), 1.01 (s, 3H, 19-H, 7α OH), 1.05 (s, 3H, 19-H, 7β OH), 1.09 (d, 7.2Hz, 6H, 26-H and 27-H), 2.33-2.51 (m, 3H), 2.61 (m, 1H, 25-H), 3.39 (t, 13.6Hz, 1H, 4-H), 3.62 (br, 1H, 7-H, 7β OH), 3.93 (m, 1H, 7-H, 7α OH)

### [Example 23]

### <Step 8B: production of 5β-cholesta-24-one-3,7-diol (compound 21)>

247 mg (0.593 mmol) of the 5β-cholesta-3,24-dion-7-ol (compound 20) which was obtained in Example 22 was dissolved in a mixed solution of 20 ml of methanol and 4 ml of water. Thereafter, 0.09 g (2.25 mmol) of sodium hydroxide and 1 g of a Raney nickel catalyst (R-100 water content product manufactured by Nikkorika) were added to the solution. The obtained mixture was stirred in a hydrogen atmosphere at room temperature for 5.5 hours. After completion of the reaction, the catalyst was filtrated, and the methanol was then distilled away, followed by extraction with ethyl acetate. The resultant was dried and was then concentrated, so as to obtain 260 mg of a crude compound, 5β-cholesta-24-one-3,7-diol. The yield was found to be 95%. The NMR shift value (δ ppm) thereof is shown below.
δ: 0.66 (s, 3H, 18-H), 0.91 (s, 3H, 19-H, 3α 7α OH), 0.94 (s, 3H, 19-H, 3α 7β OH), 0.95 (s, 3H, 19-H, 3β 7α OH), 0.92 (d, 7.6Hz, 3H, 21-H), 0.98 (dt, 3.2Hz and 14Hz, 1H), 1.09 (d, 6.4Hz, 6H, 26-H and 27-H), 2.21 (q, 13.2Hz, 1H), 2.32-2.51 (m, 2H, 23-H), 2.61 (m, 1H, 25-H), 3.31 (m, 2H, 3-H and 7-H, 3α 7β OH), 3.46 (m, 1H, 3-H, 3α 7α OH), 3.85 (m, 1H, 7-H, 3α 7α OH and 3β 7α OH), 4.06 (m, 1H, 3-H, 3β 7α OH)

### [Example 24]

### <Step 9B: production of 5β-cholesta-24-one-3,7-diol diacetate (compound 16a)>

153 mg of the crude compound, 5β-cholesta-24-one-3,7-diol (compound 21), which was obtained in Example 23, was dissolved in 2 ml of ethyl acetate, and thereafter, 0.15 g of acetic anhydride, 0.15 g of triethylamine and a catalytic amount of N,N-dimethylaminopyridine were then added thereto. The obtained mixture was stirred at room temperature for 23 hours. After completion of the reaction, water was added to the reaction solution, and the obtained mixture was then stirred at room temperature for 1 hour, followed by separation of the water phase. The resultant was washed with a saturated sodium bicarbonate solution, dried, and then concentrated, so as to obtain 133 mg of a crude compound, 5β-cholesta-24-one-3,7-diol diacetate. The yield was found to be 72%. The NMR shift value (δ ppm) thereof is shown below.
δ: 0.65 (s, 3H, 18-H), 0.93 (s, 3H, 19-H, 3α 7α OAc), 0.88 (s, 3H, 19-H, 3α 7β OAc), 0.97 (s, 3H, 19-H, 3β 7α OAc), 0.91 (d, 6.8Hz, 3H, 21-H), 1.09 (d, 6.8Hz, 6H, 26-H and 27-H), 2.32-2.51 (m, 2H, 23-H), 2.61 (m, 1H, 25-H), 4.59 (m, 1H, 3-H, 3α 7α OAc), 4.68 (m, 2H, 3-H and 7-H, 3α 7β OAc), 4.88 (m, 1H, 7-H, 3α 7α OAc and 3β 7α OAc), 5.03 (m, 1H, 3-H, 3β 7α OAc)

### [Example 25]

### <Step 11: production of a 5β-3,7-diacetoxycholanic acid isopropyl ester (compound 17a)>

292 mg (0.58 mmol) of the 5β-cholesta-24-one-3,7-diol diacetate (compound 16a) which was obtained in accordance with Example 19 or 24 was dissolved in 10.73 ml (7 mmol) of a monoperphthalic acid/ethyl acetate solution (0.65 M). The obtained solution was stirred at 40°C for 45 hours. After completion of the reaction, an aqueous sodium sulfite solution was added to the reaction solution to decompose an excessive oxidizing agent, followed by extraction with ethyl acetate. Subsequently, the organic phase was washed with an aqueous saturated sodium bicarbonate solution, dried, and then concentrated, so as to obtain 303 mg of a crude compound, 5β-3,7-diacetoxycholanic acid isopropyl ester. The yield was found to be 98%. The NMR shift value (δ ppm) thereof is shown below.
δ: 0.65 (s, 3H, 18-H), 0.93 (s, 3H, 19-H, 3α 7α OAc), 0.90 (s, 3H, 19-H, 3α 7β OAc), 0.97 (s, 3H, 19-H, 3β 7α OAc), 0.92 (d, 6.0Hz, 3H, 21-H, , 3α 7α OAc and
3β 7α OAc), 0.89 (d, 6.8Hz, 3H, 21-H, , 3α 7β OAc), 1.22 (d, 6.4Hz, 6H, isopropyl), 2.03 (s, 3H, OAc, 3α 7α OAc), 2.05 (s, 3H, OAc, 3α 7α OAc), 2.04 (s, 3H, OAc, 3β 7α OAc), 2.04 (s, 3H, OAc, 3β 7α OAc), 2.04 (s, 3H, OAc, 3α 7β OAc), 2.14-2.34 (m, 2H, 23-H), 4.59 (m, 1H, 3-H, 3α 7α OAc), 4.68 (m, 2H, 3-H and 7-H, 3α 7β OAc), 4.88 (m, 1H, 7-H, 3α 7α OAc and
3β 7α OAc), 5.00 (m, 1H, isopropoxy), 5.04 (m, 1H, 3-H, 3β 7α OH)

### [Example 26]

### <Step 12: production of 5β-3,7-dioxocholanic acid (compound 8)>

244 mg (0.47 mmol) of the 5β-3,7-diacetoxycholanic acid isopropyl ester (compound 17a) which was obtained in Example 25 was dissolved in a mixed solution of 4 ml of methanol and 1 ml of water. Thereafter, 0.47 g of sodium hydroxide was added thereto, and the obtained mixture was then stirred for 7 hours while heating to reflux. After completion of the reaction, the methanol was distilled away under a reduced pressure, and diluted hydrochloric acid was then added thereto for acidification. The deposited precipitate was collected by filtration, and it was then dried, so as to obtain 167 mg of 5β-3,7-dioxocholanic acid. The yield was found to be 91%. The NMR shift value (δ ppm) thereof is shown below.
δ: 0.67 (s, 3H, 18-H), 0.91 (s, 3H, 19-H) , 0.94 (d, 6.8Hz, 3H, 21-H), 3.47 (m, 1H, 3-H, 3α 7α OH), 3.85 (m, 1H, 7-H), 4.07 (m, 1H, 3-H, 3β 7α OH)

Subsequently, 37 mg (0.094 mmol) of the obtained 5β-3,7-dioxocholanic acid was dissolved in a mixed solution of 1 ml of acetone and 0.3 ml of water. Thereafter, 84 mg (0.282 mmol) of sodium dichromate dihydrate and 111 mg (1.128 mmol) of concentrated sulfuric acid were successively added to the above solution under cooling on ice, and the obtained mixture was then stirred for 5 hours while cooling on ice. After completion of the reaction, 7 ml of water was added to the reaction solution, and the deposited precipitate was filtrated and was then dried, so as to obtain 29 mg of 5β-3,7-dioxocholanic acid. The yield was found to be 79%. It was confirmed that the NMR shift value (δ ppm) thereof was identical to that of Example 7.

### [Example 27]

### <Step 6D: production of cholan-4,6-dien-3-one-24-al (compound 22)>

1.78 g (4.29 mmol) of the cholesta-4,6-dien-3-one-24,25-diol (compound 11) which was obtained in accordance with Example 11-2 was dissolved in a mixed solution of 48 ml of acetonitrile and 36 ml of water. Thereafter, 1.38 g (6.44 mmol) of sodium periodate was added thereto, and the obtained mixture was then stirred at room temperature for 20 hours. Subsequently, the acetonitrile was distilled away under a reduced pressure, followed by extraction with ethyl acetate. The organic phase was washed with a saturated saline solution, dried, and then concentrated, so as to obtain 1.50 g of cholan-4,6-dien-3-one-24-al. The yield was found to be 98%. The NMR shift value (δ ppm) thereof is shown below.
δ: 0.76 (s, 3H, 18-H), 0.94 (d, 6.4Hz, 3H, 21-H), 1.11 (s, 3H, 19-H), 2.21 (t, 1H), 2.32-2.64 (m, 4H), 5.67 (s, 1H, 4-H), 6.08-6.15 (m, 2H, 6-H and 7-H), 9.77 (s, 1H, CHO)

### [Example 28]

### <Step 6E: production of cholan-4,6-dien-3-one-24-oic acid (compound 23)>

0.99 g (2.79 mmol) of the cholan-4,6-dien-3-one-24-al (compound 22) which was obtained in Example 27 was dissolved in a mixed solution of 15 ml of acetone and 4 ml of water. Thereafter, 831 mg (2.79 mmol) of sodium dichromate dihydrate and 1.1 g (11.26 mmol) of concentrated sulfuric acid were successively added to the above solution under cooling on ice, and the obtained mixture was then stirred for 2 hours while cooling on ice. After completion of the reaction, 1 ml of 2-propanol was added to the reaction solution, and the obtained mixture was then stirred for 1 hour. Thereafter, 100 ml of water was added to the reaction solution, and the deposited crystal was filtrated and was then dried, so as to obtain 0.90 g of cholan-4,6-dien-3-one-24-oic acid. The yield was found to be 87%. The NMR shift value (δ ppm) thereof is shown below.
δ :0.76 (s, 3H, 18-H), 0.95 (d, 6Hz, 3H, 21-H), 1.11 (s, 3H, 19-H), 2.19 (t, 1H), 2.24-2.62 (m, 4H), 5.67 (s, 1H, 4-H), 6.08-6.15 (m, 2H, 6-H and 7-H)

### [Example 29]

### <Step 13: production of a cholan-4,6-dien-3-one-24-oic acid methyl ester (compound 24a)>

1.22 g (3.29 mmol) of the cholan-4,6-dien-3-one-24-oic acid (compound 23) which was obtained in accordance with Example 28 was dissolved in 20 ml of acetone. Thereafter, 0.91 g (6.58 mmol) of potassium carbonate and 0.83 g (6.58 mmol) of dimethyl sulfate were added to the solution, and the obtained mixture was then stirred at 60°C for 1 hour. After completion of the reaction, the reaction solution was cooled to room temperature, and 2 ml of water was then added thereto, followed by stirring for several hours. Thereafter, 60 ml of water was further added to the reaction solution, and the deposited crystal was filtrated and was then dried, so as to obtain 1.26 g of a cholan-4,6-dien-3-one-24-oic acid methyl ester. The yield was found to be 99%. The NMR shift value (δ ppm) thereof is shown below.
δ: 0.76 (s, 3H, 18-H), 0.94 (d, 6.4Hz, 3H, 21-H), 1.11 (s, 3H, 19-H), 2.19-2.62 (m, 5H), 3.67 (s, 3H, COOMe), 5.67 (s, 1H, 4-H), 6.08-6.15 (m, 2H, 6-H and 7-H)

### [Example 30]

### <Step 3E: production of a 6,7-epoxycholan-4-en-3-one-24-oic acid methyl ester (compound 25a)>

1.20 g (3.12 mmol) of the cholan-4,6-dien-3-one-24-oic acid methyl ester (compound 24a) which was obtained in Example 29 was dissolved in 16.1 ml of butyl acetate, and 4.8 ml of water was then added thereto. Thereafter, (*1) 517 µl (0.62 mmol) of a 1.21 M 2-methylperbenzoic acid/n-butyl acetate solution (hereinafter abbreviated as a peracid solution) was added to the above mixed solution at 73°C, and the obtained mixture was then stirred at 73°C for 0.5 hours. Subsequently, 517 µl (0.62 mmol) of a peracid solution was added to the reaction solution, and the obtained mixture was then stirred at 73°C for 2.5 hours. Thereafter, the water phase was separated and eliminated, and the resultant was then washed with a saturated sodium bicarbonate solution and then with water. Thereafter, 4.8 ml of water was added thereto. The same operation as described in *1 above was repeated once on the mixed solution. Thereafter, 0.50 ml (0.53 mmol) of a peracid solution was further added to the mixed solution at 73°C, and the obtained mixture was then stirred at 78°C for 0.5 hours. Thereafter, 517 µl (0.62 mmol) of a peracid solution was further added to the reaction solution, and the obtained mixture was then stirred for 2 hours. Thereafter, the reaction solution was cooled to room temperature, and sodium sulfite was then added thereto, so as to decompose the residual peroxide, followed by extraction with ethyl acetate. Subsequently, the organic phase was washed with a saturated sodium bicarbonate solution, dried, and then concentrated. Thereafter, the concentrate was purified by silica gel column chromatography, so as to obtain 0.86 g of a 6,7-epoxycholan-4-en-3-one-24-oic acid methyl ester. The yield was found to be 69%. The NMR shift value (δ ppm) thereof is shown below.
δ: 0.73 (s, 3H, 18-H), 0.93 (d, 6.8Hz, 3H, 21-H), 1.09 (s, 3H, 19-H, α-epoxy), 2.19-2.60 (m, 4H), 3.34 (d, 3.6Hz, 1H, 7-H, α-epoxy), 3.36 (s, 2H, 6-H and 7-H, β-epoxy), 3.45 (d, 3.6Hz, 1H, 6-H, α-epoxy), 3.67 (s, 3H, COOMe), 6.11 (s, 1H, 4-H, α-epoxy), 6.15 (s, 1H, 4-H, β-epoxy)

### [Example 31]

### <Step 4D: production of a 5β-cholan-3-one-7-ol-24-oic acid methyl ester (a 5β-7-hydroxy-3-ketocholanic acid methyl ester) (compound 32d)>

52 mg of 5% palladium carbon (water content: 55%) was suspended in 1,600 µl of methanol, and 222 µl (1.47 mmol) of tetramethylethylenediamine was added thereto. The obtained mixture was stirred in a nitrogen atmosphere at 50°C for 1 hour. Subsequently, the reaction solution was cooled to room temperature, and 440 µl of water was added thereto. The inside of the reaction system was substituted with hydrogen of 1 atm, and the reaction solution was then stirred at room temperature for 1 hour. Subsequently, the pre-treated catalyst suspension was cooled to 5°C. Thereafter, 4,200 µl of an ethyl acetate solution containing 220 mg (0.55 mmol) of the 6,7-epoxycholan-4-en-3-one-24-oic acid methyl ester (compound 25a) obtained in the above Example 30 and 8,000 µl of methanol were added thereto in a hydrogen atmosphere. The obtained mixture was stirred in a hydrogen atmosphere of 1 atm at 5°C for 23 hours. After completion of the reaction, the catalyst was filtrated, and the filtrate was then concentrated, followed by extraction with ethyl acetate. Subsequently, the organic phase was successively washed with diluted hydrochloric acid and then with a saturated sodium bicarbonate solution. The resultant was dried and was then concentrated, so as to obtain 217 mg of 5β-24,25-epoxycholesta-3-one-7-ol. The yield was found to be 95%. The NMR shift value (δ ppm) thereof is shown below.
δ: 0.70 (s, 3H, 18-H, 7α OH), 0.72 (s, 3H, 18-H, 7β OH), 0.94 (d, 6.8Hz, 3H, 21-H), 1.01 (s, 3H, 19-H, 7α OH), 1.05 (s, 3H, 19-H, 7β OH), 2.52 (t, 14.4Hz, 1H, 4-H, 7β OH), 3.39 (t, 13.6Hz, 1H, 4-H, 7α OH), 3.62 (br, 1H, 7-H, 7β OH), 3.67 (s, 3H, COOMe), 3.93 (m, 1H, 7-H, 7α OH)

### [Example 32]

### <Step 6F: production of 5β-3,7-dioxocholanic acid methyl ester (compound 8)>

80 mg (0.20 mmol) of the 5β-7-hydroxy-3-ketocholanic acid methyl ester (compound 32d) which was obtained in Example 31 was dissolved in a mixed solution of 3 ml of acetone and 0.5 ml of water. Thereafter, 118 mg (0.396 mmol) of sodium dichromate dihydrate and 155 mg of sulfuric acid were added to the solution under cooling on ice, and the obtained mixture was then stirred for several hours while cooling on ice. After completion of the reaction, water was added to the reaction solution, and the deposited crystal was collected by filtration, washed with water, and then dried, so as to obtain 65 mg of a 5β-3,7-dioxocholanic acid methyl ester. The yield was found to be 82%. The NMR shift value (δ ppm) thereof is shown below.
δ: 0.70 (s, 3H, 18-H), 0.93 (d, 6.0Hz, 3H, 21-H), 1.30 (s, 3H, 19-H), 2.49 (t, 10.2Hz, 1H), 2.88 (dd, 5.8Hz and 12.6Hz, 1H), 3.67 (s, 3H, COOMe)

### [Example 33]

### <Production of benzoic acid-6,7-dihydroxy-3,7-dimethyloctyl ester>

0.50 g (1.92 mmol) of 1-benzoyl citronellol was dissolved in 6 ml of chloroform. Thereafter, 0.99 g (5.76 mmol) of m-chloroperbenzoic acid was added thereto, and the obtained mixture was then stirred at room temperature for 1 hour. After completion of the reaction, an aqueous 10% sodium bisulfite solution was added to the reaction solution to decompose the residual peroxide, followed by extraction with chloroform. Subsequently, the organic phase was washed with an aqueous saturated potassium bicarbonate solution, dried, and then concentrated, so as to obtain 0.49 g of a crude compound, benzoic acid-5(3,3-dimethyloxiranyl)-3-methylpentyl ester. The yield was found to be 92%. The NMR shift value (δ ppm) is shown below.
δ :1.00 (d, 7.7Hz, 3H), 1.28 (s, 3H), 1.30 (s, 3H), 1.4-1.9 (m, 7H), 2.72 (t, 6.6Hz, 1H), 4.37 (m, 2H), 7.45 (m, 2H), 7.57 (m, 1H), 8.04 (m, 2H)

Subsequently, 0.10 g (0.36 mmol) of the obtained benzoic acid-5(3,3-dimethyloxiranyl)-3-methylpentyl ester was dissolved in 2 ml of ethyl acetate. Thereafter, 22 mg of water, 17 mg of acetic acid and 0.20 g of silica gel were added to the solution, and the obtained mixture was then stirred at 40°C for 24 hours. After completion of the reaction, the silica gel was filtrated, and the filtrate was then concentrated, so as to obtain 0.10 g of a crude benzoic acid-6,7-dihydroxy-3,7-dimethyloctyl ester. The conversion rate was found to be 100%, and the yield was found to be 94%. By-products other than the product of interest were not detected. The NMR shift value (δ ppm) thereof is shown below.
δ: 0.99 (t, 5.3Hz, 3H), 1.16 (s, 3H), 1.21 (s, 3H), 1.2-1.95 (m, 7H), 2.14-2.25 (m, 1H), 3.32 (m, 1H), 4.3-4.45 (m, 2H), 7.44 (t, 7.7Hz, 2H), 7.54 (m, 1H), 8.04 (d, 8.2Hz, 2H)

The reaction formula of the present example is shown below.

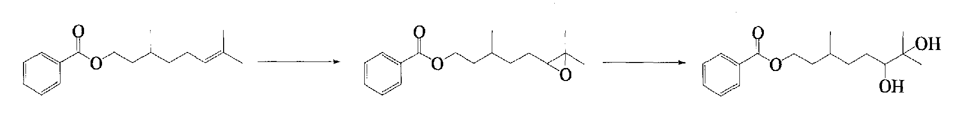

### INDUSTRIAL APPLICABILITY

According to the present invention, cholesta-4,6,24-trien-3-one useful as a synthetic intermediate of various steroid medicaments, or 5β-3,7-dioxo-cholanic acid and an ester derivative thereof useful as important synthetic intermediates of various steroid medicaments, such as ursodeoxycholic acid or chenodeoxycholic acid, can be efficiently and economically produced using, as a raw material, cholesta-5,7,24-trien-3β-ol, which is a sterol having double bonds at position 5 and at position 24. Such 5β-3,7-dioxo-cholanic acid and an ester derivative thereof can be stably supplied as a result of the establishment of an inexpensive chemical synthesis method, thereby greatly contributing to expansion of the intended use.

The present application claims priority from Japanese Patent Application No. 2006-4710, filed on January 12, 2006, and Japanese Patent Application No. 2006-10233, filed on January 18, 2006; the disclosure of which is hereby incorporated by reference. Moreover, the contents of all publications cited herein are incorporated herein by reference in their entirety.

## Claims

1. A method for producing 5β-3,7-dioxocholanic acid (8), ursodeoxycholic acid (32a), chenodeoxycholic acid (32b), 5β-3α-hydroxy-7-ketocholanie acid (32c) or 5β-7-hydroxy-3-ketocholanic acid (32d), represented by the following formula (8), (32a), (32b), (32c) or (32d), or an ester derivative thereof: 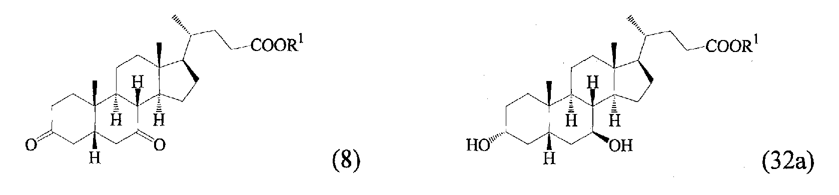 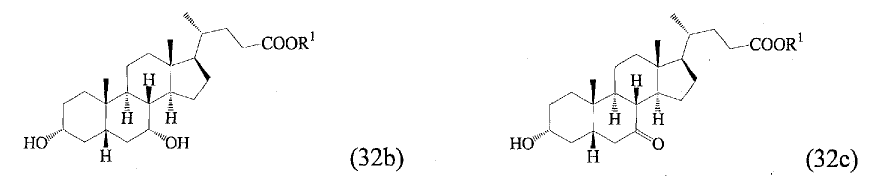 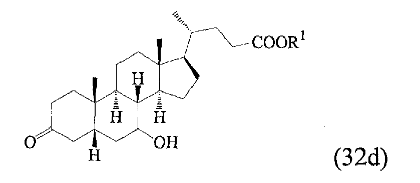 wherein R¹ represents a hydrogen atom or an alkyl group containing 1 to 6 carbon atoms,
which comprises a step of constructing a 5β configuration by reductive saturation of a double bond at position 4, using, as a raw material, a steroid compound containing 22 or more carbon atoms generated from carbohydrate by a fermentation method.

2. A method for producing 5β-3,7-dioxocholanic acid (8), ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (8), (32a), (32b), (32c) or (32d), or an ester derivative thereof: 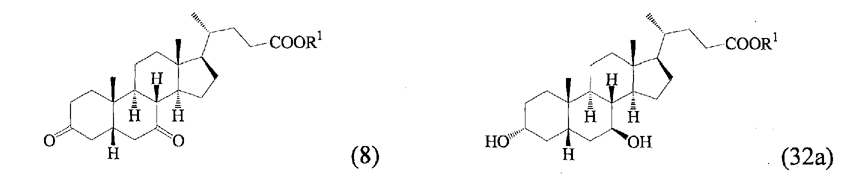 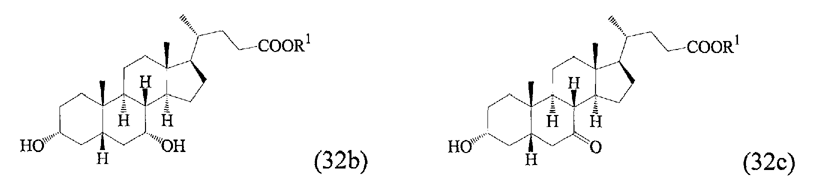 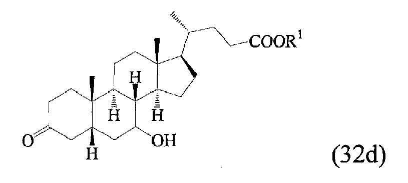 wherein R¹ represents a hydrogen atom or an alkyl group containing 1 to 6 carbon atoms,
wherein a 5β configuration is constructed by reductive saturation of a double bond at position 4 in a steroid compound represented by the following formula (A1), (A2), (A3), (A4), (A5), (A6), (A7) or (A8): 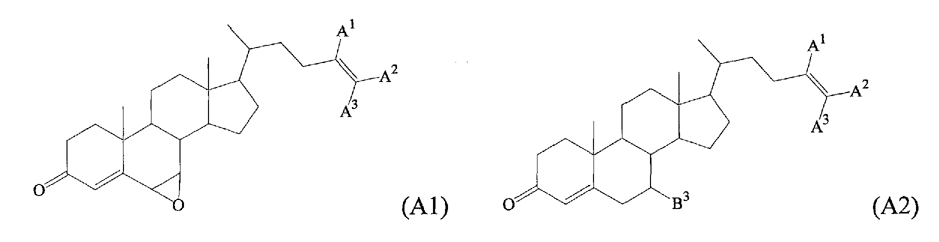 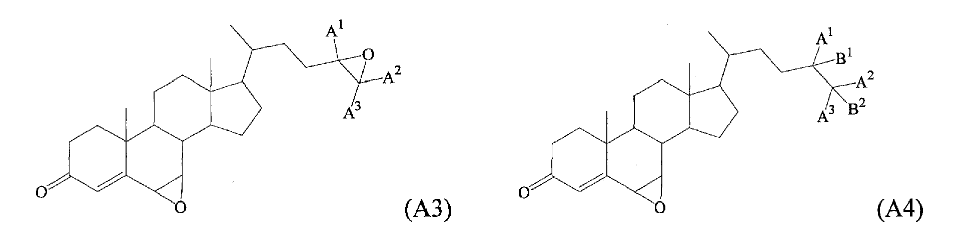 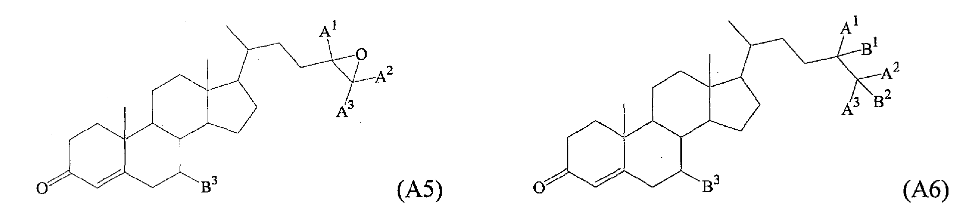 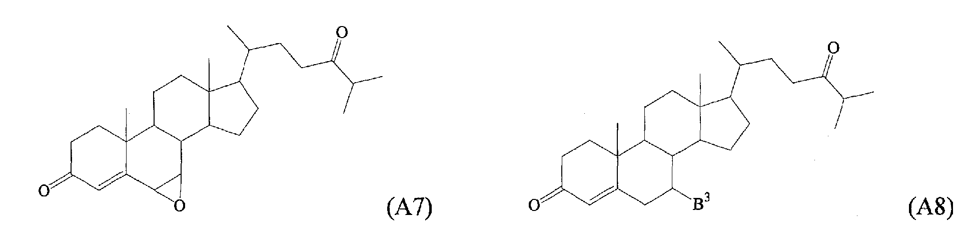 wherein A¹ represents a hydrogen atom or an isopropyl group; each of A² and A³ independently represents a methyl group when A¹ is a hydrogen atom, or a hydrogen atom or a methyl group when A¹ is an isopropyl group; and each of B¹, B² and B³ independently represents a hydroxyl group or a protected hydroxyl group.

3. A method for producing 5β-3,7-dioxocholanic acid (8), ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (8), (32a), (32b), (32c) or (32d), or an ester derivative thereof: 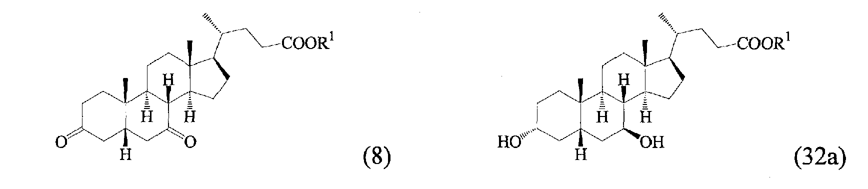 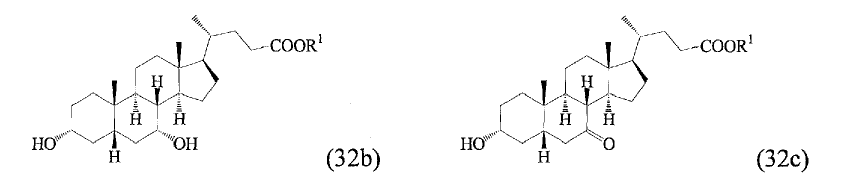 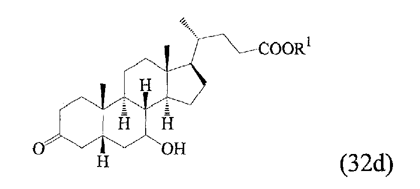 wherein R¹ represents a hydrogen atom or an alkyl group containing 1 to 6 carbon atoms,
wherein a 5β configuration is constructed by reductive saturation of a double bond at position 4 in a steroid compound represented by the following formula (A1), (A2), (A3), (A4), (A5), (A6), (A7), (A8), (A9) or (A10): 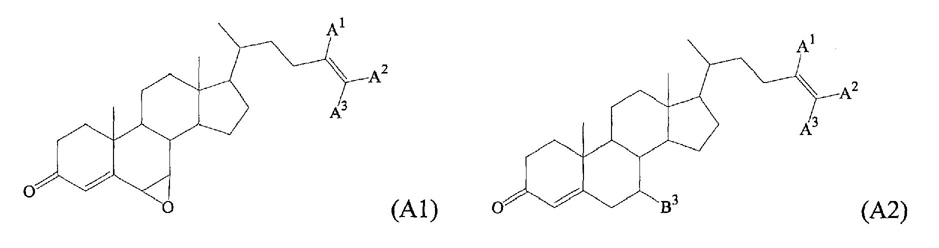 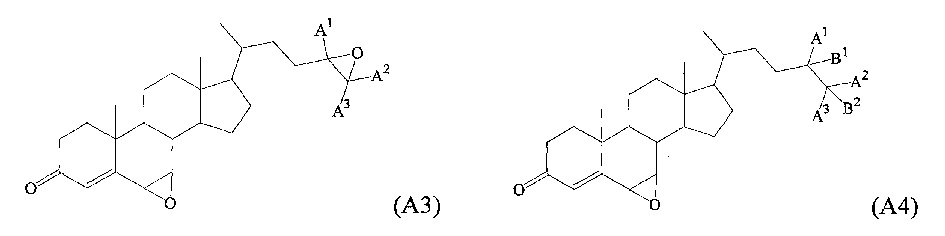 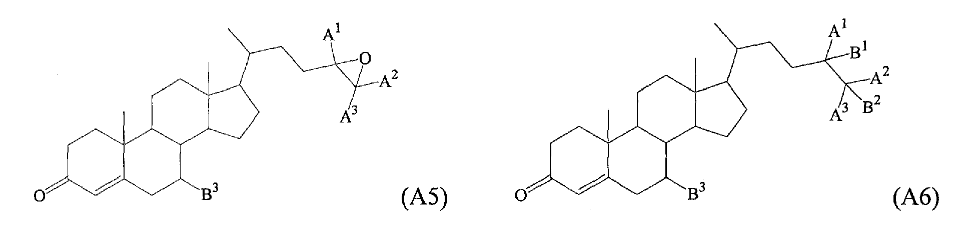 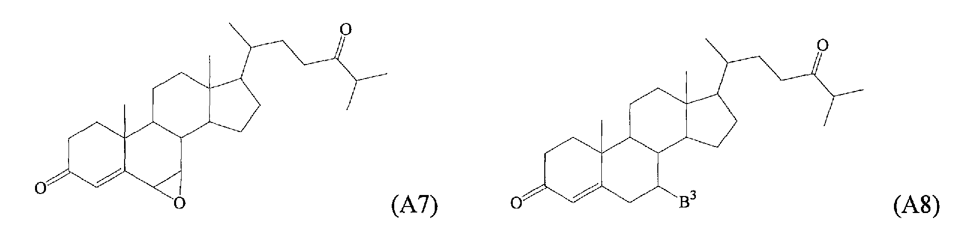 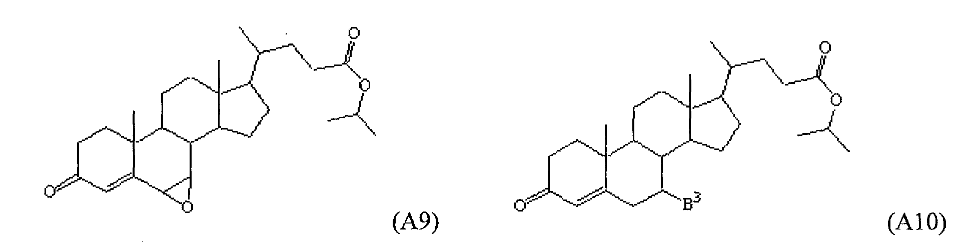 wherein A¹ represents a hydrogen atom or an isopropyl group; each of A² and A³ independently represents a methyl group when A¹ is a hydrogen atom, or a hydrogen atom or a methyl group when A¹ is an isopropyl group; and each of B¹, B² and B³ independently represents a hydroxyl group or a protected hydroxyl group,
wherein said steroid compound is induced from a sterol compound represented by the following formula (1): 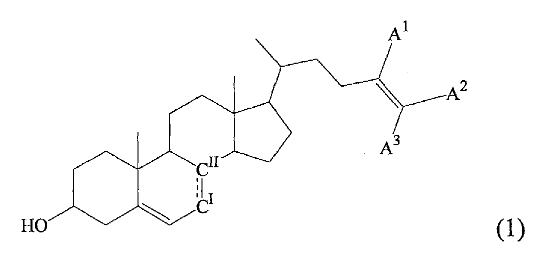 wherein A¹ represents a hydrogen atom or an isopropyl group; each of A² and A³ independently represents a methyl group when A¹ is a hydrogen atom, or a hydrogen atom or a methyl group when A¹ is an isopropyl group; and the bond between C^{I} and C^{II} represents a single bond or a double bond.

4. A method for producing 5β-3,7-dioxocholanic acid (8), ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (8), (32a), (32b), (32c) or (32d), or an ester derivative thereof: 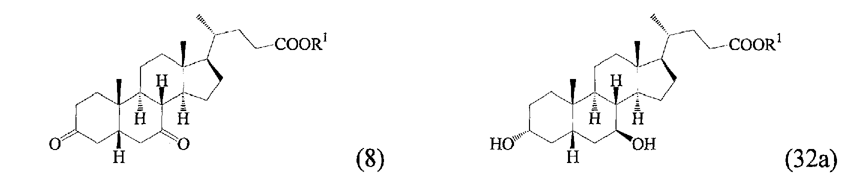 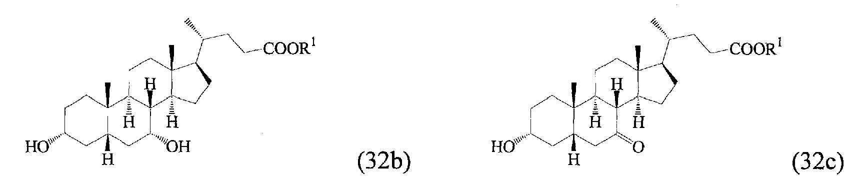 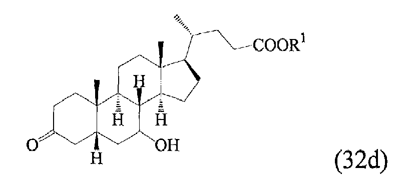 wherein R¹ represents a hydrogen atom or an alkyl group containing 1 to 6 carbon atoms,
wherein a sterol compound represented by the following formula (1) is used as a raw material: 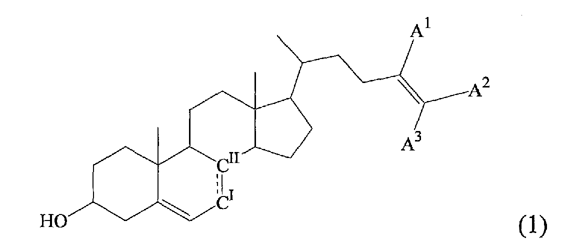 wherein A¹ represents a hydrogen atom or an isopropyl group; each of A² and A³ independently represents a methyl group when A¹ is a hydrogen atom, or a hydrogen atom or a methyl group when A¹ is an isopropyl group; and the bond between C^{I} and C^{II} represents a single bond or a double bond, and
said production method comprises the following steps:
(I) a step involving oxidation of a hydroxyl group at position 3 and isomerization of a double bond at position 5 to position 4;
(II) a step involving the oxidative cleavage of a side chain to convert position 24 to a carboxyl group or an ester derivative thereof;
(III) a step of introducing an oxygen functional group into position 7; and
(IV) a step of constructing a 5β configuration by reductive saturation of a double bond at position 4.

5. The method according to claim 4, wherein the sterol compound represented by the following formula (1) is cholesta-5,7,24-trien-3β-ol, ergosta-5,7,24(28)-trien-3β-ol, desmosterol, fucosterol, or ergosta-5,24(28)-dien-3β-ol: 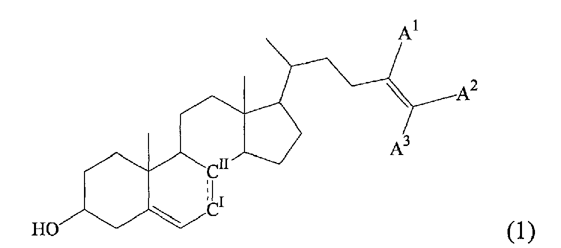 wherein A¹ represents a hydrogen atom or an isopropyl group; each of A² and A³ independently represents a methyl group when A¹ is a hydrogen atom, or a hydrogen atom or a methyl group
when A¹ is an isopropyl group; and the bond between C^{I} and C^{II} represents a single bond or a double bond.

6. The method according to claim 4, wherein the sterol compound represented by the following formula (1) is cholesta-5,7,24-trien-3β-ol: 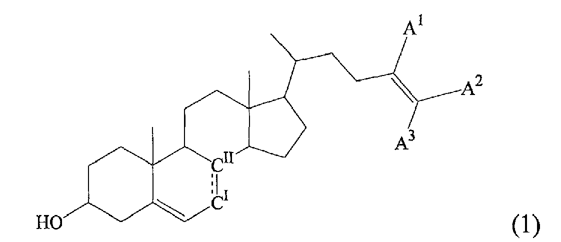 wherein A¹ represents a hydrogen atom or an isopropyl group; each of A² and A³ independently represents a methyl group when A¹ is a hydrogen atom, or a hydrogen atom or a methyl group
when A¹ is an isopropyl group; and the bond between C^{I} and C^{II} represents a single bond or a double bond.

7. A method for producing cholesta-4,6,24-trien-3-one represented by the following formula (4): 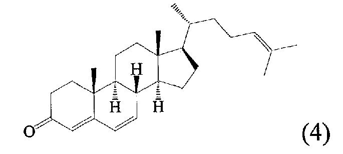 which comprises oxidizing cholesta-5,7,24-trien-3β-ol represented by the following formula (2) to obtain cholesta-4,7,24-trien-3-one represented by the following formula (3), and then isomerizing it:

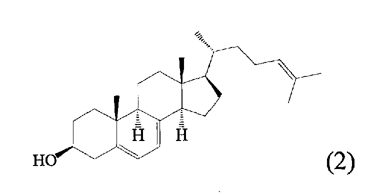

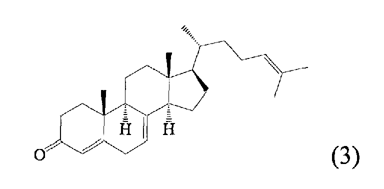

8. The method according to claim 7, wherein the oxidation reaction is carried out in the presence of a ketone compound and a metal alkoxide.

9. The method according to claim 8, wherein the oxidation reaction is carried out while oxygen is blocked.

10. The method according to claim 8, wherein the ketone compound is represented by the formula R²(C=O)R³ wherein each of R² and R³ independently represents a chain or cyclic alkyl group containing 1 to 10 carbon atoms, or R² and R³ may bind to each other to form a ring structure containing 3 to 8 carbon atoms).

11. The method according to claim 7, wherein the isomerization reaction is carried out in the presence of a basic compound.

12. The method according to claim 11, wherein the basic compound is hydroxide, carbonate or alkoxide of alkaline metal or alkaline-earth metal.

13. The method according to claim 11, wherein the isomerization reaction is carried out while oxygen is blocked.

14. A method for producing 3-oxo-4,7-diene steroid compound, wherein a method of oxidizing a 3-hydroxy-5,7-diene steroid compound represented by the following formula (2a), (2b), (2c), (2d) or (2e) to a compound represented by the following formula (3a), (3b), (3c), (3d) or (3e) is carried out while oxygen is blocked and in the presence of a ketone compound and a metal alkoxide: 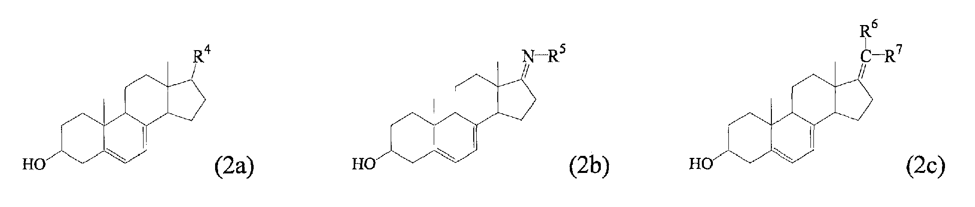 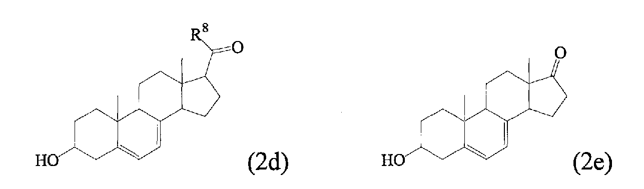 wherein each of R⁴ to R⁸ independently represents a hydrogen atom; a protected hydroxyl group; a halogen atom; or an alkyl group, alkenyl group or alkynyl group containing 1 to 10 carbon atoms that may be substituted with a carbonyl group, an ether group, a protected hydroxyl group, a halogen atom or a carboxyl group, 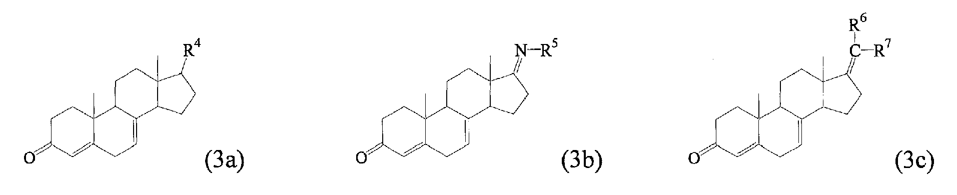 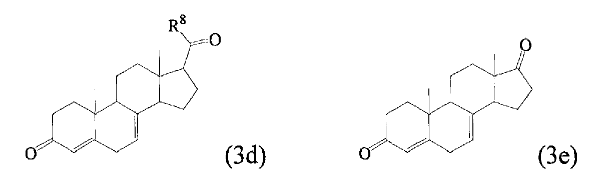 wherein each of R⁴ to R⁸ independently represents a hydrogen atom; a protected hydroxyl group; a halogen atom; or an alkyl group, alkenyl group or alkynyl group containing 1 to 10 carbon atoms that may be substituted with a carbonyl group, an ether group, a protected hydroxyl group, a halogen atom or a carboxyl group.

15. A method for producing 3-oxo-4,6-diene steroid compound, which comprises isomerizing a 3-oxo-4,7-diene steroid compound represented by the following formula (3a), (3b), (3c), (3d) or (3e) to a compound represented by the following formula (4a), (4b), (4c), (4d) or (4e), using a base as a catalyst: 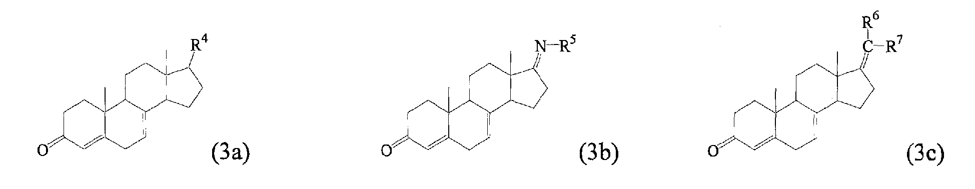 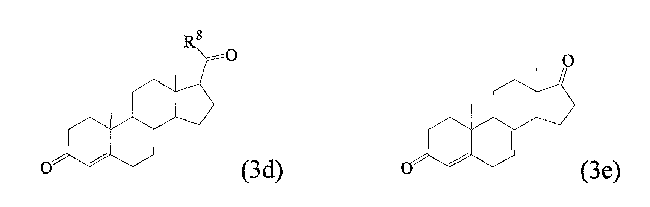 wherein each of R⁴ to R⁸ independently represents a hydrogen atom; a hydroxyl group; a protected hydroxyl group; a halogen atom; or an alkyl group, alkenyl group or alkynyl group containing 1 to 10 carbon atoms that may be substituted with a carbonyl group, an ether group, a hydroxyl group, a protected hydroxyl group, a halogen atom or a carboxyl group, 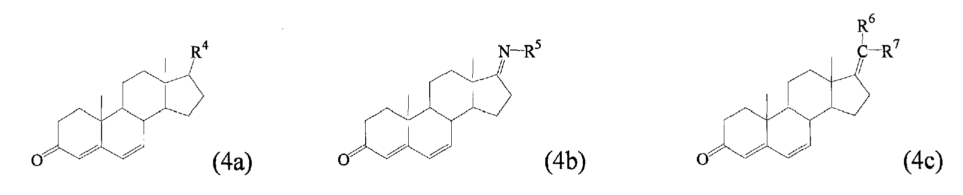 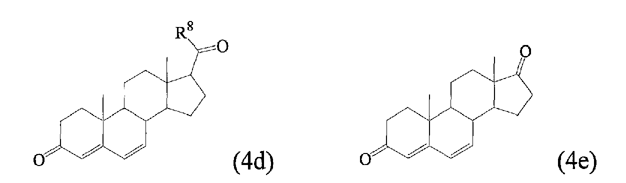 wherein each of R⁴ to R⁸ independently represents a hydrogen atom; a hydroxyl group; a protected hydroxyl group; a halogen atom; or an alkyl group, alkenyl group or alkynyl group containing 1 to 10 carbon atoms that may be substituted with a carbonyl group, an ether group, a hydroxyl group, a protected hydroxyl group, a halogen atom or a carboxyl group.

16. A method for producing 5β-3,7-dioxocholanic acid represented by the following formula (8) or an ester derivative thereof: 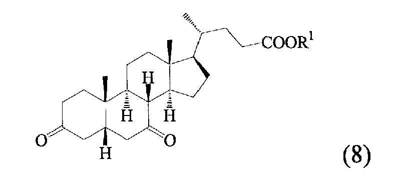 wherein R¹ represents a hydrogen atom or an alkyl group containing 1 to 6 carbon atoms,
which comprises epoxidizing cholesta-4,6,24-trien-3-one represented by the following formula (4) to obtain 6,7:24,25-diepoxycholesta-4-en-3-one represented by the following formula (5), then hydrogenating it to obtain 5β-24,25-epoxycholesta-3-one-7-ol represented by the following formula (6), further hydrolyzing it to obtain 5β-cholesta-3-one-7,24,25-triol represented by the following formula (7), further oxidizing it, and in some cases, further esterifying it:

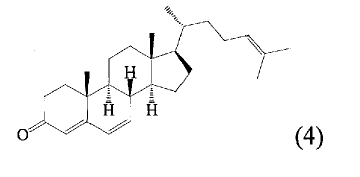

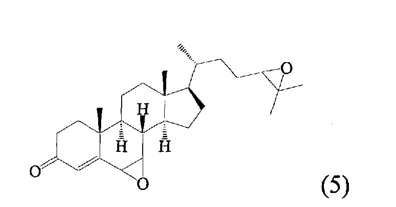

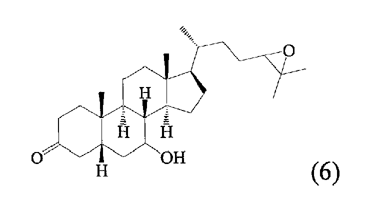

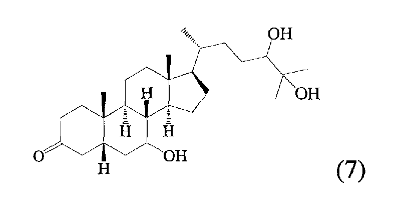

17. A method for producing 5β-3,7-dioxocholanic acid represented by the following formula (8) or an ester derivative thereof: 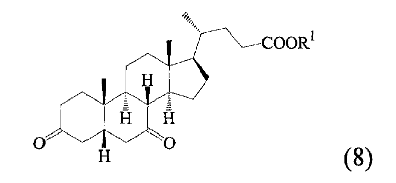 wherein R¹ represents a hydrogen atom or an alkyl group containing 1 to 6 carbon atoms,
which comprises epoxidizing cholesta-4,6,24-trien-3-one represented by the following formula (4) to obtain 6,7:24,25-diepoxycholesta-4-en-3-one represented by the following formula (5), then hydrolyzing it to obtain 6,7-epoxycholesta-4-en-3-one-24,25-diol represented by the following formula (9), further hydrogenating it to obtain 5β-cholesta-3-one-7,24,25-triol represented by the following formula (7), further oxidizing it, and in some cases, further esterifying it:

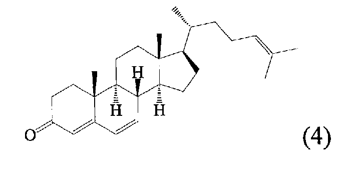

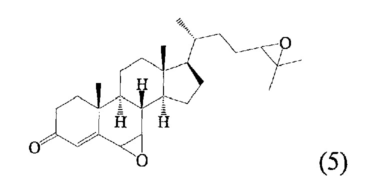

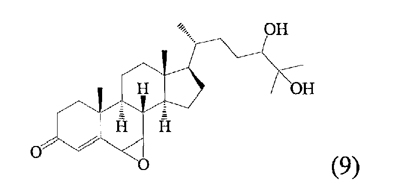

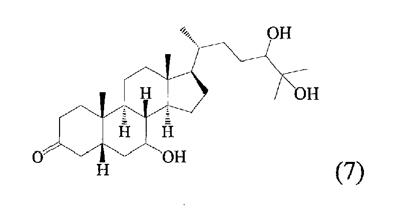

18. A method for producing 5β-3,7-dioxocholanic acid represented by the following formula (8) or an ester derivative thereof: 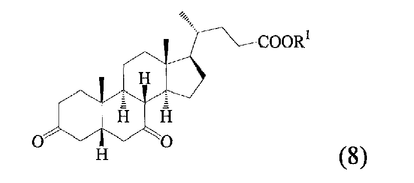 wherein R¹ represents a hydrogen atom or an alkyl group containing 1 to 6 carbon atoms,
which comprises epoxidizing cholesta-4,6,24-trien-3-one represented by the following formula (4) to obtain 24,25-epoxycholesta-4,6-dien-3-one represented by the following formula (10), then hydrolyzing it to obtain cholesta-4,6-dien-3-one-24,25-diol represented by the following formula (11), then epoxidizing it to obtain 6,7-epoxycholesta-4-en-3-one-24,25-diol represented by the following formula (9), further hydrogenating it to obtain 5β-cholesta-3-one-7,24,25-triol represented by the following formula (7), further oxidizing it, and in some cases, further esterifying it:

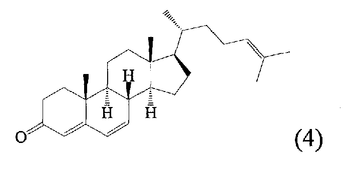

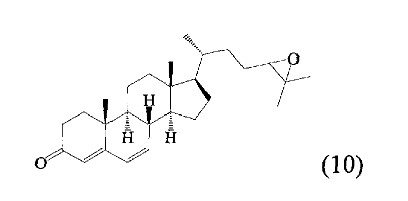

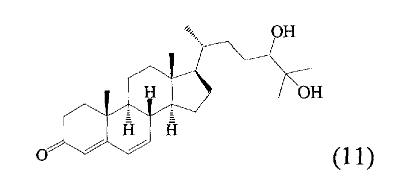

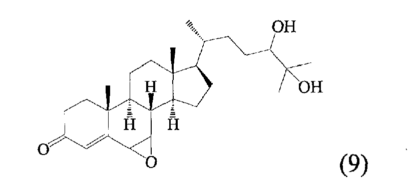

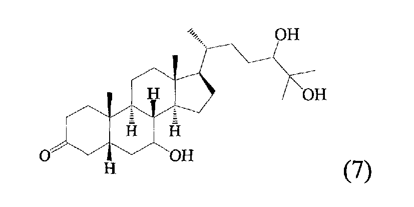

19. A method for producing 5β-3,7-dioxocholanic acid represented by the following formula (8) or an ester derivative thereof: 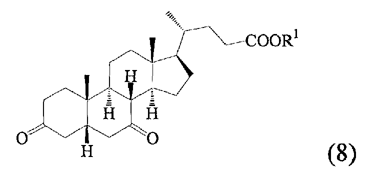 wherein R¹ represents a hydrogen atom or an alkyl group containing 1 to 6 carbon atoms,
which comprises epoxidizing cholesta-4,6,24-trien-3-one represented by the following formula (4) to obtain 6,7:24,25-diepoxycholesta-4-en-3-one represented by the following formula (5), then hydrogenating it to obtain 5β-24,25-epoxycholesta-3-one-7-ol represented by the following formula (6), further oxidizing it to obtain 5β-24,25-epoxycholesta-3,7-dione represented by the following formula (12), then hydrolyzing it to obtain 5β-cholesta-3,7-dion-24,25-diol represented by the following formula (13), further oxidizing it, and in some cases, further esterifying it:

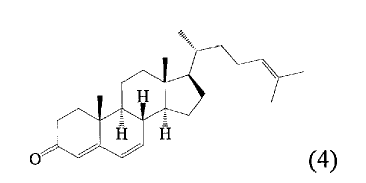

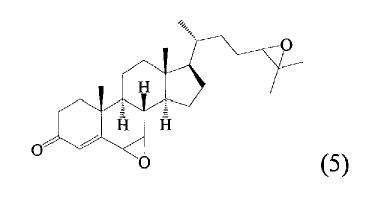

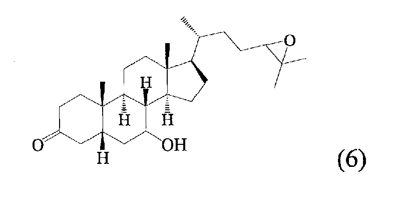

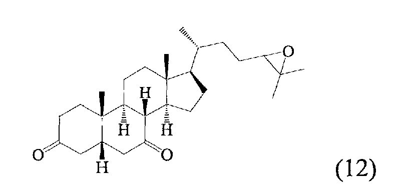

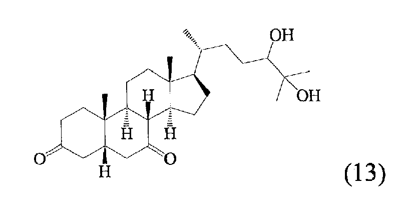

20. A method for producing 5β-3,7-dioxocholanic acid represented by the following formula (8) or an ester derivative thereof: 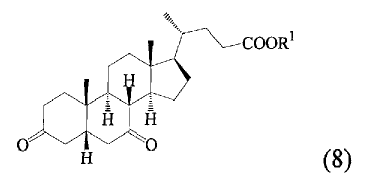 wherein R¹ represents a hydrogen atom or an alkyl group containing 1 to 6 carbon atoms,
which comprises epoxidizing cholesta-4,6,24-trien-3-one represented by the following formula (4) to obtain 6,7:24,25-diepoxycholesta-4-en-3-one represented by the following formula (5), then hydrogenating it to obtain 5β-24,25-epoxycholesta-3-one-7-ol represented by the following formula (6), further reducing it to obtain 5β-24,25-epoxycholesta-3,7-diol represented by the following formula (14), further protecting a hydroxyl group thereof to obtain a 5β-24,25-epoxycholesta-3,7-dion-24,25-diol derivative represented by the following formula (15), then isomerizing the epoxy to obtain a 5β-cholesta-24-one-3,7-diol derivative represented by the following formula (16), then oxidizing it to obtain a 5β-3,7-dihydroxycholanic acid isopropyl ester derivative represented by the following formula (17), then performing deprotection and oxidation thereon, and in some cases, further esterifying it: 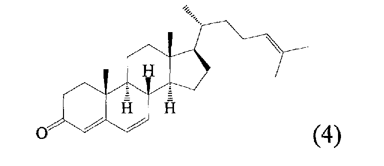 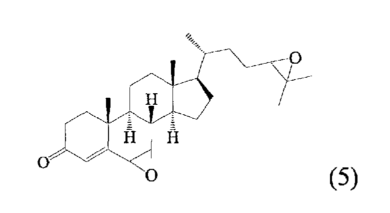 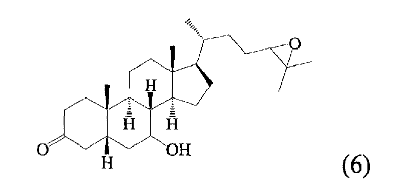 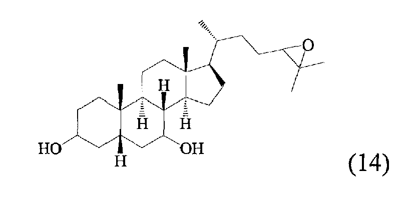 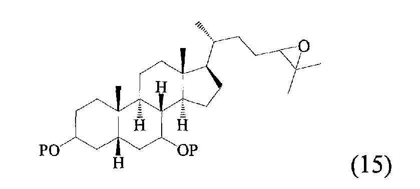 wherein P represents a protecting group for a hydroxyl group 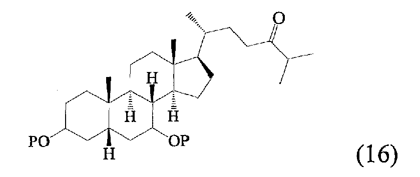 wherein P represents a protecting group for a hydroxyl group; 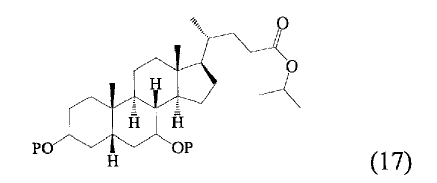 wherein P represents a protecting group for a hydroxyl group.

21. A method for producing 5β-3,7-dioxocholanic acid represented by the following formula (8) or an ester derivative thereof: 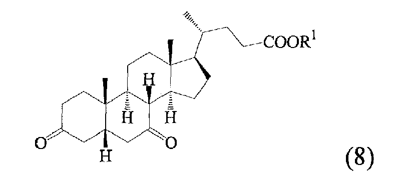 wherein R¹ represents a hydrogen atom or an alkyl group containing 1 to 6 carbon atoms,
which comprises epoxidizing cholesta-4,6,24-trien-3-one represented by the following formula (4) to obtain 24,25-epoxycholesta-4,6-dien-3-one represented by the following formula (10), then isomerizing it to obtain cholesta-4,6-dien-3,24-dione represented by the following formula (18), then epoxidizing it to obtain 6,7-epoxycholesta-4-en-3,24-dione represented by the following formula (19), further hydrogenating it to obtain 5β-cholesta-3,24-dion-7-ol represented by the following formula (20), further reducing it to obtain 5β-cholesta-24-one-3,7-diol represented by the following formula (21), further protecting a hydroxyl group thereof to obtain a 5β-cholesta-24-one-3,7-diol derivative represented by the following formula (16), further oxidizing it to obtain a 5β-3,7-dihydroxycholanic acid isopropyl ester derivative represented by the following formula (17), then performing deprotection and oxidation thereon, and in some cases, further esterifying it: 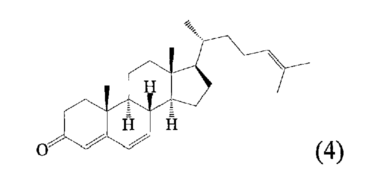 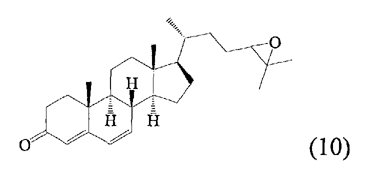 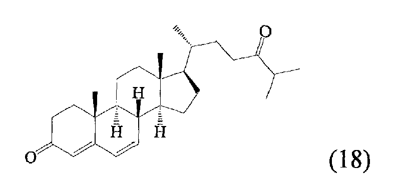 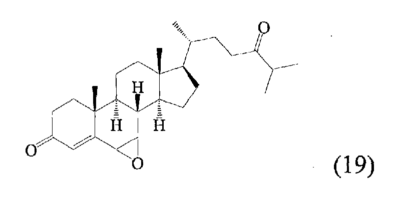 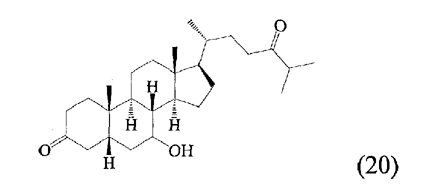 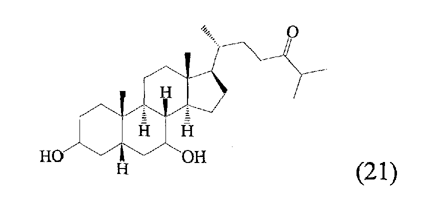 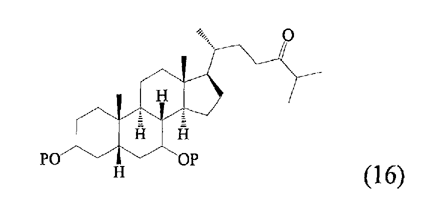 wherein P represents a protecting group for a hydroxyl group; 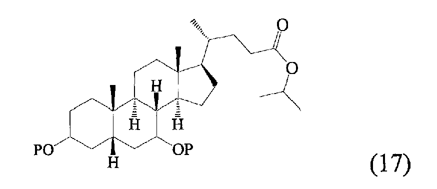 wherein P represents a protecting group for a hydroxyl group.

22. A method for producing 5β-3,7-dioxocholanic acid represented by the following formula (8) or an ester derivative thereof: 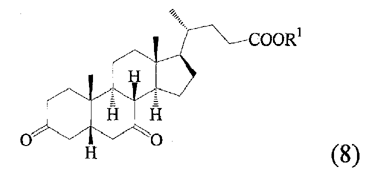 wherein R¹ represents a hydrogen atom or an alkyl group containing 1 to 6 carbon atoms,
which comprises epoxidizing cholesta-4,6,24-trien-3-one represented by the following formula (4) to obtain 24,25-epoxycholesta-4,6-dien-3-one represented by the following formula (10), then hydrolyzing it to obtain cholesta-4,6-dien-3-one-24,25-diol represented by the following formula (11), then oxidizing it to obtain 3-oxochola-4,6-dien-24-al represented by the following formula (22), further oxidizing it to obtain 3-oxochola-4,6-dienoic acid represented by the following formula (23), further esterifying it to obtain a 3-oxochola-4,6-dienoic acid ester derivative represented by the following formula (24), further epoxidizing it to obtain a 6,7-epoxy-3-oxochola-4-enoic acid ester derivative represented by the following formula (25), further hydrogenating it, further hydrolyzing the ester thereof in some cases to obtain a 5β-7-hydroxy-3-ketocholanic acid (32d) derivative represented by the following formula (32d), and further oxidizing it: 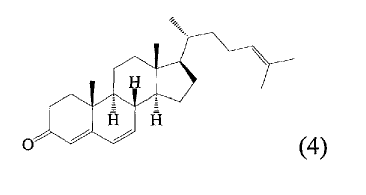 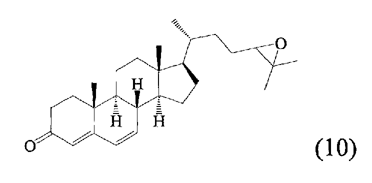 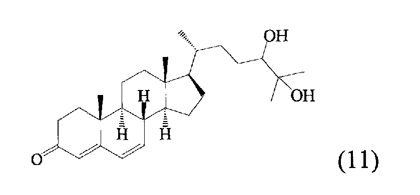 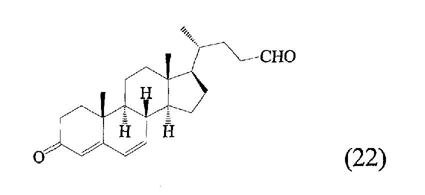 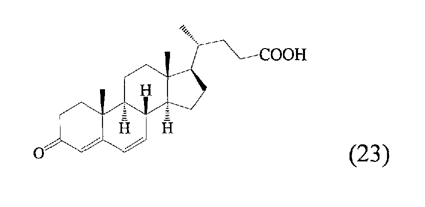 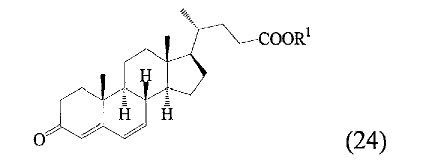 wherein R¹ represents an alkyl group containing 1 to 6 carbon atoms; 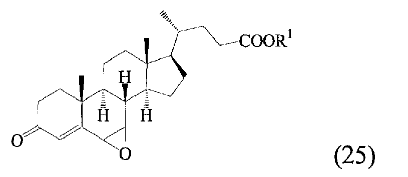 wherein R¹ represents an alkyl group containing 1 to 6 carbon atoms; 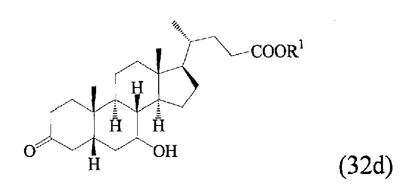 wherein R¹ represents a hydrogen atom or an alkyl group containing 1 to 6 carbon atoms.

23. The method according to any one of claims 16 to 22, wherein an organic peroxide is used as an epoxidizing agent.

24. The method according to claim 23, wherein percarboxylic acid represented by the formula A⁴CO₃H wherein A⁴ represents a hydrogen atom, an alkyl group containing 1 to 20 carbon atoms that may be substituted with a halogen atom, or an aryl group that may have a substituent, periminocarboxylic acid represented by the formula A⁵(C=NH)OOH wherein A⁵ represents a hydrogen atom, an alkyl group containing 1 to 20 carbon atoms that may be substituted with a halogen atom, or an aryl group that may have a substituent, or a dioxolane derivative represented by the following formula (26) is used as an organic peroxide: 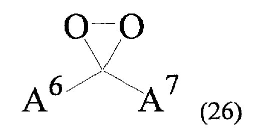 wherein each of A⁶ and A⁷ independently represents an alkyl group containing 1 to 20 carbon atoms that may be substituted with a halogen atom, or A⁶ and A⁷ may bind to each other to form a ring structure containing 3 to 8 carbon atoms.

25. The method according to claim 24, wherein perbenzoic acid or 2-methylperbenzoic acid is used as an organic peroxide.

26. The method according to claim 25, wherein water is added in the epoxidation reaction.

27. The method according to claim 25, wherein the concentration of peracid and the concentration of carboxylic acid are maintained at 0.3 M or lower in the epoxidation reaction.

28. The method according to claim 16, 17, 19 or 20, which comprises haloesterifying cholesta-4,6,24-trien-3-one represented by the following formula (4) to obtain a 7,24-dihalo-cholesta-4-en-3-one-6,25-diol diester represented by the following formula (27), and then performing the alkaline hydrolysis of the ester and cyclization to obtain 6,7:24,25-diepoxycholesta-4-en-3-one represented by the following formula (5): 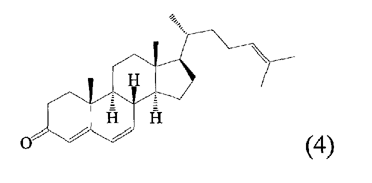 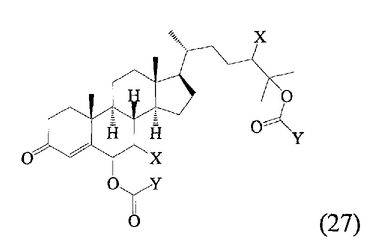 wherein X represents a halogen atom, and Y represents a hydrogen atom or an alkyl group containing 1 to 10 carbon atoms that may be substituted with a halogen atom;

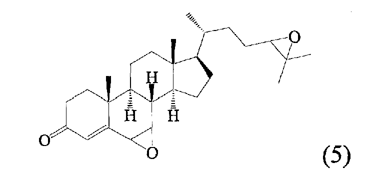

29. The method according to claim 28, wherein organic carboxylic acid and a halocation generator represented by the formula Z-X wherein X represents a halogen atom, and Z represents succinimide, phthalimide, acetamide, hydantoin, or a t-butoxy group are used loesterification agent.

30. The method according to any one of claims 16 to 22 and 28, wherein the hydrogenation reaction is carried out in the presence of a noble metal catalyst.

31. The method according to claim 30, wherein powdery palladium, or one or two or more types of metal palladium selected from the group consisting of activated carbon-supported palladium, aluminum oxide-supported palladium, barium carbonate-supported palladium, barium sulfate-supported palladium and calcium carbonate-supported palladium with a palladium content of 0.5% to 50% by weight, is used as a noble metal catalyst.

32. The method according to claim 30 or 31, wherein a base is allowed to coexist in the hydrogenation reaction in the presence of a noble metal catalyst.

33. The method according to claim 32, wherein amine is used as a base.

34. The method according to any one of claims 16 to 19, 22 and 28, wherein the epoxy hydrolysis reaction is carried out in the presence of silica gel or protonic acid.

35. The method according to claim 34, wherein hydrochloric acid, sulfuric acid, nitric acid, perchloric acid, phosphoric acid, phosphorous acid, hypophosphorous acid, organic carboxylic acid or organic sulfonic acid is used as protonic acid.

36. The method according to any one of claims 16 to 22 and 28, wherein halogen acid or a salt thereof, molecular halogen, permanganic acid, dichromic acid, or chromic acid is used as an oxidizing agent in the oxidation reaction.

37. The method according to claim 20 or 21, wherein Lewis acid, protonic acid, or a salt thereof is used as a catalyst in the isomerization reaction of the side chain 24,25-epoxy group to 24-ketone.

38. The method according to claim 37, wherein zinc chloride (II), zinc bromide (II) or zinc iodide (II) is used as Lewis acid.

39. The method according to claim 37, wherein halogen acid or a salt thereof is used as protonic acid or a salt thereof.

40. The method according to claim 20 or 21, wherein an organic peroxide is used in the oxidation reaction of the side chain ketone at position 24 to an isopropyl ester.

41. The method according to claim 40, wherein monoperphthalic acid or m-chloroperbenzoic acid is used as an organic peroxide.

42. The method according to claim 20 or 21, wherein hydrogen is used in the presence of a transition metal catalyst or hydride reduction is carried out as a means for reducing the ketone at position 3.

43. The method according to claim 42, wherein platinum oxide or Raney nickel is used as a transition metal catalyst.

44. The method according to any one of claims 16 to 22 and 28, wherein a compound obtained by isomerization of cholesta-4,7,24-trien-3-one represented by the following formula (3) is used as cholesta-4,6,24-trien-3-one represented by the following formula (4):

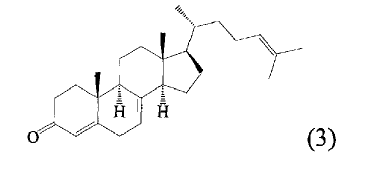

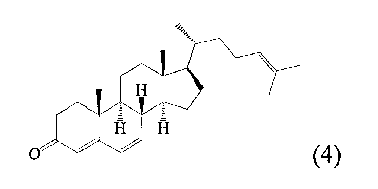

45. The method according to claim 44, wherein a compound obtained by oxidation of cholesta-5,7,24-trien-3β-ol represented by the following formula (2) is used as cholesta-4,7,24-trien-3-one represented by the following formula (3):

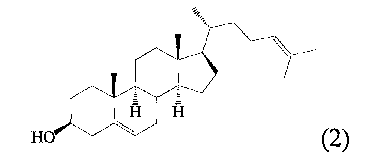

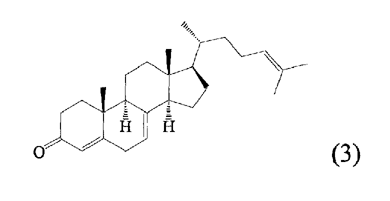

46. A method for producing a vicinal diol compound represented by the following formula (29): 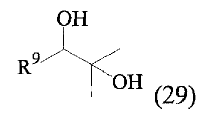 wherein R⁹ represents an alkyl group, alkenyl group or alkynyl group containing 1 to 20 carbon atoms that may be substituted with a hydroxyl group, a protected hydroxyl group, a carboxyl group, an ester group, a carbonyl group, a cyano group, an amino group or a halogen atom,
which comprises hydrolyzing an epoxy compound represented by the following formula (28), using silica gel as a catalyst: 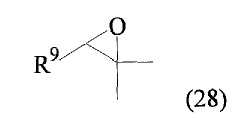 wherein R⁹ represents an alkyl group, alkenyl group or alkynyl group containing 1 to 20 carbon atoms that may be substituted with a hydroxyl group, a protected hydroxyl group, a carboxyl group, an ester group, a carbonyl group, a cyano group, an amino group or a halogen atom.

47. A method for producing a vicinal diol compound represented by the following formula (31): 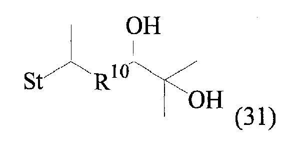 wherein St represents a steroid skeleton consisting of ring A, ring B, ring C, and ring D, wherein with regard to the aforementioned steroid skeleton, (1) it binds to a side chain shown in the formula at position C 17, (2) it may have a hydroxyl group, a protected hydroxyl group, a keto group, or an epoxy group on the ring A, ring B, ring C, and ring D, (3) C-C bonds at one or more positions selected from the group consisting of positions C1 to C8 may have double bonds, and (4) one or more positions selected from the group consisting of positions C4, C10, C13 and C14 may be substituted with methyl groups; and R¹⁰ represents an alkylene group, alkenylene group or alkynylene group containing 1 to 20 carbon atoms that may be substituted with a hydroxyl group, a protected hydroxyl group, a carboxyl group, an ester group, a carbonyl group, a cyano group, an amino group or a halogen atom,
which comprises hydrolyzing a steroid epoxy compound represented by the following formula (30), using silica gel as a catalyst: 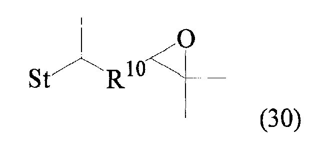 wherein St represents a steroid skeleton consisting of ring A, ring B, ring C, and ring D, wherein with regard to the aforementioned steroid skeleton, (1) it binds to a side chain shown in the formula at position C17, (2) it may have a hydroxyl group, a protected hydroxyl group, a keto group, or an epoxy group on the ring A, ring B, ring C, and ring D, (3) C-C bonds at one or more positions selected from the group consisting of positions C1 to C8 may have double bonds, and (4) one or more positions selected from the group consisting of positions C4, C10, C 13 and C 14 may be substituted with methyl groups; and R¹⁰ represents an alkylene group, alkenylene group or alkynylene group containing 1 to 20 carbon atoms that may be substituted with a hydroxyl group, a protected hydroxyl group, a carboxyl group, an ester group, a carbonyl group, a cyano group, an amino group or a halogen atom.

48. 5β-24,25-epoxycholesta-3,7-diol represented by the following formula (14):

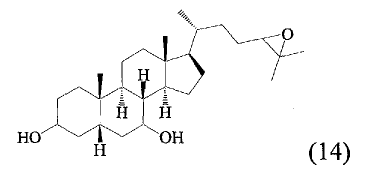

49. A 5β-24,25-epoxycholesta-3,7-diol derivative represented by the following formula (15): 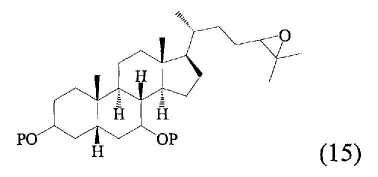 wherein P represents a protecting group for a hydroxyl group.

50. A 5β-cholesta-24-one-3,7-diol derivative represented by the following formula (16): 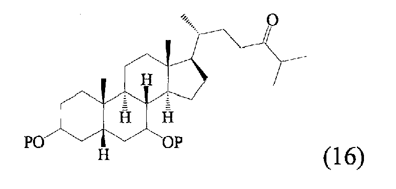 wherein P represents a protecting group for a hydroxyl group.

51. Cholesta-4,6-dien-3,24-dione represented by the following formula (18):

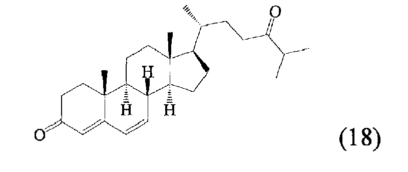

52. 6,7-epoxycholesta-4-en-3,24-dione represented by the following formula (19):

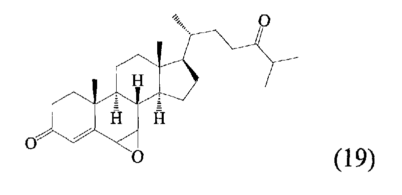

53. 5β-cholesta-3,24-dion-7-ol represented by the following formula (20):

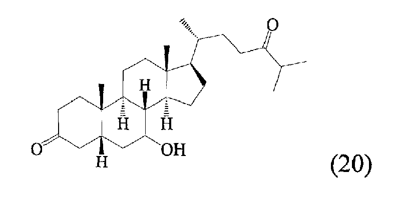

54. 5β-cholesta-24-one-3,7-diol represented by the following formula (21):

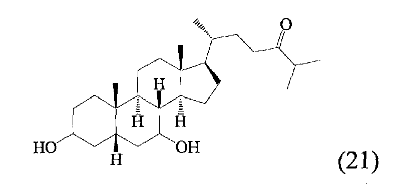

55. 3-oxochola-4,6-dien-24-al represented by the following formula (22):

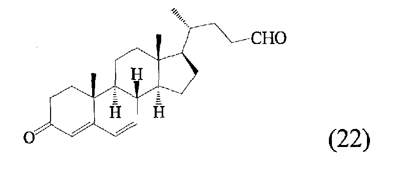

56. A method for producing ursodeoxycholic acid (32a), chenodeoxycholic acid (32b), 5β-3α-hydroxy-7-ketocholanic acid (32c) or 5β-7-hydroxy-3-ketocholanic acid (32d), represented by the following formula (32a), (32b), (32c) or (32d), or an ester derivative thereof: 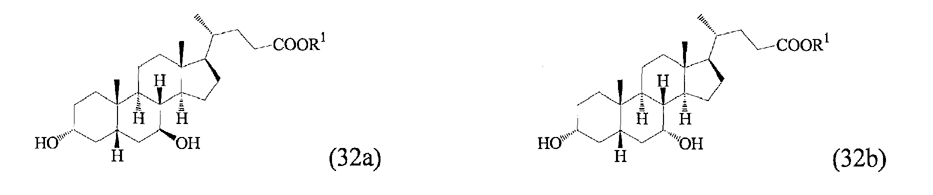 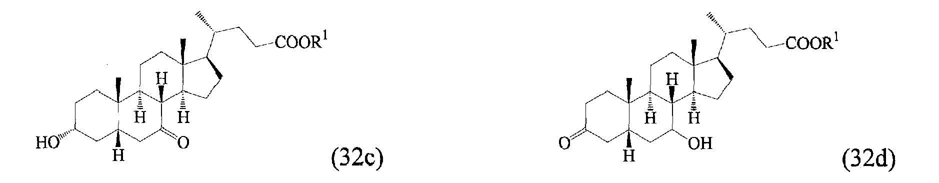 wherein R¹ represents a hydrogen atom or an alkyl group containing 1 to 6 carbon atoms,
which comprises reducing 5β-3,7-dioxocholanic acid represented by the following formula (8) or an ester derivative thereof, which is produced by the method according to any one of claims 16 to 22, 28, 44 and 45, and then, in some cases, reoxidizing it: 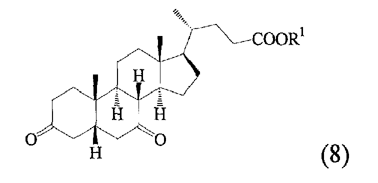 wherein R¹ represents a hydrogen atom or an alkyl group containing 1 to 6 carbon atoms.
